# EUROPEAN PATENT SPECIFICATION

(11) **EP 3 820 869 B1**
(45) Date of publication and mention of the grant of the patent: **30.08.2023**
(21) Application number: 19744868.1
(22) Date of filing: 12.07.2019
(51) Int. Cl.: C07D 471/04, C07D 491/107, A61P 25/18, A61K 31/397

(54) **SPIROCHROMANE DERIVATIVES**
SPIROCHROMANDERIVATE
DÉRIVÉS DE SPIROCHROMANE

(30) Priority: 13.07.2018 HU 1800248
(43) Date of publication of application: 19.05.2021
(73) Proprietor: Richter Gedeon Nyrt., 1103 Budapest (HU)
(72) Inventor: ÉLES, János, 1121 Budapest (HU); DUDÁSNÉ MOLNÁR, Katalin, 8200 Veszprém (HU); LEDNECZKI, István, 7025 Bölcske (HU); TAPOLCSÁNYI, Pál, 1046 Budapest (HU); HORVÁTH, Anita, 1171 Budapest (HU); NÉMETHY, Zsolt, 2131 Göd (HU); LÉVAY, György István, 2092 Budakeszi (HU)
(74) Representative: Hoffmann Eitle
(86) International application number: PCT/IB2019/055948
(87) International publication number: WO 2020/012422

(56) References cited:
- WO-A1-2009/127609
- WO-A1-2014/141091
- WO-A1-2017/165256
- WO-A1-2018/112204
- DATABASE REGISTRY [Online] CHEMICAL ABSTRACTS SERVICE, COLUMBUS, OHIO, US; 20 May 2018 (2018-05-20), XP002793751, retrieved from STN Database accession no. 2224106-02-7
- DATABASE REGISTRY [Online] CHEMICAL ABSTRACTS SERVICE, COLUMBUS, OHIO, US; 15 July 2016 (2016-07-15), XP002793752, retrieved from stn Database accession no. 1953198-57-6
- DATABASE REGISTRY [Online] CHEMICAL ABSTRACTS SERVICE, COLUMBUS, OHIO, US; 7 July 2016 (2016-07-07), XP002793753, retrieved from stn Database accession no. 1947149-33-8
- DATABASE REGISTRY [Online] CHEMICAL ABSTRACTS SERVICE, COLUMBUS, OHIO, US; 4 July 2016 (2016-07-04), XP002793754, retrieved from stn Database accession no. 1944825-80-2
- DATABASE pubchem [Online] ncbi; 22 April 2017 (2017-04-22), XP002793755, retrieved from pubchem Database accession no. 126539609

## Description

### FIELD OF THE INVENTION

The present invention relates to pharmacologically active spirochromane compounds, or pharmaceutically acceptable salts, racemates, enantiomers, diastereomers, solvates and hydrates thereof, as well as to pharmaceutical compositions containing them and to their use as modulators of a7 nicotinic acetylcholine receptor activity in a mammalian subject.

### BACKGROUND OF THE INVENTION

Acetylcholine (ACh) exerts its functions as a neurotransmitter in the mammalian central nervous system (CNS) by binding to cholinergic receptors. The mammalian CNS contains two predominant types of ACh receptors: muscarinic (mAChR) and nicotinic (nAChR) receptors, based on the agonist activities of muscarine and nicotine, respectively. Nicotinic acetylcholine receptors are ligand-gated ion channels made up of five subunits (Purves et al. Neuroscience 4th ed. (2008) 122-126). The subunits of the nicotinic receptors belong to a multigene family and have been divided into two groups based on their amino acid sequences; one containing alpha, and another containing beta subunits. Pentameric assemblies of different subunit combinations result in large number of receptor subtypes with various pharmacological properties. Assembly of the most broadly expressed subtypes include muscle-type ((α1)₂β₁δε), ganglion-type ((α3)₂(β4)₃) and CNS-type (α4)₂(β2)₃ or (α7)₅) nAChR subtypes (Le Novère N et al. Journal of Molecular Evolution 40 (1995) 155-172). α7 subunits have been shown to form functional receptors when expressed alone, and thus are presumed to form homooligomeric pentameric receptors.

Activation of the nAChR ion channel is primarily controlled by binding of ligands at conventional agonist binding sites, but is also regulated by either negative, or positive allosteric modulators (NAMs and PAMs). The allosteric transition state model of the nAChR involves at least a resting state, an activated state and a "desensitized" closed channel state, a process by which receptors become insensitive to the agonist. Different nAChR ligands can stabilize the conformational state of a receptor, to which they preferentially bind. For example, the agonists ACh and (-)-nicotine respectively stabilize the active and desensitized states. Changes of the activity of nicotinic receptors have been implicated in a number of diseases. Reductions in nicotinic receptors have been hypothesized to mediate cognitive deficits seen in diseases, such as Alzheimer's disease and schizophrenia. The effects of nicotine from tobacco are also mediated by nicotinic receptors, and since the effect of nicotine is to stabilize receptors in a desensitized state, an increased activity of nicotinic receptors may reduce the desire to smoke.

However, treatment with nicotinic receptor agonists, which act at the same site as ACh is problematic, because ACh not only activates, but also blocks receptor activity through processes, which include desensitization and uncompetitive blockade. Furthermore, prolonged activation appears to induce a long-lasting inactivation. Therefore, agonists of ACh can be expected to lose effectiveness upon chronic administration.

While the α7 nAChR is characterized by its fast activation kinetics and high permeability to Ca²⁺ compared to other subtypes (Delbono et al. J. Pharmacol. Exp. Ther. 280 (1997) 428-438), it also exhibits rapid desensitization following exposure to agonists at the orthosteric site (Castro et al. Neurosci. Lett. 164 (1993) 137-140; Couturier et al. Neuron 5 (1990) 847-856). In spite that development of a variety of a7-selective agonists and partial agonists has been carried out in the recent years, their clinical efficacy proved to be suboptimal, due to this receptor blockade (desensitisation) following the agonist activation. This problem may be overcomed by treatment with PAMs, enhancing α7 nAChR activation mediated by the endogenous agonist. The positive modulation of α7 nAChRs has been shown to have cognitive benefits in various preclinical models (Thomsen et al. Curr Pharm Des 16 (2010) 323-343; Lendvai et al. Brain Res Bull 93 (2013) 86-96).

The compounds of the present invention may be useful for the treatment of diseases and conditions mediated by, or associated to the positive allosteric modulation of the α7 nAChR, including, but not limited to psychotic disorders, for example schizophrenia (Deutsch SI et al. Schizophr Res 148 (2013) 138-144), schizophreniform disorder (Rowe AR et al. J Psychopharmacol 29 (2015) 197-211), schizoaffective disorder (Martin LF et al. Am J Med Genet B Neuropsychiatr Genet 144B (2007) 611-614), delusional disorder (Carson R et al. Neuromolecular Med 10 (2008) 377-384), brief psychotic disorder, psychotic disorder due to a general medical condition, substance-induced psychotic disorder, or psychotic disorder not otherwise specified, cognitive impairment including, for example the treatment of impairment of cognitive functions, as well as cognitive impairment as a result of stroke, Alzheimer's disease (Lewis AS et al. Prog Neuropsychopharmacol Biol Psychiatry 75 (2017) 45-53), Huntington's disease (Foucault-Fruchard L et al. Neural Regen Res 13 (2018) 737-741), Pick disease (Fehér A et al. Dement Geriatr Cogn Disord 28 (2009) 56-62), HIV associated dementia (Capó-Vélez CM et al. Sci Rep 8 (2018) 1829), frontotemporal dementia (Minami SS et al. Biochem Pharmacol 97 (2015) 454-462), Lewy body dementia (Perry EK et al. Neuroscience 64 (1995) 385-395), vascular dementia (Putignano S et al. Clin Interv Aging 7 (2012) 113-118), cerebrovascular disease (Si ML and Lee TJF Circ Res 91 (2002) 62-69), or other dementia states, and dementia associated to other degenerative disorders (amyotrophic lateral sclerosis (Kawamata et al. Ther Adv Chronic Dis 2 (2011) 197-208), etc.), other acute or sub-acute conditions that may cause cognitive decline, such as delirium (Sfera A et al. Front Med 2 (2015) 56), traumatic brain injury (Shin SS et al. Neural Regen Res 10 (2015) 1552-1554), senile dementia (Whitehouse PJ et et al. Science 215 (1982) 1237-1239), mild cognitive impairment (Ikonomovic MD et al. Arch Neurol 66 (2009) 646-651), Down's syndrome (Deutsch SI et al. Clin Neuropharmacol 26 (2003) 277-283), depression and cognitive deficit related to other diseases and dyskinetic disorders (Parameswaran N et al. Soc Neurosci Abstr (2007)), such as Parkinson's disease (Quik M et al. Biochem Pharmacol 97 (2015) 399-407), as well as neuroleptic-induced parkinsonism, or tardive dyskinesias (Terry AV and Gearhart DA Eur J Pharmacol 571 (2007) 29-32), depression and mood disorders, including depressive disorders and episodes (Philip NS et al. Psychopharmacology 212 (2010) 1-12), bipolar disorders (Leonard S and Freedman R. Biol Psychiatry 60 (2006) 115-122), cyclothymic disorder(Ancin I et al. J Affect Disord 133 (2011) 340-345), and bipolar disorder not otherwise specified, other mood disorders (Shytle RD et al. Depression and Anxiety 16 (2002) 89-92), substance-induced mood disorder and mood disorder not otherwise specified, anxiety disorders (Picciotto MR et al. Neuropharmacology 96 (2015) 235-243), panic disorder and panic attacks (Zvolensky MJ et al. Clin Psychol Rev 25 (2005) 761-789), obsessive compulsive disorder (Tizabi Yet al. Biol Psychiatry 51 (2002) 164-171), posttraumatic stress disorder (Sun R et al. Neuroscience 344 (2017) 243-254), acute stress disorder (Mineur YS et al. Neuropsychopharmacology 41 (2015) 1579-1587), generalized anxiety disorder (Cocores JA Prim Care Companion J Clin Psychiatry 10 (2008) 253-254), anxiety disorder due to a general medical condition, substance-induced anxiety disorder, phobias, and anxiety disorder not otherwise specified, substance related disorders for example substance use or substance-induced disorders, e.g., alcohol- (de Fiebre NC and de Fiebre CM Alcohol 31 (2003) 149-153; Diaper AM et al. Br J Clin Pharmacol 77 (2014) 302-314) nicotine- (Leslie FM et al. Mol Pharmacol 83 (2013) 753-758), amphetamine-(Pubill D et al. Pharmaceuticals 4 (2011) 822-847), phencyclidine- (Thomsen MS et al. Neuropharmacology 56 (2009) 1001-1009), opioid- (Zhang W, Int J Clin Exp Med 8 (2015) 1871-1879), cannabis- (Solinas M et al. J Neurosci 27 (2007) 5615-5620), cocaine- (Francis MM et al. Mol Pharmacol 60 (2001) 71-79), caffeine-, hallucinogen-, inhalant-, sedative-, hypnotic-, anxiolytic-, polysubstance- or other substance-related disorders; sleep disorders (McNamara JP et al. Psychol Health Med 19 (2014) 410-419) such as narcolepsy (Krahn et al J Clin Sleep Med 5 (2009) 390), dyssomnias, primary hypersomnia, breathing-related sleep disorders, circadian rhythm sleep disorder, and dyssomnia not otherwise specified; parasomnias, sleep terror disorder, sleepwalking disorder, and parasomnia not otherwise specified; sleep disorders related to another mental disorder (including insomnia related to another mental disorder and hypersomnia related to another mental disorder), sleep disorder due to a general medical condition and substance-induced sleep disorder; metabolic and eating disorders (Somm E Arch Immunol Ther Exp 62 (2014) 62: 87-101), such as anorexia nervosa (Cuesto G et al. J Neurogenet 31 (2017) 266-287), bulimia nervosa, obesity (Lakhan SE and Kirchgessner A J Transl Med 9 (2011) 129-139), compulsive eating disorder, binge eating disorder and eating disorder not otherwise specified; diabetes mellitus (Marrero MB et al. J Pharmacol Exp Ther 332 (2010) 173-180), ulcerative colitis (Salaga et al. JPET 356 (2016) 157-169), Crohn's disease (Bencherif M et al. Cell Mol Life Sci 68 (2011) 931-949), irritable bowel syndrome (Keszthelyi D et al. Neurogastroenterol Motil 21 (2009) 1239-1249), autism spectrum disorders (Deutsch et al. Clin Neuropharmacol 33 (2010) 114-120), including autistic disorder, Asperger's disorder, Rett's disorder, childhood disintegrative disorder and pervasive developmental disorder not otherwise specified; attention deficit hyperactivity disorder (Wilens TE and Decker MW Biochem Pharmacol 74 (2007) 1212-1223), disruptive behaviour disorders, oppositional defiant disorder and disruptive behaviour disorder not otherwise specified; and tic disorders such as Tourette's disorder (Gotti C and Clementi F Prog Neurobiol 74 (2004) 363-396), personality disorders (Kamens HM et al. Behav Genet 46 (2016) 693-704); sexual dysfunctions such as sexual desire disorders, sexual arousal disorders, orgasmic disorders, sexual pain disorder, sexual dysfunction not otherwise specified, paraphilias, gender identity disorders, infertility (Bray C et al. Biol Reprod 73 (2005) 807-814), premenstrual syndrome (Gündisch D and Eibl C Expert Opin Ther Pat 21 (2011) 1867-1896), and sexual disorders not otherwise specified, disorders of the respiratory system like cough (Canning BJ Am J Respir Crit Care Med 195 (2017) A4498), asthma (Santana FPR et al. Eur Respir J 48 (2016) PA5066), chronic obstructive pulmonary disease (Maouche K et al. Proc Natl Acad Sci USA 110 (2013) 4099-4104), lung inflammation (Enioutina EY et al. PLoS One 10 (2015) e0121128), disorders of the cardiovascular system such as cardiac failure (Mai XK et al. J Immunol 200 (2018) 108.11), heart arrhythmia (Mazloom R et al. PLoS One 8 (2013) e82251), and hypertension (Chen JK et al. BMC Cardiovasc Disord 12 (2012) 38).

The compounds of the invention are also useful in treating inflammation, inflammatory and neuropathic pain (Alsharari SD et al. Biochem Pharmacol 86 (2013) 1201-1207), rheumatoid arthritis (van Maanen MA et al. Arthritis & Rheumatism 60 (2009) 1272-1281), osteoarthritis (Lee SE Neurosci Lett 548 (2013) 291-295), allergy (Yamamoto T et al. PLoS One 9 (2014) e85888), sarcoidosis (Nicotine Treatment for Pulmonary Sarcoidosis: A Clinical Trial Pilot Study Elliott Crouser MD, Principal Investigator, Ohio State University ClinicalTrials.gov Identifier: NCT02265874), psoriasis (Westman M et al. Scand J Immunol 70 (2009) 136-140), ataxia (Taslim N et al. Behav Brain Res 217 (2011) 282-292), dystonia (Zimmerman CN et al. Front Syst Neurosci 11 (2017) 43), systemic lupus erythematosus (Fairley AS and Mathis KW Physiol Rep 5 (2017) e13213), mania (Janowsky DS et al. Lancet 2 (1972) 632-635), restless legs syndrome (Buchfuhrer MJ Neurotherapeutics 9 (2012) 776-790), progressive supranuclear palsy (Warren NM et al. Brain 128 (2005) 239-245), epilepsy (Bertrand D Epilepsy Curr 2 (2002) 191-193), myoclonus (Leppik IE Epilepsia 44 (2003) 2-6), migraine (Liu Q et al. J Pain Res 11 (2018) 1129-1140), amnesia (Bali Zs K et al. Front Cell Neurosci 11 (2017) 271), chronic fatigue syndrome (Shan ZY et al. J Magn Reson Imaging 44 (2016) 1301-1311), cataplexy (Ebben MR and Krieger AC J Clin Sleep Med 8 (2012) 195-196), brain ischemia (Han Z et al. J Neurochem 131 (2014) 498-508), multiple sclerosis (Di Bari M et al. Cent Nerv Syst Agents Med Chem 17 (2017) 109-115), encephalomyelitis (Hao J et al. Exp Neurol 227 (2011): 110-119), jetlag (Shi M et al. eLife 3 (2014) e01473), cerebral amyloid angiopathy (Clifford PM et al. Brain Res 1234 (2008) 158-171), sepsis (Ren C et al. Int J Biol Sci 14 (2018) 748-759), and in general, in treating all types of diseases and disorders connected to the positive allosteric modulation of the α7 nAChR.

Furthermore, these compounds can also be combined with other therapeutic agents including, but not limited to acetylcholinesterase inhibitors (such as galantamine, rivastigmine, donepezil, tacrine, phenserine, ladostigil and ABT-089); NMDA receptor agonists or antagonists (such as memantine, neramexane, EVT101 and AZD4282); anti-amyloid antibodies including anti-amyloid humanized monoclonal antibodies (such as bapineuzumab, ACCOO1, CAD 106, AZD3102, H12A11V1); beta- (such as verubecestat, and AZD3293) or gamma-secretase inhibitors (such as LY450139 and TAK 070) or modulators; tau phosphorylation inhibitors; ApoE4 conformation modulators; p25/CDK5 inhibitors; NK1/NK3 receptor antagonists; COX-2 inhibitors (such as celecoxib, rofecoxib, valdecoxib, 406381 and 644784); LRRK2 inhibitors; HMG-CoA reductase inhibitors; NSAIDs (such as ibuprofen); vitamin E; glycine transport inhibitors; glycine site antagonists (such as lacosamide); LXR β agonists; androgen receptor modulators; blockers of Aβ oligomer formation; NR2B antagonists, anti-inflammatory compounds (such as (R)-flurbiprofen, nitroflurbiprofen, ND-1251, VP-025, HT-0712, and EHT-202); PPAR gamma agonists (such as pioglitazone and rosiglitazone); CB-1 receptor antagonists or inverse agonists (such as AVE1625); CB-2 agonists (such as 842166 and SAB378); VR-1 antagonists (such as AMG517, 705498, 782443, PAC20030, VI 14380 and A425619); bradykinin Bl receptor antagonists (such as SSR240612 and NVPSAA164); sodium channel blockers and antagonists (such as VX409 and SPI860); NOS inhibitors (such as SD6010 and 274150); antibiotics; growth hormone secretagogues (such as ibutamoren, ibutamoren mesylate, and capromorelin); potassium channel openers; AMPA agonists or AMPA modulators (such as CX-717, LY 451395, LY404187 and S-18986); GSK3 inhibitors (such as AZD1080, SAR502250 and CEP16805); neuronal nicotinic agonists; MARK ligands; M₁ or M₄ mAChR agonists or PAMs; mGluR2 antagonists or NAMs or PAMs; mGluR5 antagonists (such as AZD9272); alpha-adrenerg agonists; ADAM-10 ligands; sedatives, hypnotics, anxiolytics, antipsychotics, cyclopyrrolones, imidazopyridines, pyrazolopyrimidines, minor tranquilizers, melatonin agonists and antagonists, melatonergic agents; orexin antagonists and agonists; prokineticin agonists and antagonists; T-type calcium channel antagonists; triazolopyridines benzodiazepines, barbiturates; 5-HT_{1A} antagonists (such as lecozotan); 5-HT₂ antagonists; 5-HT₄ agonists (such as PRX-03140); 5-HT₆ antagonists (such as GSK 742467, SGS-518, FK-962, SL-65.0155, SRA- 333 and xaliproden); histamine H₃ receptor antagonists and inverse agonists (such as S38093, ABT-834, ABT 829, GSK 189254 and CEP16795); PDE₄ inhibitors (such as HT0712); PDE₉ inhibitors (such as BI40936); PDE₁₀ inhibitors; HDAC inhibitors; KCNQ antagonists; GABA_{A} inverse agonists; GABA signalling enhancers; GABA agonists, GABA_{A} receptor alpha5 subunit NAMs or PAMs, antipsychotics; MAO-B inhibitors; dopamine transport inhibitors; noradrenaline transport inhibitors; D₂ agonists and partial agonists; anticholinergics (such as biperiden); COMT inhibitors (such as entacapone); A2a adenosine receptor antagonists; cholinergic agonists; compounds from the phenothiazine, thioxanthene (such as chlorprothixene and thiothixene), heterocyclic dibenzazepine (such as clozapine), butyrophenone (such as haloperidol), diphenylbutylpiperidine (such as pimozide) and indolone (such as molindolone) classes of neuroleptic agents; loxapine, sulpiride and risperidone; levodopa; calcium channel blockers (such as ziconotide and NMED160); MMP inhibitors; thrombolytic agents; opioid analgesics (such as codeine, fentanyl, hydromorphone, levorphanol, meperidine, methadone, morphine, oxycodone, oxymorphone, pentazocine, propoxyphene); pramipexole; ropinirole; neutrophil inhibitory factor; SSRIs or SSNRIs; tricyclic antidepressant drugs; norepinephrine modulators; lithium; valproate; gabapentin; pregabalin; rizatriptan; zolmitriptan; naratriptan and sumatriptan or other drugs that affect receptors or enzymes that either increase the efficacy, safety, convenience, or reduce unwanted side effects or toxicity of the compounds of the present invention.

Known positive allosteric modulators of the a7 nicotinic acetylcholine receptor include 2-aniline-4-aryl thiazole derivatives (WO 2007/031440 A2, JANSSEN PHARMACEUTICA NV), amide derivatives (WO 2009/100294 A2, ABBOT LAB.), trisubstituted 1,2,4-triazoles (WO 2009/115547 A1, JANSSEN PHARMACEUTICA NV), indole derivatives (WO 2009/127678 A1, GLAXO GROUP LTD. and WO 2009/127679 A1, GLAXO GROUP LTD.), tetrazole-substituted aryl amide derivatives (WO 2009/043780 A1, HOFFMANN LA ROCHE), cyclopropyl aryl amide derivatives (WO 2009/043784 A1, HOFFMANN LA ROCHE), trisubstiuted pyrazoles (WO 2009/135944 A1, JANSSEN PHARMACEUTICA NV), pyrrole derivatives (WO 2014/141091 A1, LUPIN LTD), cyclopropylbenzene derivatives (WO 2017/165256 A1, MERCK SHARP & DOHME CORP.), and substituted bicyclic heteroaryl derivatives (WO 2018/085171 A1, MERCK SHARP & DOHME CORP.).

The present invention is directed to a novel class of compounds that exhibit positive allosteric modulation of the a7 nicotinic acetylcholine receptor.

### BRIEF DESCRIPTION OF THE DRAWINGS

An exemplary embodiment of the present invention is illustrated by way of example in the accompanying drawings in which like reference numbers indicate the same or similar elements and in which:

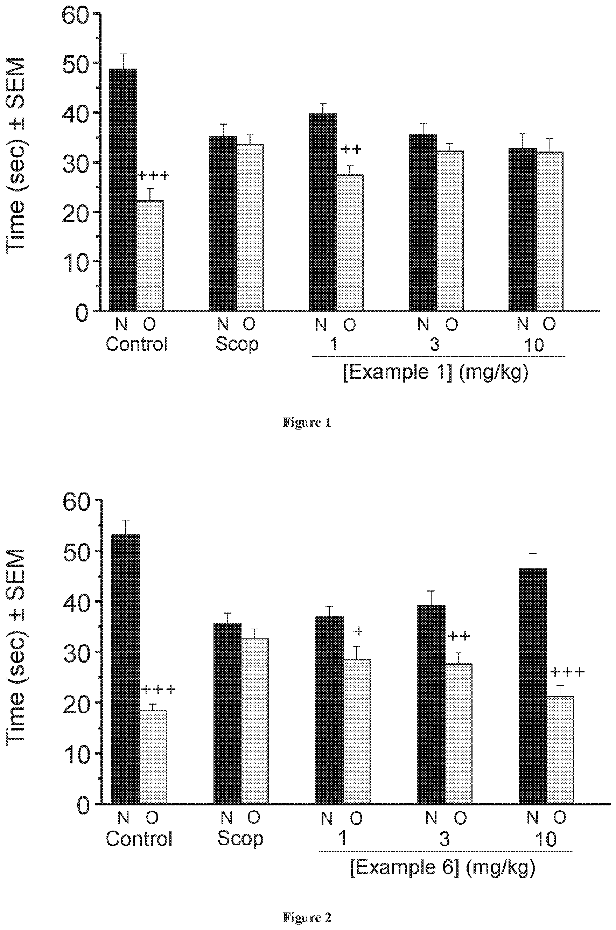

Figure 1 illustrates the results of place recognition test of compound Example 1. Exploration times spent in the novel [N] vs. familiar [O] arms of the Y maze are depicted). Scop: scopolamine (1 mg/kg, ip.). ⁺p<0.05; ⁺⁺p<0.01; ⁺⁺⁺p<0.001.
Figure 2 illustrates the results of place recognition test of compound Example 6. Exploration times spent in the novel [N] vs. familiar [O] arms of the Y maze are depicted). Scop: scopolamine (1 mg/kg, ip.). ⁺p<0.05; ⁺⁺p<0.01; ⁺⁺⁺p<0.001.

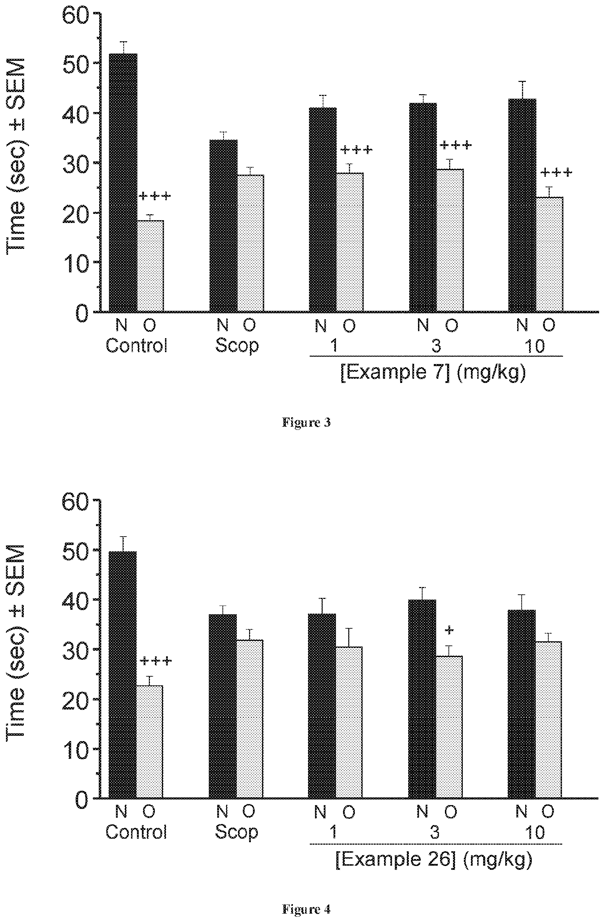

Figure 3 illustrates the results of place recognition test of compound Example 7. Exploration times spent in the novel [N] vs. familiar [O] arms of the Y maze are depicted). Scop: scopolamine (1 mg/kg, ip.). ⁺p<0.05; ⁺⁺p<0.01; ⁺⁺⁺p<0.001.
Figure 4 illustrates the results of place recognition test of compound Example 26. Exploration times spent in the novel [N] vs. familiar [O] arms of the Y maze are depicted). Scop: scopolamine (1 mg/kg, ip.). ⁺p<0.05; ⁺⁺p<0.01; ⁺⁺⁺p<0.001.

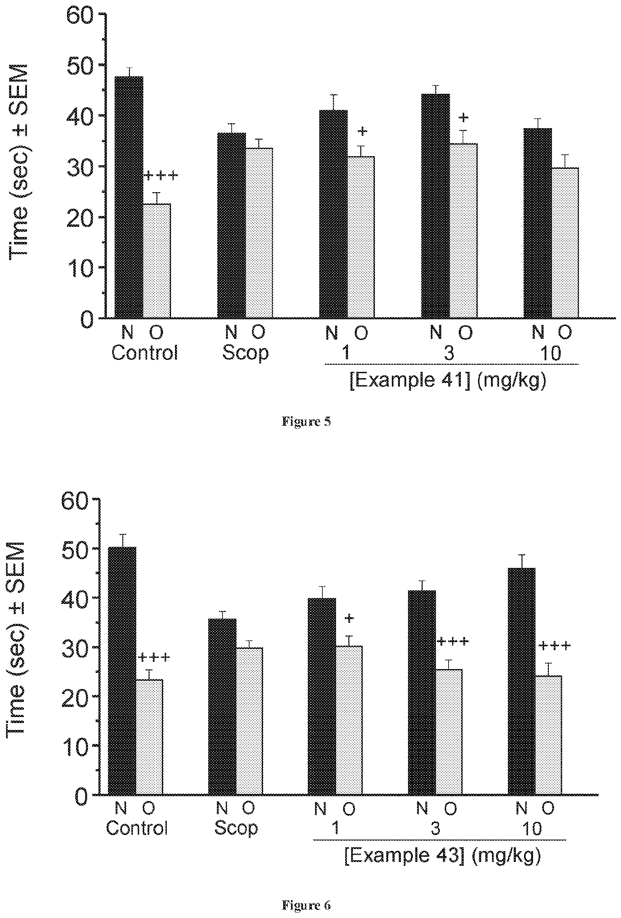

Figure 5 illustrates the results of place recognition test of compound Example 41. Exploration times spent in the novel [N] vs. familiar [O] arms of the Y maze are depicted). Scop: scopolamine (1 mg/kg, ip.). ⁺p<0.05; ⁺⁺p<0.01; ⁺⁺⁺p<0.001.
Figure 6 illustrates the results of place recognition test of compound Example 43. Exploration times spent in the novel [N] vs. familiar [O] arms of the Y maze are depicted). Scop: scopolamine (1 mg/kg, ip.). ⁺p<0.05; ⁺⁺p<0.01; ⁺⁺⁺p<0.001.

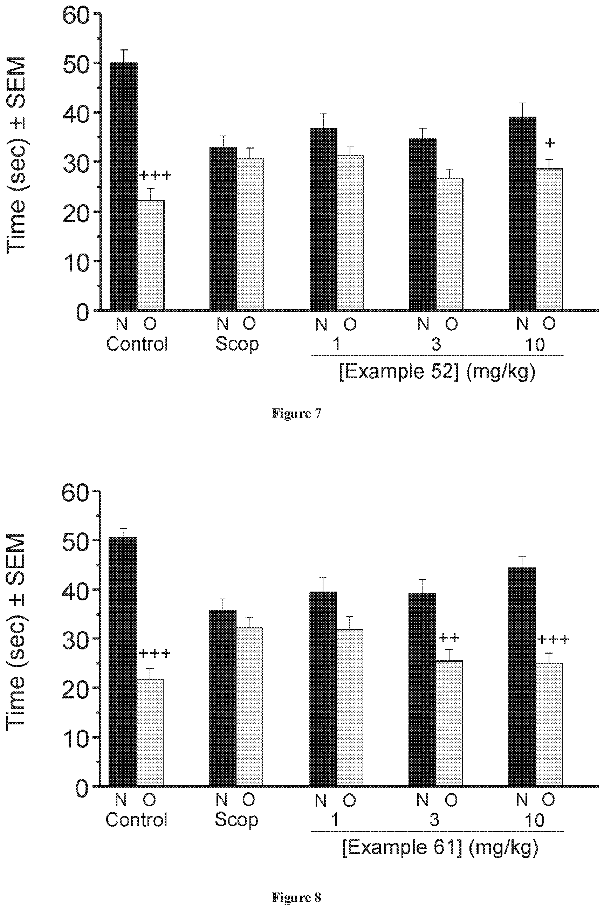

Figure 7 illustrates the results of place recognition test of compound Example 52. Exploration times spent in the novel [N] vs. familiar [O] arms of the Y maze are depicted). Scop: scopolamine (1 mg/kg, ip.). ⁺p<0.05; ⁺⁺p<0.01; ⁺⁺⁺p<0.001.
Figure 8 illustrates the results of place recognition test of compound Example 61. Exploration times spent in the novel [N] vs. familiar [O] arms of the Y maze are depicted). Scop: scopolamine (1 mg/kg, ip.). ⁺p<0.05; ⁺⁺p<0.01; ⁺⁺⁺p<0.001.

### SUMMARY OF THE INVENTION

The present invention relates to compounds of formula (I) for use in the treatment or prevention of a disease associated with alpha 7 nicotinic acetylcholine receptor activity 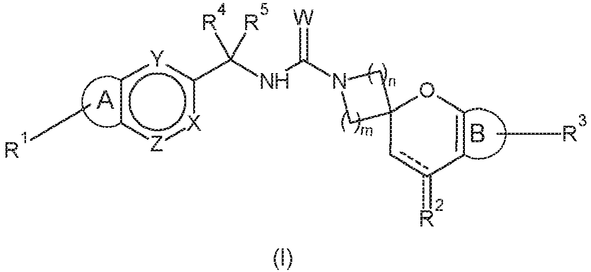 wherein
**A** is a five or six membered heterocycle;
**B** is a six membered carbocycle or heterocycle;
**X** is Cor N;
**Y** is C or N;
**Z** is Cor N;
**W** is O or S;
**R¹** is H, C₁₋₆alkyl, halogen or haloC₁₋₆alkyl;
**R²** is H or O;
**R³** is H, C₁₋₆alkyl, halogen, haloC₁₋₆alkyl or C₁₋₆alkoxy;
**R⁴** is H or C₁₋₆alkyl;
**R⁵** is H or C₁₋₆alkyl;
**n and m** are independently 1 or 2;
--- is a single - or double-bond;
or pharmaceutically acceptable salts, racemates, enantiomers, diastereomers, solvates and hydrates thereof.

In a further aspect, the present invention relates to compounds of formula (II), 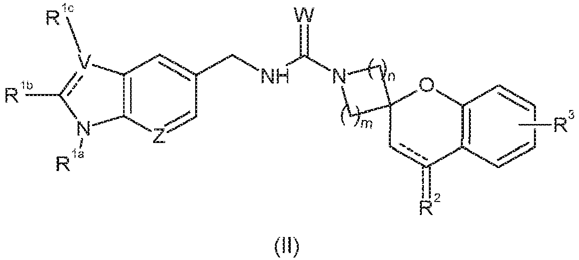 wherein
**V** is C or S;
**Z** is Cor N;
**W** is O or S;
**R^{1a}** is H, C₁₋₆alkyl, or haloC₁₋₆alkyl;
**R^{1b}** is H, C₁₋₆alkyl, halogen, or haloC₁₋₆alkyl;
**R**^{**1**c} is H, C₁₋₆alkyl, halogen, or haloC₁₋₆alkyl when V is C; or R^{1c} is absent when V is S;
**R¹** is H or O;
**R³** is H, C₁₋₆alkyl, halogen, haloC₁₋₆alkyl, or C₁₋₆alkoxy;
**n and m**: independently 1 or 2;
--- is a single - or double-bond;
or pharmaceutically acceptable salts, racemates, enantiomers, diastereomers, solvates and hydrates thereof.

In a further aspect, the present invention provides a compound of formula (II), as defined above for use in the treatment or prevention of a disease associated with a7 nicotinic acetylcholine receptor activity.

In a further aspect, the present invention provides the use of a compound of formula (I) or formula (II), as defined above, for the manufacture of a medicament for the treatment or prevention of a disease associated with a7 nicotinic acetylcholine receptor activity.

In a further aspect, the compounds of formula (I) or formula (II), as defined above, can be administered in combination with other compounds used for the treatment or prevention of a disease associated with a7 nicotinic acetylcholine receptor activity.

In a further aspect, the present invention provides a process for the manufacture of the compounds of formula (II).

### DETAILED DESCRIPTION OF THE INVENTION

The present invention relates to compounds of formula (I) for use in the treatment or prevention of a disease associated with alpha 7 nicotinic acetylcholine receptor activity 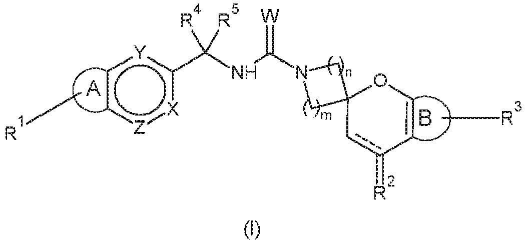 wherein:
**A** is a five or six membered heterocycle;
**B** is a six membered carbocycle or heterocycle;
**X** is Cor N;
**Y** is Cor N;
**Z** is Cor N;
**W** is O or S;
**R¹** is H, C₁₋₆alkyl, halogen or haloC₁₋₆alkyl;
**R²** is H or O;
**R³** is H, C₁₋₆alkyl, halogen, haloC₁₋₆alkyl or C₁₋₆alkoxy;
**R⁴** is H or C₁₋₆alkyl;
**R⁵** is H or C₁₋₆alkyl;
**n and m** are independently 1 or 2;
--- is a single - or double-bond;
or pharmaceutically acceptable salts, racemates, enantiomers, diastereomers, solvates and hydrates thereof.

The term "five membered heterocycle", as used herein, refers to to an optionally substituted saturated, unsaturated or aromatic ring system having five atoms and incorporating one, two, three or four heteroatoms (chosen from nitrogen, oxygen or sulfur). Examples of five membered heterocyclyc moieties include, but are not limited to, pyrrolydinyl, pyrrolyl, tetrahydrofuryl, dihydrofuryl, furyl, tetrahydrothiophenyl, thiophenyl, imidazolidinyl, imidazolyl, pyrazolidinyl, pyrazolyl, oxazolidinyl, isoxazolidinyl, oxazolyl, isoxazolyl, thiazolidinyl, isothiazolidinyl, thiazolyl, isothiazolyl, dioxolanyl, dithiolanyl, triazolyl, oxadiazolyl, thiadiazolyl, tetrazolyl.

The term "six membered heterocycle", as used herein, refers to an optionally substituted saturated, unsaturated or aromatic ring system having six atoms and incorporating one, two, three or four heteroatoms (chosen from nitrogen, oxygen or sulfur). Examples of six membered heterocycles include, but are not limited to, piperidinyl, pyridinyl, pyridazinyl, pyrimidinyl, dihydropyranyl, tetrahdydropyranyl, pyranyl, thiopyranyl, piperazinyl, homopiperazinyl, morpholinyl, thiomorpholinyl.

The term "six membered carbocycle" as used herein, refers to an optionally substituted saturated, unsaturated or aromatic ring system having six carbon atoms including, cyclohexyl, cyclohexenyl, cyclohexadienyl, and phenyl.

The term "halo" or "halogen", as used herein as such or as part of another group, refers to fluoro, chloro, bromo or iodo.

The term "C₁₋₆alkyl", as used herein as such or as part of another group, refers to a branched or straight chain saturated hydrocarbon group having one, two, three, four, five or six carbon atoms including, but not limited to, methyl, ethyl, *n*-propyl, *i*-propyl, *n*-butyl, *sec*-butyl, and *tert*-butyl.

The term "haloC₁₋₆alkyl", as used herein, refers to at least one halogen, as defined above, bonded to the parent molecular moiety through an "C₁₋₆alkyl" group, as defined above. When there are several halogens, the halogens can be identical or different and the halogens can be attached to different carbon atoms or several halogens can be attached to the same carbon atom. HaloC₁₋₆alkyl groups include, but are not limited to, difluoromethyl, trifluoromethyl and 2-chloroethyl.

The term "C₁₋₆alkoxy", as used herein refers to an "C₁₋₆alkyl" group, as defined above, bonded to the parent molecular moiety through an oxygen atom including, but not limited to, methoxy, ethoxy, *n*-propoxy, *i*-propoxy and *tert*-butoxy.

The term "pharmaceutically acceptable" describes an ingredient that is useful in preparing a pharmaceutical composition, is generally safe, non-toxic and neither biologically nor otherwise undesirable, and includes those acceptable for veterinary use as well as human pharmaceutical use.

The term " pharmaceutically acceptable salt" refers a conventional acid addition salt or a base addition salt, which preserves the biological efficacy and properties of the compounds of formula (I) or formula (II) and which can be formed with suitable non-toxic organic or inorganic acids or organic or inorganic bases. Examples of acid addition salts include salts derived from inorganic acids, such as, but not limited to, hydrochloric acid, hydrobromic acid, hydroiodic acid, sulfuric acid, sulphamic acid, phosphoric acid, nitric acid and perchloric acid and derived from various organic acids, such as, but not limited to, acetic acid, propionic acid, benzoic acid, glycolic acid, phenylacetic acid, salicylic acid, malonic acid, maleic acid, oleic acid, pamoic acid, palmitic acid, benzenesulfonic acid, toluenesulfonic acid, methanesulfonic acid, oxalic acid, tartaric acid, succinic acid, citric acid, malic acid, lactic acid, glutamic acid, fumaric acid and the like. Examples of base addition salts are salts derived from ammonium-, potassium-, sodium- and quaternary ammonium hydroxides such as tetramethylammonium hydroxide.

The term "hydrate" means non-covalent combinations between water and solute.

The term "solvate" means non-covalent combinations between solvent and solute. Solvents include, but are not limited to, ethanol, 2-propanol, acetonitrile and tetrahydrofuran.

"Optional" or "optionally" means that the subsequently described event or circumstance may but need not occur, and that the description includes instances where the event or circumstance occurs and instances in which it does not.

"Treating" or "treatment" of a disease state includes:
a) preventing the disease state, i.e. causing the clinical symptoms of the disease state not to develop in a subj ect that may be exposed to or predisposed to the disease state, but does not yet experience or display symptoms of the disease state,
b) inhibiting the disease state, i.e., arresting the development of the disease state or its clinical symptoms, or
c) relieving the disease state, i.e., causing temporary or permanent regression of the disease state or its clinical symptoms.

In one embodiment, the present invention relates to compounds of formula (I) for use in the treatment or prevention of a disease associated with alpha 7 nicotinic acetylcholine receptor activity, wherein
**A** is five membered heterocycle, wherein the members of the ring are selected from the group consisting of carbon, nitrogen, oxygen, and sulphur;
**B** is a six membered carbocycle or a heterocycle, wherein the members of the ring are selected from the group consisting of carbon, nitrogen, oxygen, and sulphur;
**X** is C;
**Y** is C;
**Z** is Cor N;
**W** is O or S;
**R¹** is H, C₁₋₆alkyl, halogen or haloC₁₋₆alkyl;
**R²** is H or O;
**R³** is H, C₁₋₆alkyl, halogen, haloC₁₋₆alkyl or C₁₋₆alkoxy;
**R⁴** is H;
**R⁵** is H;
**n and m** are independently 1 or 2;
--- is a single - or double-bond;
or pharmaceutically acceptable salts, racemates, enantiomers, diastereomers, solvates and hydrates thereof.

In one embodiment, the present invention relates to compounds of formula (II), 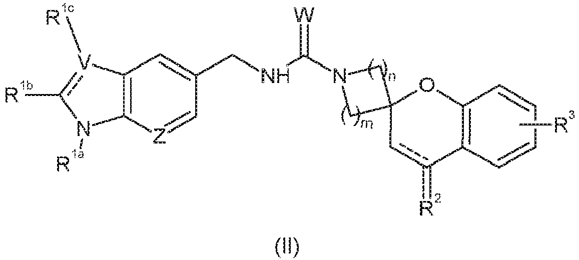 wherein
**V** is C or S;
**Z** is Cor N;
**W** is O or S;
**R^{1a}** is H, C₁₋₆alkyl, or haloC₁₋₆alkyl;
**R^{1b}** is H, C₁₋₆alkyl, halogen, or haloC₁₋₆alkyl;
**R^{1c}** is H, C₁₋₆alkyl, halogen, or haloC₁₋₆alkyl when V is C; or R^{1c} is absent when V is S; **R²** is H or O;
**R³** is H, C₁₋₆alkyl, halogen, haloC₁₋₆ alkyl, or C₁₋₆alkoxy;
**n and m** are independently 1 or 2;
--- is a single - or double-bond;
or pharmaceutically acceptable salts, racemates, enantiomers, diastereomers, solvates and hydrates thereof.

In one embodiment, the present invention relates to compounds of formula (II), wherein
**V** is C or S;
**Z** is C or N;
**W** is O or S;
**R^{1a}** is H, C₁₋₄alkyl, or haloC₁₋₄alkyl;
**R^{1b}** is H, C₁₋₄alkyl, halogen, or haloC₁₋₄alkyl;
**R^{1c}** is H, C₁₋₄alkyl, halogen, or haloC₁₋₄alkyl when V is C; or R^{1c} is absent when V is S;
**R²** is H;
**R³** is H, C₁₋₄alkyl, halogen, haloC₁₋₄alkyl, or C₁₋₄alkoxy;
**n and m** are independently 1 or 2;
--- is a single bond when it is attached to R², and a single - or double bond within the ring; or pharmaceutically acceptable salts, racemates, enantiomers, diastereomers, solvates and hydrates thereof.

In one embodiment, the present invention relates to compounds of formula (II), wherein
**V** is C or S;
**Z** is Cor N;
**W** is O or S;
**R^{1a}** is H, C₁₋₄alkyl, or haloC₁₋₄alkyl;
**R^{1b}** is H, C₁₋₄alkyl, halogen, or haloC₁₋₄alkyl;
**R^{1c}** is H, C₁₋₄alkyl, halogen, or haloC₁₋₄alkyl when V is C; or R^{1c} is absent when V is S;
**R²** is H;
**R³** is H, C₁₋₄alkyl, halogen, haloC₁₋₄alkyl, or C₁₋₄alkoxy;
**n and m** are 1;
--- is a single bond when it is attached to R², and a single - or double bond within the ring;
or pharmaceutically acceptable salts, racemates, enantiomers, diastereomers, solvates and hydrates thereof.

In one embodiment, the present invention relates to compounds of formula (II), wherein
**V** is C or S;
**Z** is Cor N;
**W** is O or S;
**R^{1a}** is H, C₁₋₄alkyl, or hatoC₁₋₄alkyl;
**R^{1b}** is H, C₁₋₄alkyl, halogen, or haloC₁₋₄alkyl;
**R^{1c}** is H, C₁₋₄alkyl, halogen, or haloC₁₋₄alkyl when V is C; or R^{1c} is absent when V is S;
**R²** is H;
**R³** is H, C₁₋₄alkyl, halogen, haloC₁₋₄alkyl, or C₁₋₄alkoxy;
**n and m** are 2;
--- is a single bond when it is attached to R², and a single - or double bond within the ring; or pharmaceutically acceptable salts, racemates, enantiomers, diastereomers, solvates and hydrates thereof.

In one embodiment, the present invention relates to compounds of formula (II), wherein
**V** is C or S;
**Z** is C or N;
**W** is O or S;
**R^{1a}** is H, C₁₋₄alkyl, or hatoC₁₋₄alkyl;
**R^{1b}** is H, Ci-aalkyl, halogen, or haloC₁₋₄alkyl;
**R^{1c}** is H, C₁₋₄alkyl, halogen, or haloC₁₋₄alkyl when V is C; or R^{1c} is absent when V is S;
**R²** is H,
**R³** is H, C₁₋₄alkyl, halogen, haloC₁₋₄alkyl, or C₁₋₄alkoxy;
**n** is 1;
**m** is 2;
--- is a single bond when it is attached to R², and a single - or double bond within the ring;
or pharmaceutically acceptable salts, racemates, enantiomers, diastereomers, solvates and hydrates thereof.

In one embodiment, the present invention relates to compounds of formula (II), wherein
**V** is C or S;
**Z** is Cor N;
**W** is O or S;
**R^{1a}** is H, C₁₋₄alkyl, or haloC₁₋₄alkyl;
**R^{1b}** is H, C₁₋₄alkyl, halogen, or haloC₁₋₄alkyl;
**R^{1c}** is H, Ci-aalkyl, halogen, or haloC₁₋₄alkyl when V is C; or R^{1c} is absent when V is S;
**R²** is O;
**R³** is H, C₁₋₄alkyl, halogen, haloC₁₋₄alkyl, or C₁₋₄alkoxy;
**n and m** are independently 1 or 2;
--- is a double bond when it is attached to R², and a single bond within the ring;
or pharmaceutically acceptable salts, racemates, enantiomers, diastereomers, solvates and hydrates thereof.

In one embodiment, the present invention relates to compounds of formula (II), wherein
**V** is C or S;
**Z** is C or N;
**W** is O or S;
**R^{1a}** is H, C₁₋₄alkyl, or hatoC₁₋₄alkyl;
**R^{1b}** is H, Ci-aalkyl, halogen, or haloC₁₋₄alkyl;
**R^{1c}** is H, C₁₋₄alkyl, halogen, or haloC₁₋₄alkyl when V is C; or R^{1c} is absent when V is S;
**R²** is O;
**R³** is H, C₁₋₄alkyl, halogen, haloC₁₋₄alkyl, or C₁₋₄alkoxy;
**η and m** are 1;
--- is a double bond when it is attached to R², and a single bond within the ring;
or pharmaceutically acceptable salts, racemates, enantiomers, diastereomers, solvates and hydrates thereof.

In one embodiment, the present invention relates to compounds of formula (II), wherein
**V** is C or S;
**Z** is Cor N;
**W** is O or S;
**R^{1a}** is H, C₁₋₄alkyl, or haloC₁₋₄alkyl;
**R^{1b}** is H, Ci-aalkyl, halogen, or haloC₁₋₄alkyl;
**R^{1c}** is H, C₁₋₄alkyl, halogen, or haloC₁₋₄alkyl when V is C; or R^{1c} is absent when V is S;
**R²** is O;
**R³** is H, C₁₋₄alkyl, halogen, haloC₁₋₄alkyl, or C₁₋₄alkoxy;
**n and m** are 2;
--- is a double bond when it is attached to R², and a single bond within the ring;
or pharmaceutically acceptable salts, racemates, enantiomers, diastereomers, solvates and hydrates thereof.

In one embodiment, the present invention relates to compounds of formula (II), wherein
**V** is C or S;
**Z** is Cor N;
**W** is O or S;
**R^{1a}** is H, C₁₋₄alkyl, or hatoC₁₋₄alkyl;
**R^{1b}** is H, C₁₋₄alkyl, halogen, or haloC₁₋₄alkyl;
**R^{1c}** is H, C₁₋₄alkyl, halogen, or haloC₁₋₄alkyl when V is C; or R^{1c} is absent when V is S;
**R²** is O;
**R³** is H, C₁₋₄alkyl, halogen, haloC₁₋₄alkyl, or C₁₋₄alkoxy;
**n** is 1;
**m** is 2;
--- is a double bond when it is attached to R², and a single bond within the ring;
or pharmaceutically acceptable salts, racemates, enantiomers, diastereomers, solvates and hydrates thereof.

In one embodiment, the present invention relates to compounds of formula (II), wherein
**V** is C;
**Z** is Cor N;
**W** is O;
**R^{1a}** is H, C₁₋₆alkyl, or haloC₁₋₆alkyl;
**R^{1b}** is H, C₁₋₆alkyl, halogen, or haloC₁₋₆alkyl;
**R^{1c}** is H, C₁₋₆alkyl, halogen, or haloC₁₋₆alkyl;
**R²** is H or O;
**R³** is H, C₁₋₆alkyl, halogen, or haloC₁₋₆alkyl;
**n and m** are independently 1 or 2;
--- is a single - or double-bond;
or pharmaceutically acceptable salts, racemates, enantiomers, diastereomers, solvates and hydrates thereof.

In one embodiment, the present invention relates to compounds of formula (II), wherein
**V** is C;
**Z** is Cor N;
**W** is O;
**R^{1a}** is H, C₁₋₄alkyl, or haloC₁₋₄alkyl;
**R^{1b}** is H, C₁₋₄alkyl, halogen, or haloC₁₋₄alkyl;
**R^{1c}** is H, C₁₋₄alkyl, halogen, or haloC₁₋₄alkyl;
**R²** is H or O;
**R³** is H, C₁₋₄alkyl, halogen, or haloC₁₋₄alkyl;
**n and m** are independently 1 or 2;
--- is a single - or double-bond;
or pharmaceutically acceptable salts, racemates, enantiomers, diastereomers, solvates and hydrates thereof.

In one embodiment, the present invention relates to compounds of formula (II), wherein
**V** is C;
**Z** is C or N;
**W** is O or S;
**R^{1a}** is H, C₁₋₄alkyl, or hatoC₁₋₄alkyl;
**R^{1b}** is H, Ci-aalkyl, halogen, or haloC₁₋₄alkyl;
**R^{1c}** is H, C₁₋₄alkyl, halogen, or haloC₁₋₄alkyl;
**R²** is O;
**R³** is H, C₁₋₄alkyl, halogen, haloC₁₋₄alkyl, or C₁₋₄alkoxy;
**n and m** are 1;
--- is a double bond when it is attached to R², and a single bond within the ring;
or pharmaceutically acceptable salts, racemates, enantiomers, diastereomers, solvates and hydrates thereof.

In one embodiment, the present invention relates to compounds of formula (II), wherein
**V** is C;
**Z** is Cor N;
**W** is O or S;
**R^{1a}** is H, C₁₋₄alkyl, or haloC₁₋₄alkyl;
**R^{1b}** is H, C₁₋₄alkyl, halogen, or haloC₁₋₄alkyl;
**R^{1c}** is H, C₁₋₄alkyl, halogen, or haloC₁₋₄alkyl;
**R²** is O;
**R³** is H, C₁₋₄alkyl, halogen, haloC₁₋₄alkyl, or C₁₋₄alkoxy;
**n and m** are 2;
--- is a double bond when it is attached to R², and a single bond within the ring;
or pharmaceutically acceptable salts, racemates, enantiomers, diastereomers, solvates and hydrates thereof.

In one embodiment, the present invention relates to compounds of formula (II), wherein
**V** is C;
**Z** is C or N;
**W** is O or S;
**R^{1a}** is H, C₁₋₄alkyl, or haloC₁₋₄alkyl;
**R^{1b}** is H, C₁₋₄alkyl, halogen, or haloC₁₋₄alkyl;
**R^{1c}** is H, Ci-aalkyl, halogen, or haloC₁₋₄alkyl
**R²** is O;
**R³** is H, C₁₋₄alkyl, halogen, haloC₁₋₄alkyl, or C₁₋₄alkoxy;
**n** is 1;
**m** is 2;
--- is a double bond when it is attached to R², and a single bond within the ring;
or pharmaceutically acceptable salts, racemates, enantiomers, diastereomers, solvates and hydrates thereof.

In one embodiment, the present invention relates to compounds of formula (I) or formula (II) selected from the group of:
6'-Fluoro-*N*-[(2-methyl-1*H*-indol-5-yl)methyl]-4'-oxo-3',4'-dihydrospiro[azetidine-3,2'-[1]benzopyran]-1-carboxamide;
6-chloro-*N*-[(1-methyl-1*H*-indol-5-yl)methyl]-4-oxo-3,4-dihydrospiro[1-benzopyran-2,4'-piperidine]-1'-carboxamide;
6'-chloro-*N*-[(1-methyl-1*H*-indol-5-yl)methyl]-4'-oxo-3',4'-dihydrospiro[azetidine-3,2'-[1]benzopyran]-1-carboxamide;
7'-fluoro-*N*-[(1-methyl-1*H-*indol-5-yl)methyl]-4'-oxo-3',4'-dihydrospiro[azetidine-3,2'-[1]benzopyran]-1-carboxamide;
*N*-[(1-methyl-1*H-*indol-5-yl)methyl]-4'-oxo-3',4'-dihydrospiro[azetidine-3,2'-[1]benzopyran]-1-carboxamide;
6-chloro-*N-*[(1-methyl-1*H*-indol-5-yl)methyl]-3,4-dihydrospiro[1-benzopyran-2,4'-piperidine]-1'-carboxamide;
*N*-[(3-chloro-1-methyl-1*H*-indol-5-yl)methyl]-6'-fluoro-4'-oxo-3',4'-dihydrospiro[azetidine-3,2'-[1]benzopyran]-1-carboxamide;
6'-chloro-*N*-[(1*H*-indol-5-yl)methyl]-4'-oxo-3',4'-dihydrospiro[azetidine-3,2'-[1]benzopyran]-1-carboxamide;
6'-fluoro-*N*-[(1-methyl-1*H*-indol-5-yl)methyl]-3',4'-dihydrospiro[azetidine-3,2'-[1]benzopyran]-1-carboxamide;
6'-fluoro-*N-*[(1-methyl-1*H-*indol-5-yl)methyl]spiro[azetidine-3,2'-chromene]-1-carboxamide;
*N*-[(1-methyl-1*H*-indol-5-yl)methyl]-3',4'-dihydrospiro[azetidine-3,2'-[1]benzopyran]-1-carboxamide;
6'-chloro-*N*-[(2-methyl-1*H*-indol-5-yl)methyl]-4'-oxo-3',4'-dihydrospiro[azetidine-3,2'-[1]benzopyran]-1-carboxamide;
6',8'-difluoro-*N*-[(2-methyl-1*H*-indol-5-yl)methyl]-4'-oxo-3',4'-dihydrospiro[azetidine-3,2'-[1]benzopyran]-1-carboxamide;
7'-chloro-*N*-[(2-chloro-1*H*-indol-5-yl)methyl]-4'-oxo-3',4'-dihydrospiro[azetidine-3,2'-[1]benzopyran]-1-carboxamide;
7'-chloro-*N*-[(2-methyl-1*H*-indol-5-yl)methyl]-4'-oxo-3',4'-dihydrospiro[azetidine-3,2'-[1]benzopyran]-1-carboxamide;
7'-fluoro-*N*-[(2-methyl-1*H*-indol-5-yl)methyl]-4'-oxo-3',4'-dihydrospiro[azetidine-3,2'-[1]benzopyran]-1-carboxamide;
*N*-[(2-methyl-1*H*-indol-5-yl)methyl]-4'-oxo-6'-(tritluoromethyl)-3',4'-dihydrospiro[azetidine-3,2'-[1]benzopyran]-1-carboxamide;
*N*-[(1-methyl-1*H-*indol-5-yl)methyl]-4'-oxo-7'-(trifluoromethyl)-3',4'-dihydrospiro[azetidine-3,2'-[1]benzopyran]-1-carboxamide;
*N*-[(2-methyl-1*H*-indol-5-yl)methyl]-4'-oxo-7'-(tritluoromethyl)-3',4'-dihydrospiro[azetidine-3,2'-[1]benzopyran]-1-carboxamide;
6',8'-dichloro-*N-*[(2-methyl-1*H-*indol-5-yl)methyl]-4'-oxo-3',4'-dihydrospiro[azetidine-3,2'-[1]benzopyran]-1-carboxamide;
7'-fluoro-4'-oxo-*N*-{[2-(trifluoromethyl)-1*H*-indol-5-yl]methyl} -3 ',4'-dihydrospiro[azetidine-3,2'-[1]benzopyran]-1-carboxamide;
*N*-{[2-(difluoromethyl)-1*H*-indol-5-yl]methyl}-7'-fluoro-4'-oxo-3',4'-dihydrospiro[azetidine-3,2'-[1]benzopyran]-1-carboxamide;
or pharmaceutically acceptable salts, racemates, enantiomers, diastereomers, solvates and hydrates thereof.

In a further aspect, the present invention provides a compound of formula (I) or formula (II), as defined above for use in the treatment or prevention of a disease associated with a7 nicotinic acetylcholine receptor activity.

In a further aspect, the present invention provides the use of a compound of formula (I) or formula (II), as defined above, for the manufacture of a medicament for the treatment or prevention of a disease associated with a7 nicotinic acetylcholine receptor activity.

In one embodiment, the disease associated with α7 nicotinic acetylcholine receptor activity is selected from the group of psychotic disorders, including, but not limited to, schizophrenia, schizophreniform disorder, schizoaffective disorder, delusional disorder, brief psychotic disorder, psychotic disorder due to a general medical condition, substance-induced psychotic disorder or psychotic disorder not otherwise specified; cognitive impairment, including, but not limited to, cognitive impairment as a result of stroke, Alzheimer's disease, Huntington's disease, Pick disease, HIV associated dementia, frontotemporal dementia, Lewy body dementia, vascular dementia, cerebrovascular disease or other dementia states and dementia associated to other degenerative disorders, including, but not limited to, amyotrophic lateral sclerosis, other acute or sub-acute conditions that may cause cognitive decline, including, but not limited to, delirium, traumatic brain injury, senile dementia, mild cognitive impairment, Down's syndrome, depression and cognitive deficit related to other diseases, and dyskinetic disorders including, but not limited to, Parkinson's disease, neuroleptic-induced parkinsonism, or tardive dyskinesias, depression and mood disorders, including, but not limited to, depressive disorders and episodes, bipolar disorders, cyclothymic disorder, and bipolar disorder not otherwise specified, other mood disorders, substance-induced mood disorder and mood disorder not otherwise specified; anxiety disorders, panic disorder and panic attacks, obsessive compulsive disorder, posttraumatic stress disorder, acute stress disorder, generalized anxiety disorder, anxiety disorder due to a general medical condition, substance-induced anxiety disorder, phobias, and anxiety disorder not otherwise specified; substance related disorders, including, but not limited to, substance use or substance-induced disorders, including, but not limited to, alcohol-, nicotine-, amphetamine-, phencyclidine-, opioid-, cannabis-, cocaine-, caffeine-, hallucinogen-, inhalant-, sedative-, hypnotic-, anxiolytic-, polysubstance- or other substance-related disorders; sleep disorders, including, but not limited to, narcolepsy, dyssomnias, primary hypersomnia, breathing-related sleep disorders, circadian rhythm sleep disorder and dyssomnia not otherwise specified; parasomnias, sleep terror disorder, sleepwalking disorder and parasomnia not otherwise specified; sleep disorders related to another mental disorder; sleep disorder due to a general medical condition and substance-induced sleep disorder; metabolic and eating disorders, including, but not limited to, anorexia nervosa, bulimia nervosa, obesity, compulsive eating disorder, binge eating disorder and eating disorder not otherwise specified; diabetes mellitus, ulcerative colitis, Crohn's disease, irritable bowel syndrome; autism spectrum disorders, including, but not limited to, autistic disorder, Asperger's disorder, Rett's disorder, childhood disintegrative disorder and pervasive developmental disorder not otherwise specified; attention deficit hyperactivity disorder, disruptive behaviour disorders, oppositional defiant disorder and disruptive behaviour disorder not otherwise specified; and tic disorders, including, but not limited to, Tourette's disorder; personality disorders; sexual dysfunctions such as sexual desire disorders, sexual arousal disorders, orgasmic disorders, sexual pain disorder, sexual dysfunction not otherwise specified, paraphilias, gender identity disorders, infertility, premenstrual syndrome and sexual disorders not otherwise specified; disorders of the respiratory system like cough, asthma, chronic obstructive pulmonary disease, lung inflammation, disorders of the cardiovascular system such as cardiac failure, heart arrhythmia, hypertension; inflammation, inflammatory and neuropathic pain, rheumatoid arthritis, osteoarthritis, allergy, sarcoidosis, psoriasis, ataxia, dystonia, systemic lupus erythematosus, mania, restless legs syndrome, progressive supranuclear palsy, epilepsy, myoclonus, migraine, amnesia, chronic fatigue syndrome, cataplexy, brain ischemia, multiple sclerosis, encephalomyelitis, jetlag, cerebral amyloid angiopathy, and sepsis.

In one embodiment, the disease associated with α7 nicotinic acetylcholine receptor activity is selected from the group of cognitive impairment, schizophrenia, and autism.

The invention, further relates to combination therapies wherein a compound of the invention or a pharmaceutical composition or formulation comprising a compound of the invention is administered with another therapeutic agent or agents, for the treatment of one or more of the conditions previously indicated. Such therapeutic agents may be selected from: acetylcholinesterase inhibitors, NMDA receptor agonists or antagonists, anti-amyloid antibodies including anti-amyloid humanized monoclonal antibodies, beta- or gamma-secretase inhibitors or modulators, tau phosphorylation inhibitors, ApoE4 conformation modulators, p25/CDK5 inhibitors, NK1/NK3 receptor antagonists, COX-2 inhibitors, LRRK2 inhibitors, HMG-CoA reductase inhibitors, NSAIDs, vitamin E, glycine transport inhibitors, glycine site antagonists, LXR β agonists, androgen receptor modulators, blockers of Aβ oligomer formation, NR2B antagonists, anti-inflammatory compounds, PPAR gamma agonists, CB-1 receptor antagonists or inverse agonists, CB-2 agonists, VR-1 antagonists, bradykinin Bl receptor antagonists, sodium channel blockers and antagonists, NOS inhibitors, antibiotics, growth hormone secretagogues, potassium channel openers, AMPA agonists or AMPA modulators, GSK3 inhibitors, neuronal nicotinic agonists, MARK ligands, M₁ or M₄ mAChR agonists or PAMs, mGluR2 antagonists or NAMs or PAMs, mGluR5 antagonists, alpha-adrenerg agonists, ADAM-10 ligands, sedatives, hypnotics, anxiolytics, antipsychotics, cyclopyrrolones, imidazopyridines, pyrazolopyrimidines, minor tranquilizers, melatonin agonists and antagonists, melatonergic agents, orexin antagonists and agonists, prokineticin agonists and antagonists, T-type calcium channel antagonists, triazolopyridines benzodiazepines, barbiturates, 5-HT_{1A} antagonists, 5-HT₂ antagonists, 5-HT₄ agonists, 5-HT₆ antagonists, histamine H₃ receptor antagonists and inverse agonists, PDE₄ inhibitors, PDE₉ inhibitors, PDE₁₀ inhibitors, HDAC inhibitors, KCNQ antagonists, GABA_{A} inverse agonists, GABA signalling enhancers, GABA agonists, GABA_{A} receptor alpha5 subunit NAMs or PAMs, antipsychotics, MAO-B inhibitors, dopamine transport inhibitors, noradrenaline transport inhibitors, D₂ agonists and partial agonists, anticholinergics, COMT inhibitors, A2a adenosine receptor antagonists, cholinergic agonists, neuroleptic agents, loxapine, sulpiride and risperidone, levodopa, calcium channel blockers, MMP inhibitors, thrombolytic agents, opioid analgesics, pramipexole, ropinirole, neutrophil inhibitory factor, SSRIs or SSNRIs, tricyclic antidepressant drugs, norepinephrine modulators, lithium, valproate, gabapentin, pregabalin, rizatriptan, zolmitriptan, naratriptan, and sumatriptan.

In one embodiment, the therapeutic agents are selected from the goup of acetylcholinesterase inhibitors, NMDA receptor antagonists, beta- secretase inhibitors, antipsychotics, GABA_{A} receptor alpha5 subunit NAMs or PAMs, histamine H₃ receptor antagonists, 5-HT₆ receptor antagonists, M1 or M4 mAChR agonists or PAMs, mGluR2 antagonists or NAMs or PAMs, and levodopa.

In a further aspect the present invention provides a process for the manufacture of the compounds of formula (II) according to the following reaction route:
Compounds in accordance with the present invention were synthesized in line with the synthetic routes and schemes described below.

Throughout the specification, general formulae are designated by Roman numerals (I), (II), (III) etc.

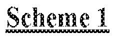

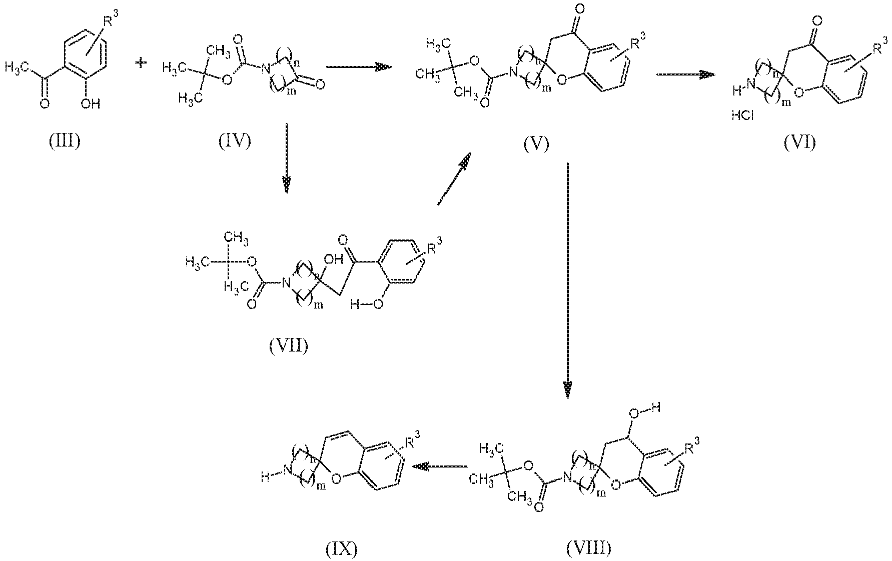

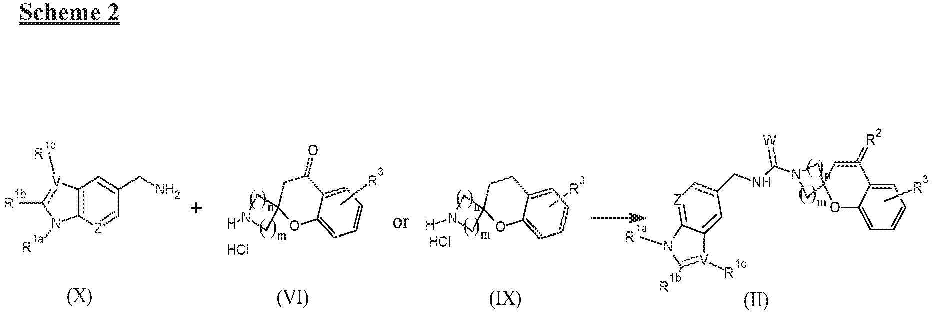

### Scheme 1

Reacting an acetophenone derivative of formula (III) 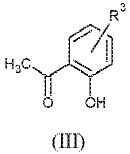
- wherein the meaning of R³ is described above for compound of formula (II) - with a nitrogen-containing cyclic aliphatic ketone derivative of formula (IV) 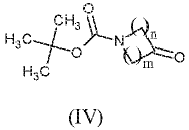
- wherein the meaning of n and m is described above for compound of formula (II) - either in two separated steps via formula (VII) 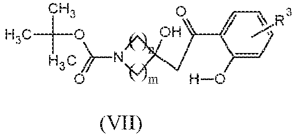
- wherein the meaning of n and m is described above for compound of formula (II) - or directly in one step to provide the spirochromanone compound of formula (V) 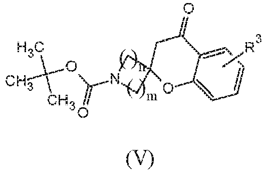
- wherein the meaning of R³, n and m is as described above for formula (II) - which is then reacted with a.) hydrogen chloride to provide the spirochromanone salt derivative of formula (VI) 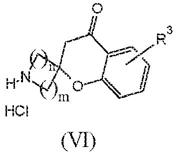
- wherein the meaning of R³, n and m is as described above for formula (II) - or b.) complex hydrides to provide the appropriate spirochromanol derivative of formula (VIII) 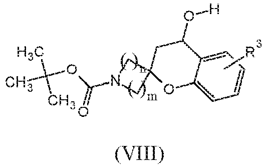
- wherein the meaning of R³, n and m is as described above for formula (II) - then compound of formula (VIII) is reduced with triethylphosphine to provide the 3,4-dihydrospirochromene/spirochromene derivative of formula (IX) 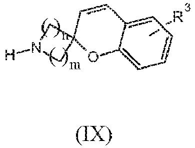
- wherein the meaning of R³, n and m is as described above for formula (II).

### Scheme 2

Then, the so obtained derivative of formula (IX) or formula (VI) is reacted with the heterocyclic amine derivatives of formula (X) 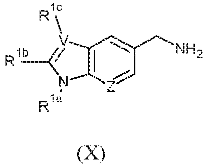
- wherein the meaning of R^{1a}, R^{1b}, R^{1c}, V and Z is as described above for formula (II) - providing the carbamide/thiocarbamide derivative of formula (II) 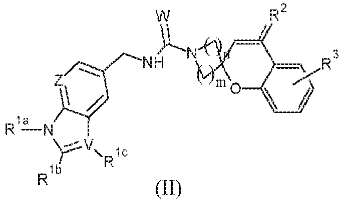
- wherein the meaning of R^{1a}, R^{1b}, R^{1c}, R², R³, V, Z, n, m and W are as described above for formula (II).

The synthesis of protected spirochroman-4-one of formula (V) can be carried out by different routes:
a) The condensation of acetophenone derivatives of formula (III) with nitrogen-containing cyclic aliphatic ketone derivatives of formula (IV) is preferably carried out in a suitable solvent, e.g., methanol, preferably in the presence of pyrrolidine. The reaction is preferably carried out at the boiling point of the solvent. The necessary reaction time is 15-20 hours. The reactions are followed by thin layer chromatography. The reaction mixture is quenched by evaporation of the solvent. The product of formula (V) is isolated by extraction with a suitable organic solvent, or by filtration, after removing the organic solvent, or by column chromatography.
b) The reaction of an acetophenone derivative of formula (III) with nitrogen-containing cyclic aliphatic ketone derivatives of formula (IV) is preferably carried out in a suitable solvent, e.g., tetrahydrofuran, preferably in the presence of a strong base, e.g., lithium diisopropyl amide. The reaction is carried out at a temperature in the range of -20 °C to room temperature. The necessary reaction time is 3-4 hours. The progress of the reaction is followed by thin layer chromatography. The reaction mixture is quenched by addition of saturated ammonium chloride solution. The product of formula (VII) is isolated by extraction with a suitable organic solvent and by filtration, after removing the organic solvent.

The dehydrocyclization of the derivatives of formula (VII) is preferably carried out in suitable solvents, in the presence of, e.g., trifluoroacetic anhydride and DBU (1,8-diazabicyclo[5.4.0]undec-7-ene).

Unless otherwise specified, solvents, temperature and other reaction conditions may be readily selected by one of ordinary skill in the art. Specific procedures are provided in the Examples section. Reactions may be further processed in a conventional manner, e.g., by eliminating the solvent from the residue and further purifying according to methodologies generally known in the art, including, but not limited to, crystallization, extraction, trituration and chromatography.

The desired deprotected spirochroman-4-on salts of formula (VI) can be obtained by using different methodologies from the prior art. It is preferably carried out in EtOAc with hydrochloric acid in the range of 0 °C to room temperature. The necessary reaction time is 2-3 hours. The progress of the reaction is followed by thin layer chromatography, and the product is isolated by filtration.

Reduction of the derivatives of formula (V) (carbonyl group of spiroketone) is preferably carried out in suitable solvents e.g., ethanol with NaBH₄. The progress of the reaction is followed by TLC. The crude spirochroman-4-ol is isolated by eliminating the solvent, then the residue is partitioned between DCM and water, and evaporating the organic phase to obtain the title compound, which is used without purification in the forthcoming step.

The reductive deoxygenation of spirochroman-4-ol derivatives of formula (VIII) is accomplished by the well-known reducing method called "ionic hydrogenation": by the treatment of the hydroxy derivatives with the Et₃SiH/CF₃COOH system at 90 °C for 6-18 hours. The progress of the reaction is followed by thin layer chromatography. The reaction mixture is evaporated, the residue is treated with saturated NaHCO₃ solution, and the product of formula (IX) is isolated by extraction with a suitable organic solvent.

Most of the primary amine derivatives of formula (X) are either commercially available, or can be synthesized from commercially available starting materials and reagents by one skilled in the art using different methods described in the prior art. The synthesis of some new amine derivatives of formula (X) are described in the Examples section.

The compounds of formula (II) above can be prepared by the activation of the appropriate primary amine compounds of formula (X) using standard procedures and reagents, e.g., CDI (1,1'-carbonyldiimidazole), chloroformates or 1,1'-thiocarbonyldiimidazole in suitable solvents, e.g., DCM under argon athmosphere followed by the addition of the reactant (formula (VI) or formula (IX)). The reaction is carried out at a temperature in the range of 0 °C to room temperature. The necessary reaction time is 15-20 hours. The progress of the reactions is followed by thin layer chromatography. The workup of the reaction mixture can be carried out by different methods, usually it is quenched by the addition of water. The product is isolated by extraction with a suitable organic solvent, and purified by crystallization or column chromatography.

The present disclosure includes within its scope all the possible isotopically labelled forms of the compounds.

The compounds of the present invention can be administered by oral, parenteral (e.g., intramuscular, intraperitoneal, intravenous, intraarticular, intrathecal, intraperitoneal, direct intraventricular, intracerebroventicular, intramedullary injection, intracisternal injection or infusion, subcutaneous injection or implant), ophtalmic, nasal, vaginal, rectal, sublingual and topical routes of administration and may be formulated, alone or together, in suitable dosage unit formulations comprising pharmaceutically acceptable excipients suitable for each route of administration.

Alternatively, one may administer the compounds in a local rather than systemic manner, for example, via injection of the compound directly in the renal or cardiac area, often in a modified release formulation. Furthermore, one may administer the drug in a targeted drug delivery system, for example, in a liposome coated with a tissue-specific antibody. The liposomes are taken up selectively by the targeted organ.

The pharmaceutical compositions of the present invention usually contain 0.01 to 500 mg of the active ingredient in a single dosage unit. However, it is possible that the amount of the active ingredient in some compositions exceeds the upper or lower limits defined above.

The compounds may be administered on a regimen of 1 to 4 times per day, preferably once or twice per day.

This dosage level and regimen can be adjusted to provide the optimal therapeutic response. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition and the host undergoing therapy.

As a further aspect of the invention, there is provided the pharmaceutical manufacture of medicaments containing the compounds of formula (I) or formula (II) or pharmaceutically acceptable salts, racemates, enantiomers, diastereomers, solvates and hydrates thereof.

The pharmaceutical compositions of the present invention may be formulated as different pharmaceutical dosage forms, including, but not limited to, solid oral dosage forms like tablets (e.g., buccal, sublingual, effervescents, chewable, orodispersible, freeze dried), capsules, lozenges, pastilles, pills, orodispersible films, granules, powders; liquid oral dosage forms, including, but not limited to, solutions, emulsions, suspensions, syrups, elixirs, oral drops; parenteral dosage forms, including, but not limited to, intravenous injections, intramuscular injections, subcutaneous injections; other dosage forms, including, but not limited to, eye drops, semi-solid eye preparations, nasal drops or sprays, transdermal dosage forms, suppositories, rectal capsules, rectal solutions, emulsions and suspensions, etc.

The pharmaceutical compositions of the present invention can be manufactured in any conventional manner, e.g., by mixing, dissolving, emulsifying, suspending, entrapping, freezedrying, extruding, laminating, film-casting, granulating, grinding, encapsulating, drageemaking or tabletting processes.

Pharmaceutical compositions for use in accordance with the present invention thus can be formulated in any conventional manner using one or more physiologically acceptable excipients. Any of the well-known techniques and excipients may be used as suitable and as understood in the art.

Suitable excipients for the preparation of the dosage forms may be selected from the following categories, including, but not limited to, tablet and capsule fillers, tablet and capsule binders, release modifying agents, disintegrants, glidants, lubricants, sweetening agents, tastemasking agents, flavoring agents, coating agents, surfactants, antioxidants, buffering agents, complexing agents, emulsifying agents, lyophilization aids, microencapsulating agents, ointment bases, penetration enhancers, solubilizing agents, solvents, suppository bases, and suspending agents.

In one embodiment, the invention relates to the use of specific excipients which are capable of improving the solubility, dissolution, penetration, absorption and/or bioavailability of the active ingredient(s), including, but not limited to, hydrophilic polymers, hot melt extrusion excipients, surfactants, buffering agents, complexing agents, emulsifying agents, lyophilization aids, superdisintegrants, microencapsulating agents, penetration enhancers, solubilizing agents, co-solvents, and suspending agents.

The above described ingredients and different routes of manufacture are merely representative. Other materials as well as processing techniques and the like well known in the art can also be used.

### EXAMPLES

The invention is further defined in the following Examples. It should be understood that the Examples are given by way of illustration only. From the above discussion and the Examples, one skilled in the art can ascertain the essential characteristics of the invention, and can make various changes and modifications to adapt the invention to various uses and conditions. As a result, the invention is not limited by the illustrative examples set forth herein below, but rather defined by the claims appended hereto.

In general, the compounds of formula (I) and formula (II) can be prepared in accordance with the general knowledge of one skilled in the art and/or using methods set forth in the Example and/or Intermediate sections that follow. Solvents, temperatures, pressures, and other reaction conditions can readily be selected by one of ordinary skill in the art. Starting materials are commercially available and/or readily prepared by one skilled in the art.

The present invention will be now illustrated by the following not limiting examples. In the following examples "room temperature" denotes a temperature in the range from 20 °C to 25 °C.

The abbreviations used in the specific examples have the following meanings:
- AcOH: acetic acid
- abs.: absolute
- aq.: aqueous
- atm: atmosphere
- Boc₂O: di-tert-butyl dicarbonate
- BH₃.THF: borane tetrahydrofuran complex solution
- CDI: 1,1'-carbonyldiimidazole
- DBU: 1,8-diazabicyclo[5.4.0]undec-7-ene
- DCE: 1,2-dichloroethane
- DCM: dichloromethane
- DIPEA: N,N-diisopropylethylamine
- DMF: N,N'-dimethylformamide
- DMSO: dimethyl sulfoxide
- EtOAc: ethylacetate
- Et₃SiH: triethylsilane
- ESI: electronspray ionization
- HEPES: (4-(2-hydroxyethyl)-1-piperazineethanesulfonic acid)
- HPLC: high-performance liquid chromatography
- LC-MS: liquid chromatography coupled with mass spectroscopy
- MeOH: methanol
- *n*-BuLi: n-butyllithium solution
- NMP: N-methyl-2-pyrrolidone
- PCC: pyridinium chlorochromate
- sat.: saturated
- TEA: triethylamine
- TFA: trifluoroacetic acid
- THF: tetrahydrofuran
- TLC: thin layer chromatography

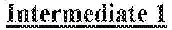

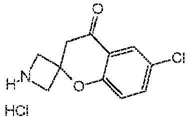

### 6'-Chloro-3',4'-dihydrospiro[azetidine-3,2'-[1]benzopyran]-4'-one hydrochloride

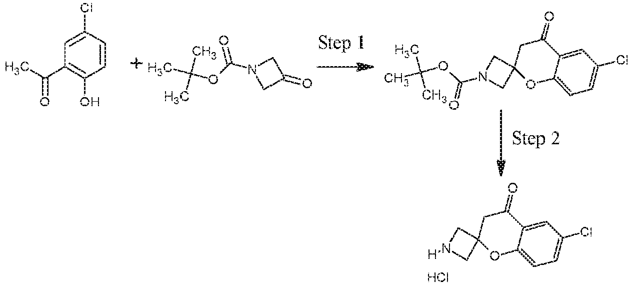

### Step 1: tert-Butyl 6'-chloro-4'-oxo-3',4'-dihydrospiro[azetidine-3.2'-[1]benzopyran]-1-carboxylate

A solution of 3.42 g (20 mmol) *tert*-butyl 3-oxoazetidine-1-carboxylate, 3.41g (20 mmol) of 1-(5-chloro-2-hydroxyphenyl)ethan-1-one and 1.42 g (1.67 mL, 20 mmol) of pyrrolidine in methanol (20 mL) was refluxed for 20 hours. The reaction mixture was evaporated *in vacuo,* the brown residue was dissolved in dichloromethane (120 mL), and partitioned with 1 N HCl, then the organic layer was washed with water (2x50 mL) and brine (50 mL), dried over anhydrous sodium sulfate, filtered and concentrated *in vacuo.* The crude product was chromatographed on silica gel eluting with DCM: MeOH to yield 3.02 g (46 %) of the title compound.

### Step 2: 6'-Chloro-3',4'-dihydrospiro[azetidine-3.2'-[1]benzopyran]-4'-one hydrochloride

To the solution of 3.0 g (8.9 mmol) of *tert*-butyl 6'-chloro-4'-oxo-3',4'-dihydrospiro[azetidine-3,2'-[1]benzopyran]-1-carboxylate (from the previous step) in EtOAc (30 mL), 20 % hydrochloric acid in EtOAc (30 mL) was added at 0 °C in 10 minutes. After the addition, the mixture was allowed to warm to room temperature and stirred at this temperature for 2 hours. Then the reaction mixture was concentrated to approximately 15 mL *in vacuo,* and was diluted with diethyl ether (20 mL). The precipitated light brown crystal was filtered off, washed with diethyl ether and dried to yield 2.0 g (90 %) of the title compound.

Compounds of Table 1 were prepared from the appropriate acetophenone and nitrogen-containing cyclic aliphatic ketone in the presence of pyrrolidine according to the method described for Intermediate 1.

**Table 1**

| **Intermediate** | **Structure** |
|---|---|
| **2** | 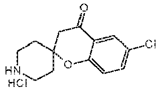 |
| **3** | 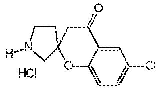 |
| **4** | 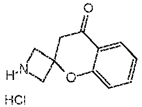 |
| **5** | 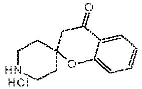 |
| **6** | 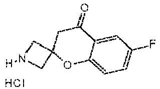 |
| **7** | 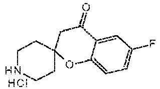 |
| **8** | 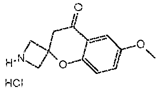 |
| **9** | 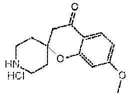 |
| **10** | 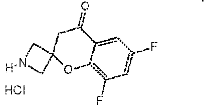 |
| **11** | 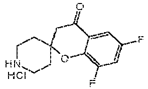 |
| **12** | 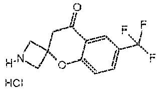 |
| **13** | 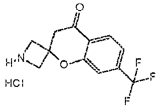 |
| **14** | 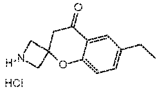 |
| **15** | 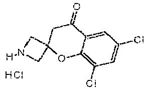 |
| **16** | 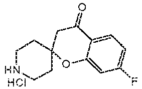 |
| **17** | 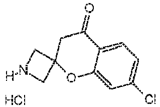 |

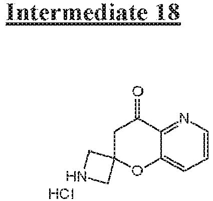

### 3',4'-Dihydrospiro[azetidine-3,2'-pyrano[3,2-b]pyridin]-4'-one hydrochloride

### Step 1: Tert-butyl 4'-oxo-3',4'-dihydrospiro[azetidine-3.2'-pyrano[3,2-b]pyridine]-1-carboxylate

A solution of 1-(3-hydroxypyridin-2-yl) ethanone (160 mg; 1.17 mmol), N-*tert-*butoxycarbonyl-3-azetidinon (205 mg, 1.2 mmol) and pyrrolidine (0.1 mL, 1.2 mmol) in toluene (4 mL) was stirred at reflux for 28 hours. Upon completion of the reaction (monitored by TLC), the mixture was concentrated under vacuum, and the obtained dark brown residue was partitioned between EtOAc (30 mL) and 1 N hydrochloric acid (10 mL). The layers were separated. The aqueous layer was extracted with EtOAc (2x 20 mL). The combined organic extracts were successively washed with water (2x 20 mL), saturated NaHCO₃ solution (20 mL) and brine (20 mL). The residue, obtained after evaporation of the solvent was purified by flash silica gel column chromatography using 30 % n-hexane in EtOAc as eluent, to yield 100 mg (29 %) of the title compound.

### Step 2: 3'.4'-Dihydrospiro[azetidine-3.2'-pyrano[3.2-b]pyridin]-4'-one hydrochloride

To the solution of 113 mg (0.389 mmol) of t*ert*-butyl 4'-oxo-3',4'-dihydrospiro[azetidine-3,2'-pyrano[3,2-b]pyridine]-1-carboxylate (from the previous step) in EtOAc (2 mL), 20 % hydrochloric acid in EtOAc (2 mL) was added at 0 °C in 10 minutes. After addition, the mixture was allowed to warm to room temperature, and stirred at this temperature for 2 hours. Then the reaction mixture was concentrated to approximately 15 mL *in vacuo,* and was diluted with diethyl ether (3 mL). The precipitated light brown crystal was filtered off, washed with diethyl ether, and dried to yield 80 mg (91 %) of the title compound.

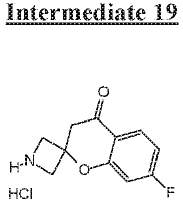

### 7'-Fluoro-3',4'-dihydrospiro[azetidine-3,2'-[1]benzopyran]-4'-one hydrochloride

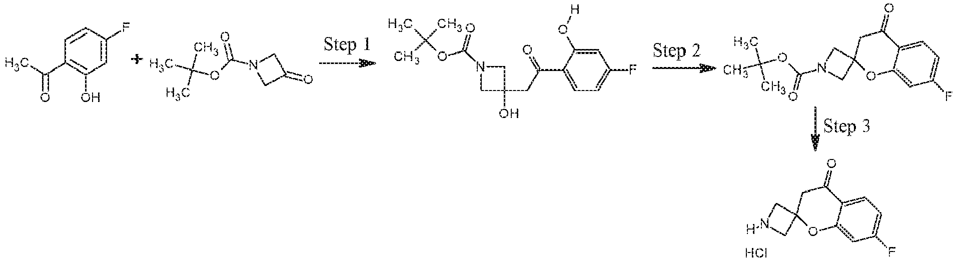

### Step 1: tert-Butyl 3-[2-(4-fluoro-2-hydroxyphenyl)-2-oxoethyl]-3-hydroxyazetidine-1-carboxylate

To a solution of diisopropylamine (17.7 mL, 125 mmol) in THF (50 mL) cooled to -15 °C, 2.5 M *n*-BuLi in hexane (50 mL) was added dropwise under nitrogen atmosphere, and the mixture was stirred at -15 °C for 30 minutes. Then a solution of 8.96 g (58.14 mmol) of 4'- fluoro- 2'-hydroxyacetophenone in THF (50 mL) was added dropwise, and the mixture was stirred at - 15 °C for 1 hour, and then treated with a solution of *tert*-butyl 3-oxoazetidine-1-carboxylate (12.9 g, 75.6 mmol) in THF (50 mL) dropwise for 20 minutes, allowed to warm to room temperature and stirred at this temperature for 1 hour. The reaction mixture was quenched by addition of saturated NH₄Cl solution (100 mL). The reaction mixture was extracted with ethyl acetate (2x90 mL), the combined organic layer was washed with brine (120 mL), dried over anhydrous Na₂SO₄, filtered and concentrated *in vacuo.* The residue was triturated with diisopropyl ether (50 mL), the precipitated white crystals were filtered off, washed with diisopropyl ether, and dried to yield 16.79 g (89 %) of the title compound.

### Step 2: tert-Butyl 7'-fluoro-4'-oxo-3',4'-dihydrospiro[azetidine-3,2'-[1]benzopyran]-1-carboxylate

To a solution of 14.2 g (43.6 mmol) of *tert*-butyl 3-[2-(4-fluoro-2-hydroxyphenyl)-2-oxoethyl]-3-hydroxyazetidine-1-carboxylate in dry pyridine (35 mL), 14.48 g (9.58 mL, 68.12 mmol) of trifuoroacetic anhydride was added dropwise under nitrogen atmosphere at - 10 °C. After addition, the mixture was allowed to warm to room temperature and stirred at this temperature for 1 to 2 hours. Then the reaction mixture was diluted with ethanol (80 mL), and treated with 49 g (48 mL) of 1,8-diazabicyclo[5.4.0]undec-7-ene. During the addition, the mixture's temperature was raised to 50 °C and kept at this temperature for 1 hour. The mixture was concentrated *in vacuo,* and the residue was dissolved in EtOAc (160 mL), and washed with water (2x80 mL), 1 N HCl (80 mL), 1 M NaHCO₃ solution (80 mL) and brine (40 mL). The organic layer was dried over anhydrous Na₂SC₄, filtered and concentrated *in vacuo.* The residue was triturated with a mixture of diethyl ether and *n*-hexane (1:1, 40 mL), the precipitated white crystals were filtered off, washed with diethyl ether and dried to yield 4.9 g (36 %) of the title compound.

The evaporated mother liquor was chromatographed on silica gel eluting with CH₂Cl₂ to yield an additional 2.05 g (15 %) of the title compound.

### Step 3: 7'-Fluoro-3'.4'-dihydrospiro[azetidine-3.2'-[1]benzopyran]-4'-one hydrochloride

To the solution of 6.05 g (19.7 mmol) of *tert*-butyl 7'-fluoro-4'-oxo-3',4'-dihydrospiro[azetidine-3,2'-[1]benzopyran]-1-carboxylate (from the previous step) in EtOAc (60 mL), 20 % HCl in EtOAc (60 mL) was added at 0 °C for 10 minutes. After addition, the mixture was allowed to warm to room temperature, and stirred at this temperature for 2 hours. Then the reaction mixture's volume was reduced to 15-20 mL *in vacuo,* and was diluted with diethyl ether (30 mL). The precipitated white crystals were filtered off, washed with diethyl ether, and dried to yield 4.53 g (94 %) of the title compound.

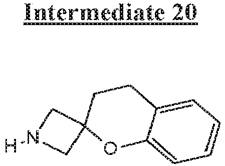

### 3',4'-Dihydrospiro[azetidine-3,2'-[1]benzopyran

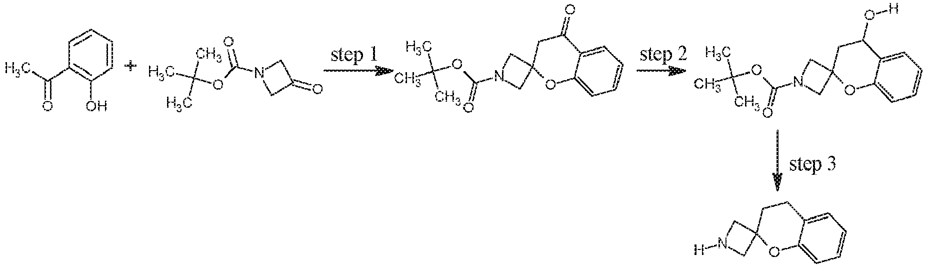

### Step 1. tert-Butyl 4'-oxo-3',4'-dihydrospiro[azetidine-3,2'-[1]benzopyran]-1-carboxylate

This intermediate was prepared from the appropriate 2-hydroxy-acetophenone and *tert-*butyl 3-oxoazetidine-1-carboxylate with pyrrolidine according to the method described for Intermediate 1.

### Step 2. tert-Butyl 4'-hydroxy-3',4'-dihydrospiro[azetidine-3,2'-[1]benzopyran]-1-carboxylate

To the solution of 400 mg (1.38 mmol) of *tert*-butyl 4'-oxo-3',4'-dihydrospiro[azetidine-3,2'-[1]benzopyran]-1-carboxylate in MeOH (5 mL), NaBH₄ (52.3 mg, 1.38 mmol) was added portionwise under ice-water cooling. After addition, the mixture was allowed to warm to room temperature, and stirred at this temperature for 4 hours. Then the reaction mixture was concentrated *in vacuo,* and the residue was dissolved in EtOAc (15 mL), washed with water and brine. The organic layer was dried over anhydrous Na₂SC₄, filtered and concentrated *in vacuo,* to yield 320 mg (79 %) of the title compound. The obtained crude spirochroman-4-ol is used in the next step without any purification.

### Step 3. 3',4'-Dihydrospiro[azetidine-3,2'-[1]benzopyran

To the solution of tert-butyl 4'-hydroxy-3',4'-dihydrospiro[azetidine-3,2'-[1]benzopyran]-1-carboxylate (292 mg, 1 mmol) in trifluoroacetic acid (4 mL), Et₃SiH (450 mg, 4 mmol) was added and the mixture was stirred at 90 °C for 16 hours. Then the reaction mixture was concentrated *in vacuo,* and the residue was dissolved in water, and the pH was adjusted to 9 by the addition of 1.5 N NaOH solution. The mixture was extracted with CH₂Cl₂ (3x), the combined extracts were dried over anhydrous Na₂SO₄, filtered and concentrated *in vacuo,* to yield 80 mg (50 %) of the title compound. The obtained crude 3,4-dihydrospirochromene derivative was used in the next step without any purification.

Compounds of Table 2 were prepared from the appropriate spirochroman-4-on, spirochroman-4-ol, 3,4-dihydrospirochromene reaction sequence by the methods described for Intermediate 20.

**Table 2**

| **Intermediate** | **Structure** |
|---|---|
| **21** | 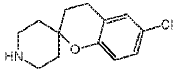 |
| **22** | 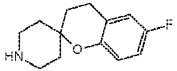 |
| **23** | 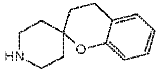 |
| **24** | 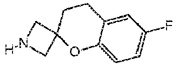 |
| **25** | 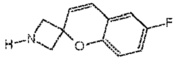 |

Intermediate 24 and 25 originated from the reductive deoxygenation reaction of tert-butyl 6'-fluoro-4'-hydroxy-3',4'-dihydrospiro[azetidine-3,2'-[1]benzopyran]-1-carboxylate were used in the next step without separation.

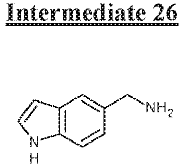

### (1H-indol-5-yl)methanamine

The compound is commercially available from Sigma Aldrich (catalog no.: 655864).

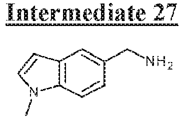

### (1-Methyl-1H-indol-5-yl)methanamine

The compound is commercially available from Maybridge (catalog no.: CC41413DA).

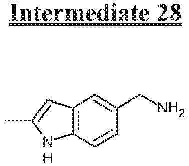

### (2-Methyl-1H-indol-5-yl)methanamine

The compound is commercially available from Enamine (catalog no.: EN300-209649).

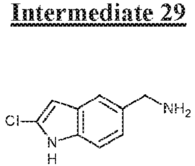

### (2-Chloro-1H-indol-5-yl)methanamine

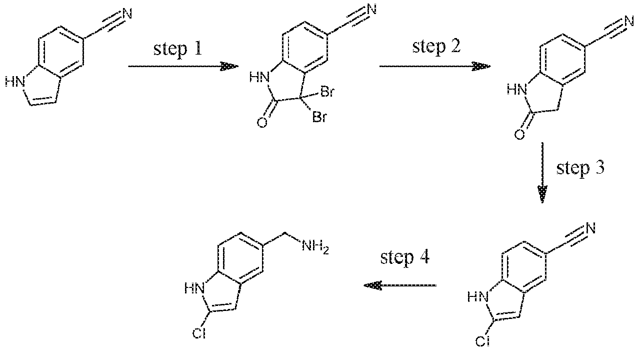

### Step 1: 3.3-Dibromo-2-oxo-2.3-dihydro-1H-indole-5-carbonitrile

To a stirred solution of 1*H-*indole-5-carbonitrile (7.10 g; 50 mmol, Combi-Blocks) in *tert*-butanol (250 mL), bromine (42.5 g; 13.5 mL, 275 mmol) was added dropwise (20 min) at 25 °C and stirred for 1.5 hours. After completion, the reaction mixture was concentrated under vacuum. This residue was diluted with EtOAc (400 mL), and water (75 mL) was added. The organic layer was washed with water (2x100 mL), brine (100 mL), dried over anhydrous Na₂SO₄, filtered and concentrated under reduced pressure. The crude residue was stirred with diisopropyl ether (100 mL) for 30 minutes. The precipitated crystals were filtered off, washed with diisopropyl ether, and dried to yield 12.89 g (81 %) of the title compound as a reddish brown solid.

### Step 2: 2-Oxo-2,3-dihydro-1H-indole-5-carbonitrile

To a suspension of 3,3-dibromo-2-oxo-2,3-dihydro-1*H*-indole-5-carbonitrile (12.89 g; 40.8 mmol) in AcOH (270 mL), Zn powder (16.3 g; 250 mmol) was added portionwise (ca. 2 g each). The temperature of the mixture was not allowed to increase above 35 °C and stirred for 2 hours at 30 °C. After completion, the reaction mixture was concentrated under vacuum. This residue was suspended in EtOAc (300 mL), filtered off, the solid was stirred with EtOAc (150 mL) again, then filtered off. The combined organic layer was concentrated *in vacuo.* The residue was stirred with 1 N HCl solution (100 mL) for 1 hour, filtered off, washed with water (2x5 mL), and dried to yield 3.86 g (60 %) of the title compound.

### Step 3: 2-Chloro-1H-indole-5-carbonitrile

To a stirred suspension of 2-oxoindoline-5-carbonitrile (5.8 g; 36.9 mmol) in DCE (23 ml), POCl₃ (11.5 g, 6.95 mL; 74.79 mmol) was added at 0 °C. The reaction mixture was refluxed for 30 minutes at 90 °C. After cooling the reaction, imidazole (2.75 g, 44.55 mmol) was added and further heated at 90 °C for 2 hours. After completion, the reaction mixture was concentrated and the residue was dissloved in EtOAc (110 mL) and washed with saturated NaHCO₃ solution (30 mL), brine (50 mL), dried over anhydrous Na₂SC₄, filtered and concentrated *in vacuo.* The product was purified by column chromatography on silica gel eluting with THF: *n*-Hexane 9:1 to yield 4.58 g (70 %) of the title compound as a yellow solid.

### Step 4: (2-Chloro-1H-indol-5-yl)methanamine

To a stirred solution of 2-chloro-1*H*-indole-5-carbonitrile (4.48 g; 25.3 mmol) in dry THF (23 mL), LiAlH₄ (1 M in THF; 45 mL; 45 mol) was added at 0 °C under nitrogen atmosphere. The reaction mixture was refluxed at 65 °C for 2 hours. TLC showed formation of the product. The reaction mixture was quenched with EtOAc (20 mL) at 0 °C and sat. aq. Na₂SO₄ solution (15 mL) was added dropwise. The reaction mixure was filtered through celite pad and thoroughly washed with ethyl acetate (100 mL). The filtrate was washed with brine (50 mL), dried over anhydrous Na₂SO₄, filtered and concentrated *in vacuo.* The crude product was purified by crystallization from EtOAc (50 mL) to give 3.9 g (85 %) of the title compound as an off white solid.

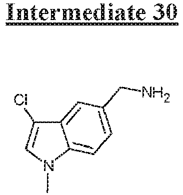

### (3-Chloro-1-methyl-1H-indol-5-yl)methanamine

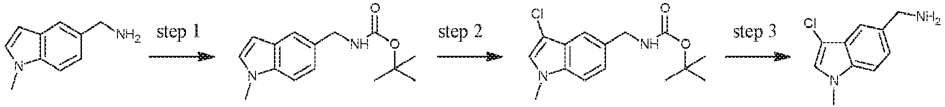

### Step 1: tert-Butyl N-[(1-methyl-1H-indol-5-yl)methyl]carbamate

To a stirred solution of (1-methyl-1*H-*indol-5-yl)methanamine (640 mg; 3.995 mmol) and DIPEA (1.03g; 1.39 mL, 7.98 mmol) in CH₂Cl₂ (40 mL), a solution of di-*tert*-butyl dicarbonate (Boc₂O) (1.74 g; 7.97 mmol) in CH₂Cl₂ (10 mL) was added at 0 °C under nitrogen atmosphere. The reaction mixture was stirred at room temperature overnight. TLC showed formation of the product. The reaction mixture was diluted with CH₂Cl₂ (20 mL) and washed with water (50 mL). The organic phase was washed with brine (50 mL), dried over anhydrous Na₂SO₄, filtered and concentrated *in vacuo.* The crude product was purified by flash chromatography to give 786 mg (76 %) of the title compound as a white solid.

### Step 2: tert-Butyl N-(3-chloro-1-methyl-1H-indol-5-yl)methyl]carbamate

To a stirred solution of *tert*-butyl *N*-[(1-methyl-1*H*-indol-5-yl)methyl]carbamate (160 mg; 0.615 mmol) in CH₂Cl₂(4 mL), N-chlorosuccinimide (82 mg; 0.61 mmol) was added at 0 °C under argon atmosphere. The reaction mixture was stirred at room temperature for 3.5 hours. TLC showed formation of the product. The reaction mixture was diluted with CH₂Cl₂ (20 mL), and washed with water (20 mL). The organic phase was washed with brine (15 mL), dried over anhydrous Na₂SO₄, filtered and concentrated *in vacuo.* The crude product was purified by flash chromatography to give 145 mg (80 %) of the title compound as an oil.

### Step 3: (3-Chloro-1-methyl-1H-indol-5-yl)methanamine

To the solution of *tert*-butyl *N*-[(3-chloro-1-methyl-1*H*-indol-5-yl)methyl]carbamate (72 mg, 0.244 mmol) in dry CH₂Cl₂ (3 mL), CF₃COOH (300 mg; 0.2 mL, 3 mmol) was added at 0-2 °C under argon atmosphere. The reaction mixture was stirred at room temperature for 3 hours. After the completion of the reaction (monitored by TLC) the mixture was concentrated *in vacuo.* The residue was dissolved in CH₂Cl₂ (20 mL), the pH was adjusted to 10 by the addition of 1 N NaOH solution (under cooling), the aqueous phase was extracted with CH₂Cl₂ (3x15 mL), the organic phase was dried over anhydrous Na₂SO₄, filtered and concentrated *in vacuo.* The crude product was purified by flash chromatography on silica, eluted by 10 % MeOH in CH₂Cl₂, to give 49 mg of the title compound as a yellow solid.

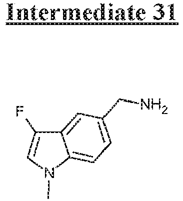

### (3-Fluoro-1-methyl-1H-indol-5-yl)methanamine

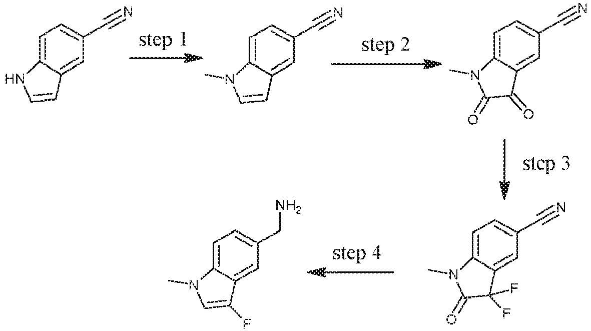

### Step 1: 1-Methyl-1H-indole-5-carbonitrile

To a solution of 1*H*-indole-5-carbonitrile (2.09 g, 14.7 mmol) in DMF (16 mL), NaH (840 mg, 60 percent in oil, 21 mmol) was added with vigorous stirring at 0 °C under argon atmosphere. The solution was stirred for 30 minutes, then iodomethane (9.12 g, 4 mL, 63 mmol) was added. The reaction mixture was stirred at room temperature for 4 hours. The reaction was quenched with water (50 mL), and extracted with EtOAc (3x120 mL). The organic layers were combined, washed with water (2x50 mL), dried over anhydrous Na₂SO₄, filtered and concentrated *in vacuo,* and dry toluene was evaporated from the residue to give 2.3 g of the title compound as an off white solid, used in the next step without any purification.

### Step 2: 1-Methyl-2,3-dioxo-2,3-dihydro-1H-indole-5-carbonitrile

To the solution of 1-methyl-1*H*-indole-5-carbonitrile (1.38 g, 8.83 mmol) in acetonitrile (40 mL), PCC (7.6 g, 35.2 mmol) was added and the mixture was refluxed for 7 hours. After the completion of the reaction (monitored by TLC), the reaction mixture was concentrated *in vacuo,* and the residue was partitioned between H₂O and EtOAc. The insoluble part was filtered off, the organic layer was washed with brine, dried over anhydrous Na₂SO₄, and the solvent was evaporated to dryness. The crude residue was purified by flash chromatography on silica eluted by CH₂Cl₂ to give 760 mg of the title compound as an orange solid.

### Step 3: 3,3-Difluoro-1-methyl-2-oxo-2,3-dihydro-1H-indole-5-carbonitrile

To the solution of bis(2-methoxyethyl)aminosulfur trifluoride (Deoxofluor, 50 % in toluene, 5 mL, 10 mmol) 1-methyl-2,3-dioxo-2,3-dihydro-1*H*-indole-5-carbonitrile (502 mg, 2.7 mmol) was added under argon atmosphere, then 25 µL abs. EtOH was added, and the mixture was heated at 90 °C for 1 hour.

The reaction was quenched by MeOH (1 mL) and diluted with CH₂Cl₂ (100 mL), then the mixture was poured onto cold saturated Na₂SO₄ solution, and the product was extracted with dichloromethane. The extract was washed with brine, dried over anhydrous Na₂SO₄, and the solvent was evaporated to dryness. The crude residue was purified by flash chromatography on silica, eluted by a gradient of 0-20 % EtOAc/hexane to give 322 mg of the title compound as a yellow solid.

### Step 4: (3-Fluoro-1-methyl-1H-indol-5-yl)methanamine

To the solution of 3,3-Difluoro-1-methyl-2-oxo-2,3-dihydro-1*H-*indole-5-carbonitrile (510 mg, 2.45 mmol) in dry THF (10 mL), BH₃.THF (1 M in THF) (7.5 mL, 7.5 mmol) was added at 0-2 °C under argon atmosphere. The reaction mixture was stirred at room temperature for 3 hours. After completion of the reaction (monitored by TLC), MeOH (10 mL) was added under cooling, and the mixture was concentrated *in vacuo.* The residue was dissolved in EtOAc (50 mL), washed with saturated NaHCO₃ solution (25 mL), then with brine (25 mL), dried over anhydrous Na₂SO₄, filtered and concentrated *in vacuo.* The crude product was purified by by flash chromatography on silica, eluted by 10 % MeOH in CH₂Cl₂ to give 70 mg of the title compound as light yellow solid.

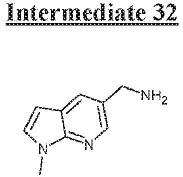

### {1-methyl-1H-pyrrolo[2,3-b]pyridin-5-yl}methanamine

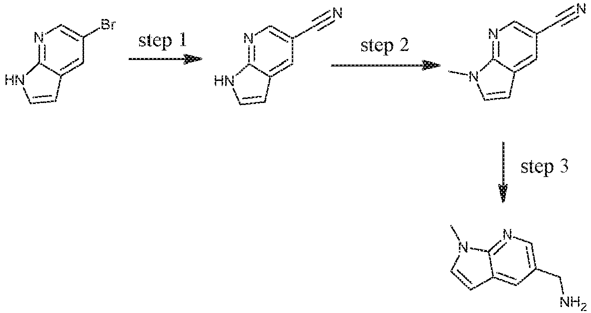

Compound 5-bromo-1*H*-pyrrolo[2,3-*b*]pyridine is commercially available from Combi Blocks (catalog no.: IN-0206) and the desired azaindole-amine is prepared in a sequence of steps illustrated above.

### Step 1: 1H-Pyrrolo[2,3-b]pyridine-5-carbonitrile

The intermediate is prepared as described in EP 1782811 A1 (EISAI R&D MAN CO LTD).

### Step 2: 1-Methyl-1H-pyrrolo[2.3-b]pyridine-5-carbonitrile

The intermediate is prepared as described in WO 2009/155017 A2 (MERCK & CO INC).

### Step 3: {1-Methyl-1H-pyrrolo[2.3-b]pyridin-5-yl}methanamine

The intermediate is prepared as described in WO 2012/042915 A1 (RAQUALIA PHARMA INC).

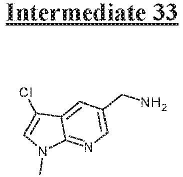

### {3-Chloro-1-methyl-1H pyrrolo[2,3-b]pyridin-5-yl}methanamine

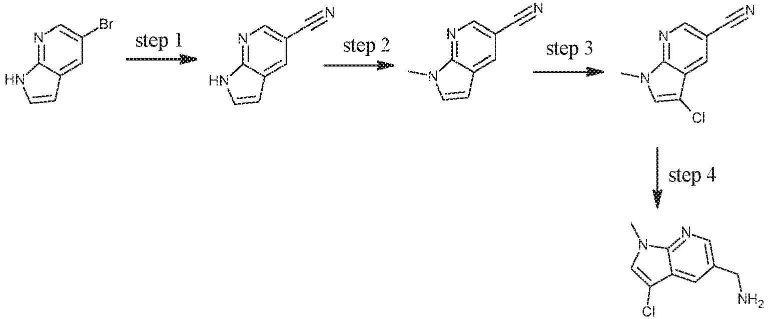

Compound 5-bromo-1*H*-pyrrolo[2,3-*b*]pyridine is commercially available from Combi Blocks (catalog no.: IN-0206), and the desired 3-chloroazaindole derivative is prepared in a sequence of steps illustrated above.

### Step 1: 1H-Pyrrolo[2.3-b]pyridine-5-carbonitrile

The intermediate is prepared as described in EP 1782811 A1 (EISAI R&D MAN CO LTD)

### Step 2: 1-Methyl-1H-pyrrolo[2.3-b]pyridine-5-carbonitrile

The intermediate is prepared as described in WO 2009/155017 A2 (MERCK & CO INC)

### Step 3: 3-Chloro-1-methyl-1H-pyrrolo[2.3-b]pyridine-5-carbonitrile

To a solution of 1-methyl-1H-pyrrolo[2,3-b]pyridine-5-carbonitrile (600 mg, 3.69 mmol) in DMF (14 mL), *N*-chlorosuccinimide (502 mg, 3.76 mmol) was added with vigorous stirring at room temperature under argon atmosphere. The solution was stirred at room temperature overnight. After completion of the reaction (monitored by TLC), the mixture was concentrated *in vacuo* (dry toluene was evaporated from the residue several times). The residue was dissolved in a mixture of diethyl ether - EtOAc (1:1, 40 mL), and washed with water (3x15 mL). The organic layer was dried over anhydrous Na₂SO₄, filtered and concentrated *in vacuo* to give 670 mg of the title compound, used in the next step without any purification.

### Step 4: {3-Chloro-1-methyl-1H-pyrrolo[2,3-b]pyridin-5-yl}methanamine

The intermediate is prepared as described in WO 2012/042915 A1 (RAQUALIA PHARMA INC).

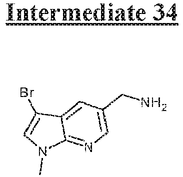

### {3-Bromo-1-methyl-1H-pyrrolo[2,3-b]pyridin-5-yl}methanamine

### Step 1-2:

The intermediates were prepared in a sequence of steps described for Intermediate 33, by the same methods as described in EP 1782811 A1 (EISAI R&D MAN CO LTD) and WO 2009/155017 A2 (MERCK & CO INC).

### Step 3: 3-Bromo-1-methyl-1H-pyrrolo[2,3-b]pyridine-5-carbonitrile

To a solution of 1-methyl-1*H*-pyrrolo[2,3-*b*]pyridine-5-carbonitrile (358 mg, 2.28 mmol) in CH₂Cl₂ (6.5 mL), *N*-bromosuccinimide (446 mg, 2.51 mmol) was added with vigorous stirring at room temperature under argon atmosphere. The solution was stirred at room temperature overnight. After completion of the reaction (monitored by TLC) the mixture was diluted with CH₂Cl₂ (25 mL) and washed with water (3x20 mL). The organic layer was dried over anhydrous Na₂SO₄, filtered and concentrated *in vacuo* to give 670 mg of the title compound, used in the next step without any purification.

### Step 4: {3-Bromo-1-methyl-1H-pyrrolo[2,3-b]pyridin-5-yl}methanamine

The intermediate is prepared as described in WO 2012/042915 A1 (RAQUALIA PHARMA INC).

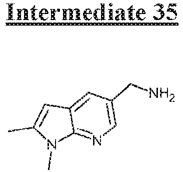

### {1,2-Dimethyl-1H-pyrrolo[2,3-b]pyridin-5-yl}methanamine

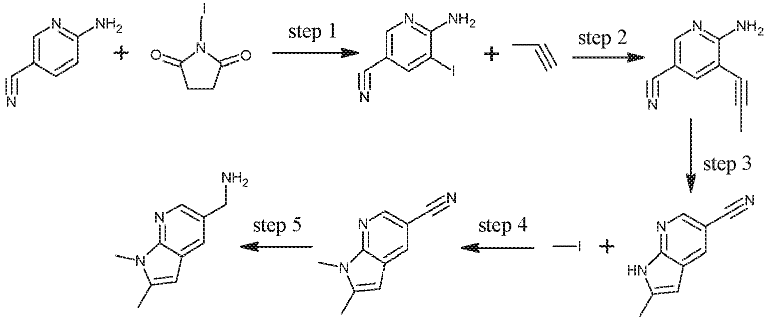

### Step 1: 6-Amino-5-iodopyridine-3-carbonitrile

To a solution of 2-amino-5-cyanopyridine (500 mg, 4.2 mmol) in DMF (5 mL), trifluoroacetic acid (574 mg, 386 uL, 1.2 mol equivalents) was added. At room temperature N-iodosuccinimide (1.04 g, 4.62 mmol, 1.1 mol equivalents) was added and the reaction mixture was heated at 50 °C for 3 hours. Complete conversion was indicated by TLC. After cooling the reaction mixture to room temperatures the product was precipitated by adding the reaction mixture to water. After neutralization with Na₂S₂O₃ and 1 N NaOH the title compound (660 mg) was collected by filtration as a brown solid. It was used in the next step without any purification.

### Step 2: 6-Amino-5-(prop-1-yn-1-yl)pyridine-3-carbonitrile

To the degassed mixture of 6-amino-5-(prop-1-yn-1-yl)pyridine-3-carbonitrile (329 mg, 1.34 mmol), bis'(triphenylphosphine)dichloropalladium(0) (95 mg, 0.134 mmol), copper (I) iodide (128 mg, 0.671 mmol) and triethylamine (976 mg, 1.34 mL, 9.64 mmol) in abs. THF (18 mL) a propyn solution (3-4 % in THF; 13.2 mL) was added via septum at 0-5 °C. The mixture was stirred for 30 minutes at 0-5 °C, then for a further 18 hours at room temperature. The reaction was quenched by the addition of NH₄Cl solution. The solid was removed by filtration and the cake was washed with CH₂Cl₂. The combined organic layer was dried with anhydrous Na₂SO₄, filtered and concentrated *in vacuo.* The crude product was purified by flash chromatography on silica, eluted by 40 % EtOAc in cyclohexane to give 150 mg of the title compound as a yellow solid (71 %).

### Step 3: 2-Methyl-1H-pyrrolo[2,3-b]pyridine-5-carbonitrile

To a solution of 6-amino-5-(prop-1-yn-1-yl)pyridine-3-carbonitrile (300 mg, 1.91 mmol) in DMF (5 mL), potassium *tert*-butoxide (428 mg, 3.82 mmol, 2 mol equivalents) was added. The reaction mixture was heated at 90 °C for 5.5 hours. Complete conversion was indicated by TLC. After cooling the reaction mixture to room temperature the mixture was poured onto water and extracted with CH₂Cl₂. The combined organic layer was dried with anhydrous Na₂SO₄, filtered and concentrated *in vacuo* to give 279 mg (93 %) of the title compound as a yellow solid. It was used in the next step without any purification.

### Step 4: 1.2-Dimethyl-1H-pyrrolo[2,3-b]pyridine-5-carbonitrile

To the solution of 2-methyl-1*H-*pyrrolo[2,3-*b*]pyridine-5-carbonitrile (279 mg, 1.78 mmol) in DMF (7.5 mL), sodium hydride (60 % in mineral oil) (92 mg, 2.31 mmol, 1.3 mol equivalents) was added at 0 °C. The reaction mixture was stirred at this temperature for 30 minutes, then iodomethane (380 mg, 167 uL) in DMF (1.5 mL) was added dropwise. The mixture was stirred at room temperature overnight. Complete conversion was indicated by TLC. The mixtute was poured onto water and extracted with CH₂Cl₂ (3x20 mL). The combined organic layer was dried over anhydrous Na₂SO₄, filtered and concentrated *in vacuo,* to give 298 mg (98 %) of the title compound as a yellow solid. It was used in the next step without any purification.

### Step 5: 1-(1,2-Dimethyl-1H-pyrrolo[2,3-b]pyridin-5yl)methanamine

To a solution of 1,2-dimethyl-1*H*-pyrrolo[2,3-*b*]pyridine-5-carbonitrile (297 mg, 1.73 mmol) in a mixture of MeOH (100 mL) and 25 % ammonia solution in water (25 mL), Raney-Ni (200 mg) was added and the mixture was stirred at 1 atm of Hz at room temperature for 16 hours. The reaction mixture was filtered through a celite pad, and the filtrate was concentrated under reduced pressure to give 226 mg (74 %) of the title compound as a yellow solid.

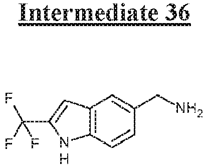

### [2-(Trifluoromethyl)-1H-indol-5-yl]methanamine

The intermediate is prepared as described in WO 2009/127678 A1 (GLAXO GROUP LTD).

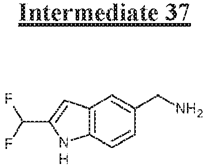

### [2-(difluoromethyl)-1H-indol-5-yl]methanamine

The compound ethyl 5-bromo-1H-indole-2-carboxylate is commercially available from Aldrich (catalog no.: 724718) and the desired 2-difluoromethylindole-amine is prepared in a sequence of steps illustrated below.

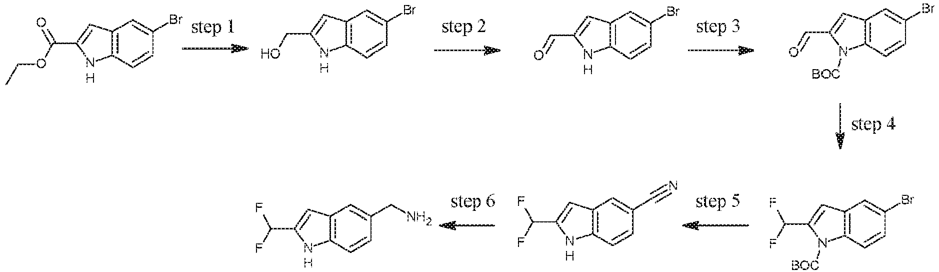

### Step 1: (5-Bromo-1H-indol-2-yl)methanol

To the solution 5-bromo-1*H*-indole-2-carboxylate (2.08 g, 7.77 mmol) in dry THF (30 mL), LiAlH₄ solution (1 M in THF) (7.0 mL , 7.0 mmol) was added at 0-2 °C under argon atmosphere, then the reaction mixture was stirred at room temperature for 4 hours. After the completion of the reaction (monitored by TLC), NH₄Cl solution (20 mL) was added under cooling, and the mixture was extracted with EtOAc (2x50 mL). The combined organic layer was washed with brine, dried over anhydrous Na₂SO₄, filtered and concentrated *in vacuo,* to give 1.73 g of the crude compound as a light brown solid. It was used in the next step without any purification.

### Step 2: 5-Bromo-1H-indole-2-carbaldehyde

To the solution (5-bromo-1*H*-indol-2-yl)methanol (1.0 g, 4.42 mmol) in dry acetonitrile (20 mL), MnO₂ (1.92 g, 22.1 mmol) was added, and the reaction mixture was stirred at room temperature overnight. After the completion of the reaction (monitored by TLC), the suspension was filtered off, and washed with EtOAc (5x15 mL). The combined organic layer was washed with brine, dried over anhydrous Na₂SO₄, filtered and concentrated *in vacuo.* The crude brown solid was crystallized from acetone to give 656 mg (66 % of the title compound.

### Step 3: tert-Butyl 5-bromo-2-formyl-1H-indole-1-carboxylate

To a stirred solution of 5-bromo-1*H*-indole-2-carbaldehyde (518 mg; 2.2 mmol) and 4-(dimethylamino)pyridine (27 mg; 0.22 mmol) in acetonitrile (20 mL), Boc₂O (965 mg; 4.4 mmol) was added at 0 °C under nitrogen atmosphere. The reaction mixture was stirred at room temperature overnight. TLC showed formation of the product. The reaction mixture was evaporated to dryness, and the crude product was purified by flash chromatography to give 664 mg (93 %) of the title compound as a yellow solid.

### Step 4: tert-Butvl 5-bromo-2-(difluoromethyl)-1H-indole-1-carboxylate

To the solution of tert-butyl 5-bromo-2-formyl-1*H*-indole-1-carboxylate (657 mg, 2.027 mmol) in dry dichloromethane (20 mL), Deoxo-Fluor^{®} (bis(2-methoxyethyl)(trifluorosulphanyl)amine) solution 50 % in THF (6.28 g, 14.2 mmol) was added, and the reaction mixture was stirred at room temperature overnight. After the completion of the reaction (monitored by TLC), the mixture was diluted with CH₂Cl₂, washed with saturated NaHCO₃ solution and brine. The organic phase was dried over anhydrous Na₂SO₄, filtered and concentrated *in vacuo.* The crude product was purified by flash chromatography to give 642 mg (91 %) of the title compound as a yellow oil.

### Step 5: 2-(pifluoromethyl)-1H-indole-5-carbonitrile

To the solution of *tert*-butyl 5-bromo-2-(difluoromethyl)-1*H*-indole-1-carboxylate (639 mg, 1.848 mmol) in NMP (15 mL), zinc cyanide (326 mg, 2.77 mmol) and tetrakis(triphenylphosphine)palladium(0) (214 mg, 0.184 mmol) was added, and the reaction mixture was stirred at 70 °C for 3 hours, then at 90 °C for 3 hours under argon atmosphere. The progress of the reaction was followed by TLC. The mixture was poured onto water (100 mL), and extracted with EtOAc (3x30 mL). The combined organic phase was washed with water (4x50 mL) and brine. The organic phase was dried over anhydrous Na₂SO₄, filtered and concentrated *in vacuo.* The crude product was purified by flash chromatography to give 241 mg (68 %) of the title compound as a white solid.

### Step 6: [2-(Difluoromethyl)-1H-indol-5-yl]methanamine

To a solution of 2-(difluoromethyl)-1*H-*indole-5-carbonitrile (211 mg, 1.08 mmol) in a mixture of MeOH (24 mL) and 25 % ammonia solution in water (6 mL), Raney-Ni (100 mg) was added and the mixture was stirred at 1 atm of H₂ at room temperature for 3 hours. The reaction mixture was filtered through a celite pad, then washed with MeOH (3x10 mL), and then the filtrate was concentrated under reduced pressure to give 218 mg (approximately 100 %) of the title compound as a light brown solid.

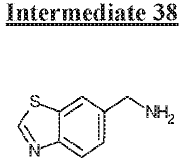

### 1-(1,3-benzothiazol-6-yl)methanamine

The intermediate is prepared as described in WO 2011/079804 A1 (HUTCHISON MEDIPHARMA LTD) or US 20100298314 A1 (SCHERING CORP).

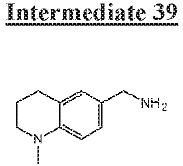

### 1-(1-Methyl-1,2,3,4-tetrahydroquinolin-6-yl)methanamine

The compound is commercially available from Enamine (catalog no.: EN300-57206).

### Example 1

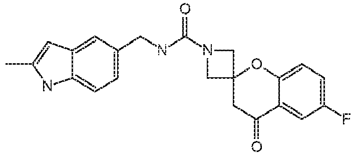

### 6'-Fluoro-N-[(2-methyl-1H-indol-5-yl)methyl]-4'-oxo-3',4'-dihydrospiro[azetidine-3,2'-[1]benzopyran]-1-carboxamide

To the mixture of 800 mg (5.0 mmol) of 2-(methyl-1*H*-indol-5-yl)methanamine (Intermediate 26) and 1.295 g (1.75 mL, 10 mmol) of DIPEA in 120 mL of dichloromethane (CH₂Cl₂), 4-nitrophenyl chloroformate (1020 mg, 5.06 mmol) was added at 0 °C under argon atmosphere. The light yellow suspension was stirred for 1 hour under this condition. After the activation period, the mixture of 6'-fluoro-3',4'-dihydrospiro[azetidine-3,2'-[1]benzopyran]-4'-one hydrochloride (1.22 g, 5 mmol, Intermediate 6) and 1.75 mL DIPEA (7.75 mmol) in 30 mL CH₂Cl₂ was added. After addition, the mixture was allowed to warm to room temperature and was stirred at this temperature for 22 hours. After the completion of the reaction (monitored by TLC), the mixture was washed with water (2x40 mL), then with brine. The organic layer was dried over anhydrous Na₂SO₄, filtered and concentrated *in vacuo,* to give 1.8 g of the crude compound. The residue was chromatographed on silica gel, eluting with a mixture of CH₂Cl₂ and MeOH (10:1) to yield 1.02 g (50 %) of the title compound. LC-MS (ESI) m/z [M+H] ⁺ = 394.1

The following examples in Table 3 were synthesized according to the procedure described for Example 1.

**Table 3**

| **Example** | **Structure** | **Intermediates** | **LC-MS (ESI) [M+H]⁺** |
|---|---|---|---|
| **2** | 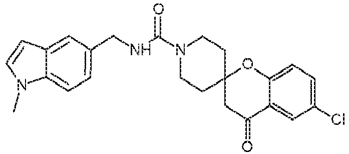 | Int. 28 and Int. 2 | 438.2 |
| **3** | 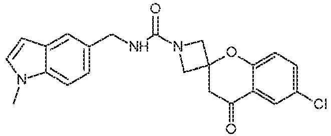 | Int. 28 and Int. 1 | 410.1 |
| **4** | 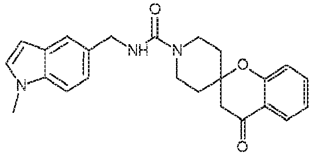 | Int. 27 and Int. 5 | 404.2 |
| **5** | 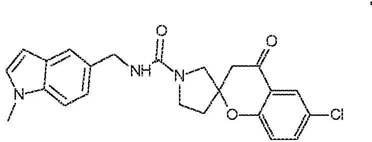 | Int. 27 and Int. 3 | 424.1 |
| **5-A^{a}** | 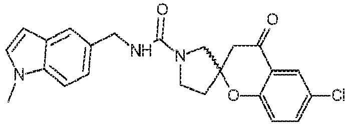 | Int. 27 and Int. 3 | 424.1 |
| **5-B^{a}** | 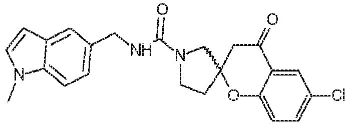 | Int. 27 and Int. 3 | 424.1 |
| **6** | 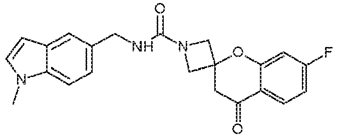 | Int. 27 and Int. 19 | 394.1 |
| **7** | 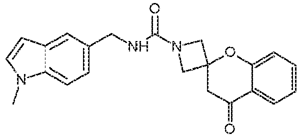 | Int. 27 and Int. 4 | 376.2 |
| **8** | 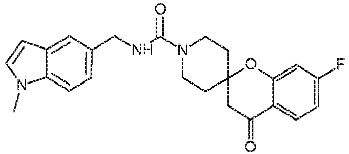 | Int. 27 and Int. 16 | 422.2 |
| **9** | 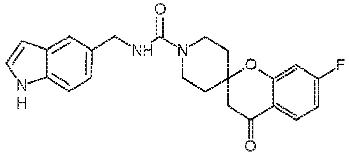 | Int. 26 and Int. 16 | 408.2 |
| **10** | 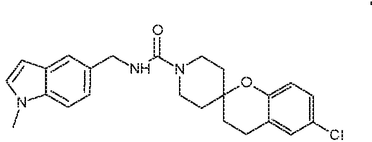 | Int. 27 and Int. 21 | 424.2 |
| **11** | 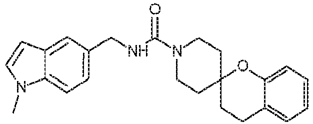 | Int. 27 and Int. 23 | 390.2 |
| **12** | 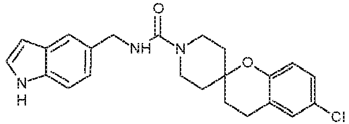 | Int. 26 and Int. 21 | 410.2 |
| **13** | 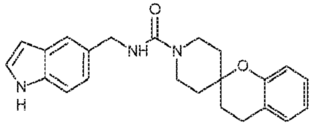 | Int. 26 and Int. 23 | 376.2 |
| **14** | 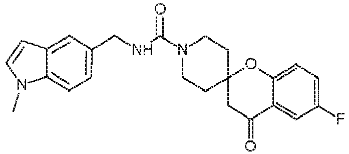 | Int. 27 and Int. 7 | 422.2 |
| **15** | 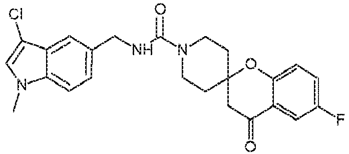 | Int. 30 and Int.7 | 456.2 |
| **16** | 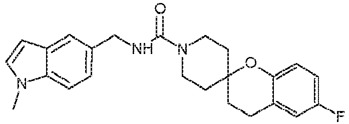 | Int. 27 and Int. 22 | 408.2 |
| **17** | 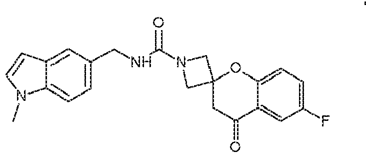 | Int. 27 and Int. 6 | 394.1 |
| **18** | 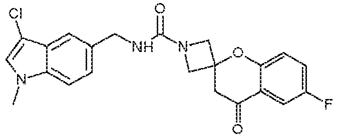 | Int. 30 and Int. 6 | 428.1 |
| **19** | 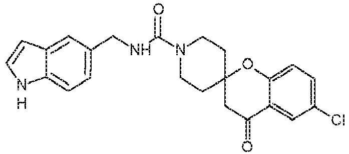 | Int. 26 and Int. 2 | 424.1 |
| **20** | 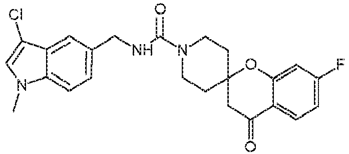 | Int. 30 and Int. 7 | 456.2 |
| **21** | 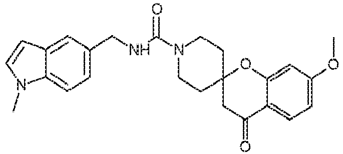 | Int. 27 and Int. 9 | 434.2 |
| **22** | 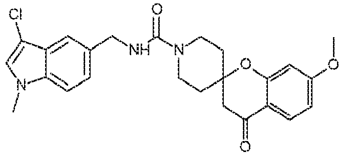 | Int. 30 and Int. 9 | 468.2 |
| **23** | 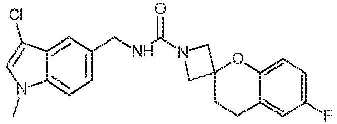 | Int. 30 and Int. 24 | 414.1 |
| **24** | 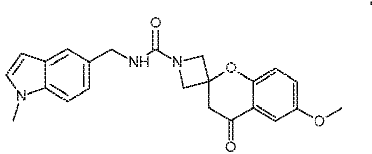 | Int. 27 and Int. 8 | 406.2 |
| **25** | 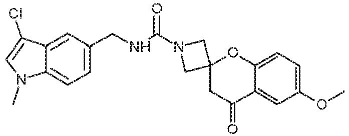 | Int. 30 and Int. 8 | 440.1 |
| **26** | 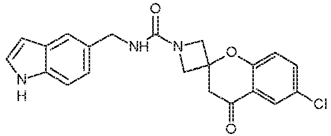 | Int. 26 and Int. 1 | 396.1 |
| **27** | 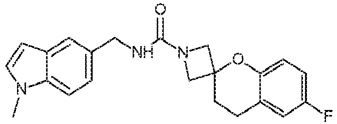 | Int. 27 and Int. 24 | 380.2 |
| **28** | 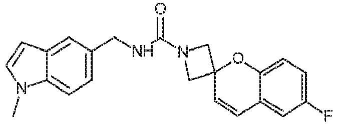 | Int. 27 and Int. 25 | 378.2 |
| **29** | 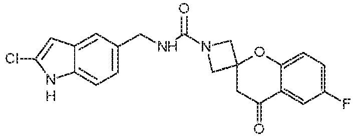 | Int. 29 and Int. 6 | 414.1 |
| **30** | 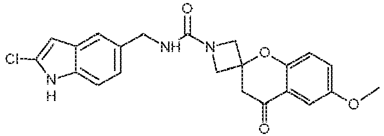 | Int. 29 and Int. 8 | 426.1 |
| **31** | 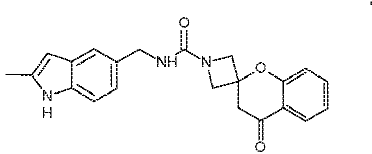 | Int. 28 and Int. 4 | 376.2 |
| **32** | 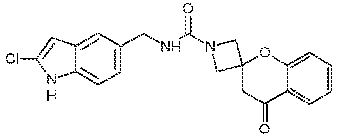 | Int. 29 and Int. 4 | 396.1 |
| **33** | 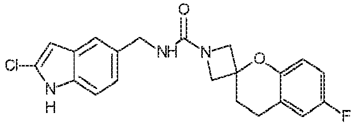 | Int. 29 and Int. 24 | 400.1 |
| **34** | 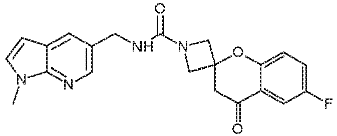 | Int. 32 and Int. 6 | 395.2 |
| **35** | 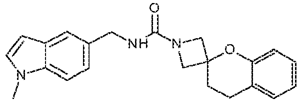 | Int. 27 and Int. 20 | 362.2 |
| **36** | 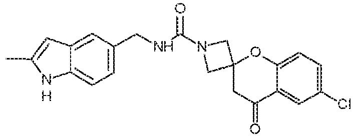 | Int. 28 and Int. 1 | 410.1 |
| **37** | 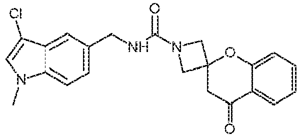 | Int. 30 and Int. 4 | 410.1 |
| **38** | 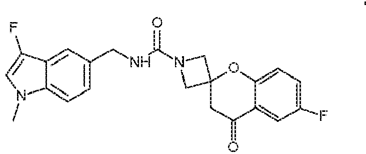 | Int. 31 and Int. 6 | 412.2 |
| **39** | 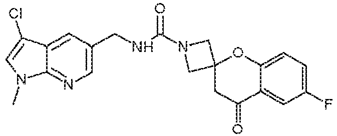 | Int. 33 and Int. 6 | 429.1 |
| **40** | 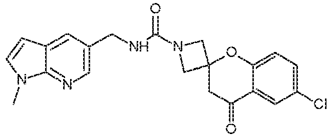 | Int. 32 and Int. 1 | 411.1 |
| **41** | 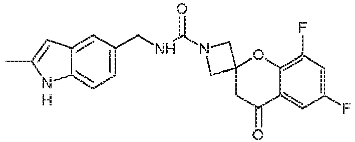 | Int. 28 and Int. 10 | 412.2 |
| **42** | 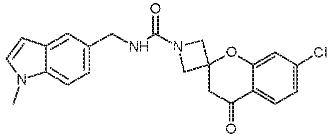 | Int. 27 and Int. 17 | 410.1 |
| **43** | 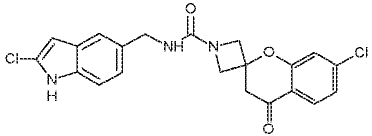 | Int. 29 and Int. 17 | 430.1 |
| **44** | 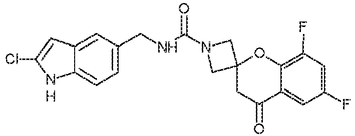 | Int. 29 and Int. 10 | 432.2 |
| **45** | 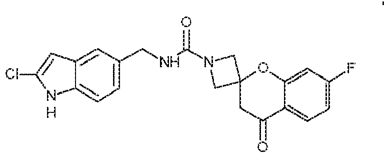 | Int. 29 and Int. 19 | 414.1 |
| **46** | 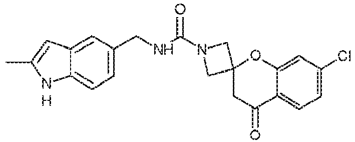 | Int. 28 and Int. 17 | 410.2 |
| **47** | 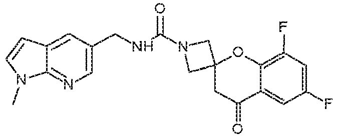 | Int. 32 and Int. 10 | 413.2 |
| **48** | 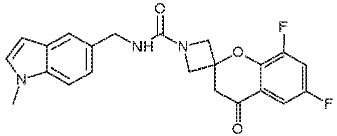 | Int.27 and Int.10 | 421.1 |
| **49** | 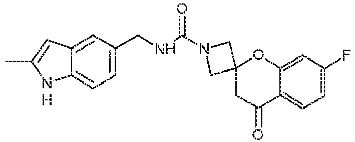 | Int. 28 and Int. 19 | 394.2 |
| **50** | 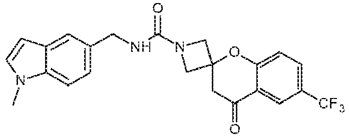 | Int. 27 and Int. 12 | 444.1 |
| **51** | 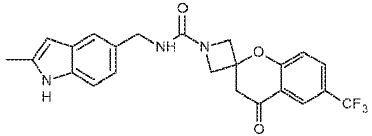 | Int. 28 and Int. 12 | 444.2 |
| **52** | 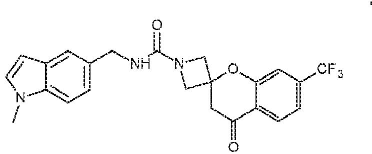 | Int. 27 and Int. 13 | 444.1 |
| **53** | 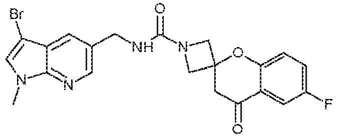 | Int. 34 and Int. 6 | 473.1 |
| **54** | 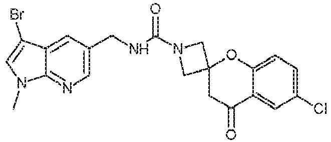 | Int. 34 and Int. 1 | 489.1 |
| **55** | 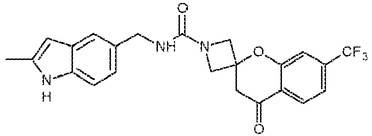 | Int. 28 and Int. 13 | 444.1 |
| **56** | 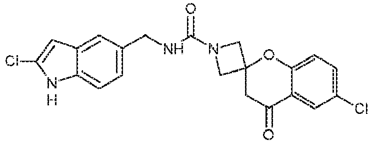 | Int. 29 and Int. 1 | 430.1 |
| **57** | 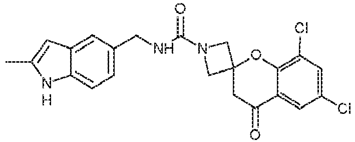 | Int. 28 and Int. 15 | 444.1 |
| **58** | 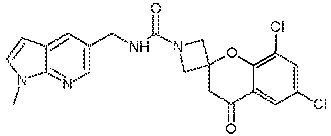 | Int. 32 and Int. 15 | 445.1 |
| **59** | 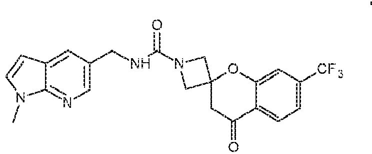 | Int. 32 and Int. 13 | 445.2 |
| **60** | 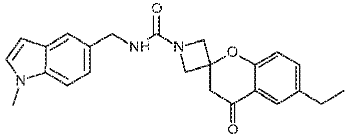 | Int. 27 and Int. 14 | 404.2 |
| **61** | 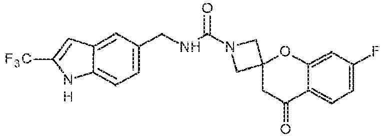 | Int. 36 and Int. 19 | 448.2 |
| **62** | 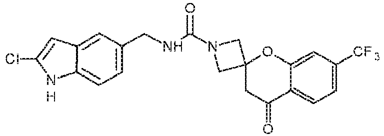 | Int. 29 and Int. 13 | 464.1 |
| **63** | 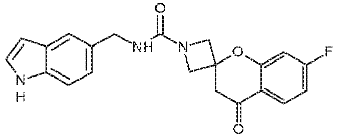 | Int. 26 and Int. 19 | 380.1 |
| **64** | 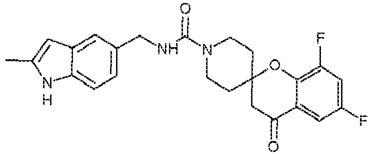 | Int. 28 and Int. 11 | 440.2 |
| **65** | 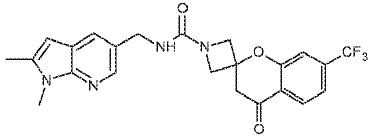 | Int. 35 and Int. 13 | 459.1 |
| **66^{b}** | 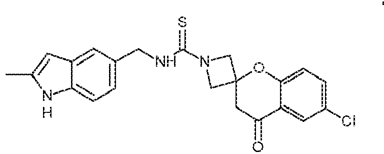 | Int. 28 and Int. 1 | 426.2 |
| **67** | 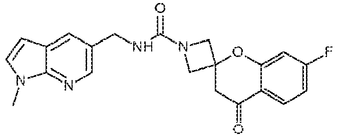 | Int. 32 and Int. 19 | 395.1 |
| **68** | 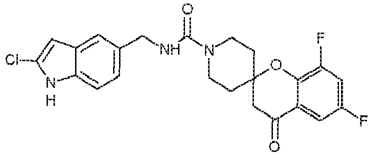 | Int. 29 and Int. 11 | 460.2 |
| **69** | 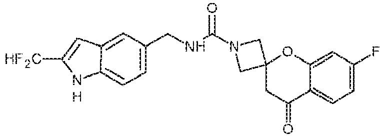 | Int. 37 and Int. 19 | 430.3 |
| **70** | 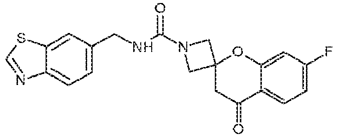 | Int. 38 and Int. 19 | 398.2 |
| **71^{c}** | 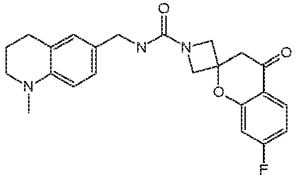 | Int. 39 and Int. 19 | 410.2 |
| **72^{d}** | 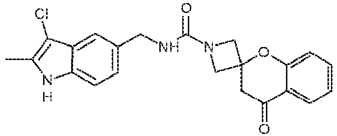 | Example 31 | 410.1 |
| **73** | 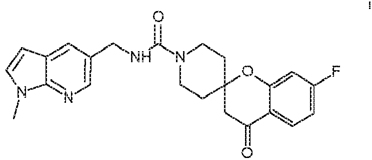 | Int. 32 and Int. 16 | 423.2 |
| **74^{e}** | 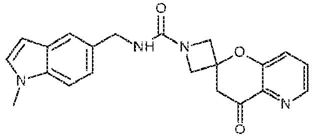 | Int. 27 and Int. 18 | 377.2 |

^{a}The racemic form of the title compound Example 5 was prepared from Intermediate 26 and Intermediate 3 according to the methods described for Example 1. The two (A and B) enantiomers were separated using chiral preparative HPLC. Their absolute configuration is not determined. ^{b,c,d,e}Syntheses see below, respectively.

### Example 66^{b}

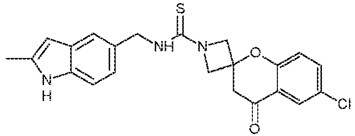

### 6'-Chloro-N-[(2-methyl-1H-indol-5-yl)methyl]-4'-oxo-3',4'-dihydrospiro[azetidine-3,2'-[1]benzopyran]-1-carbothioamide

To a solution of 80.1 mg (0.5 mmol) of 2-(methyl-1*H*-indol-5-yl)methanamine (Intermediate 28) in 2 mL of DMF, 1,1'-thiocarbonyl-diimidazole (98 mg, 0.55 mmol) was added at room temperature under argon atmosphere. The brownish-yellow solution was stirred for 1 hour under this condition. After the activation period, the mixture of 6'-chloro-3',4'-dihydrospiro[azetidine-3,2'-[1]benzopyran]-4'-one hydrochloride (130 mg, 0.5 mmol, Intermediate 1) and 0.131 mL of DIPEA (0.75 mmol) in 2 mL DMF was added, and the mixture was stirred at this temperature for 20 hours. After the completion of the reaction (monitored by TLC), the mixture was poured onto water (8 mL) and extracted with EtOAc (2x15 mL). The organic layer was dried over anhydrous Na₂SO₄, filtered and concentrated *in vacuo.* The residue was chromatographed on silica gel, eluting with a mixture of CH₂Cl₂ and MeOH (10:1) to yield 100 mg (47 %) of the title compound. LC-MS (ESI) m/z [M+H] ⁺ = 426.2

### Example 71^{c}

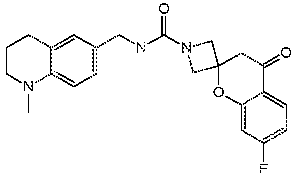

### 7'-Fluoro-N-[(1-methyl-1,2,3,4-tetrahydroquinolin-6-yl)methyl]-4'-oxo-3',4'-dihydrospiro[azetidine-3,2'-[1]benzopyran]-1-carboxamide

To the mixture of 88 mg (0.5 mmol) of 1-(1-methyl-1,2,3,4-tetrahydroquinolin-6-yl)methanamine (Intermediate 39) and 175 µL (1.0 mmol) of DIPEA in 20 mL of dichloromethane (CH₂Cl₂), 4-nitrophenyl chloroformate (111 mg, 0.55 mmol) was added at 0 °C under argon atmosphere. The light yellow suspension was stirred for 1.5 hours under this condition. After the activation period, the mixture of 7'-fluoro-3',4'-dihydrospiro[azetidine-3,2'-[1]benzopyran]-4'-one hydrochloride (122 mg, 0.5 mmol, Intermediate 19) and 131 µL DIPEA (0.75 mmol) in 5 mL CH₂Cl₂ was added. After addition, the mixture was allowed to warm to room temperature, and stirred at this temperature for 24 hours. After the completion of the reaction (monitored by TLC), the mixture was washed with water (2x20 mL), then with brine. The organic layer was dried over anhydrous Na₂SO₄, filtered and concentrated *in vacuo* to give 300 mg of the crude compound. The residue was chromatographed on silica gel, eluting with a mixture of CH₂Cl₂ and MeOH (99:1), to yield 30 mg (15 %) of the title compound. LC-MS (ESI) m/z [M+H] ⁺ = 410.2

### Example 72^{d}

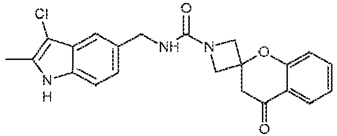

### N-[(3-Chloro-2-methyl-1H-indol-5-yl)methyl]-4'-oxo-3',4'-dihydrospiro[azetidine-3,2'-[1]benzopyran]-1-carboxamide

To a solution of N-[(2-methyl-1H-indol-5-yl)methyl]-4'-oxo-3',4'-dihydrospiro[azetidine-3,2'-[1]benzopyran]-1-carboxamide (Example 31) (100 mg, 0.266 mmol) in DCM (2 mL), N-chlorosuccinimide (36 mg, 0.266 mmol) was added with vigorous stirring at room temperature under argon atmosphere. The solution was stirred at room temperature overnight. After the completion of the reaction (monitored by TLC), the mixture was diluted with DCM (20 mL), and washed with water (3x15 mL). The organic layer was dried over anhydrous sodium sulfate, filtered and concentrated *in vacuo.* The crude product was purified by column chromatography to give 35 mg of the title compound. LC-MS (ESI) m/z [M+H] ⁺ = 410.1

### Example 74^{e}

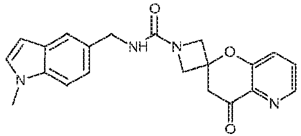

### N-[(1-methyl-1H-indol-5-yl)methyl]-4'-oxo-3',4'-dihydrospiro[azetidine-3,2'-pyrano[3,2- b]pyridine]-1-carboxamide

To the mixture of 56 mg (0.35 mmol) of (1-methyl-1*H*-indol-5-yl)methanamine (Intermediate 27) and 122 µL (0.7 mmol) of *N,N*- diisopropylethylamine (DIPEA) in 10 mL of dichloromethane (CH₂Cl₂), 4-nitrophenyl chloroformate (71 mg, 0.35 mmol) was added at 0 °C under argon atmosphere. The light yellow suspension was stirred for 1 hour under this condition. After the activation period, the mixture of 3',4'-dihydrospiro[azetidine-3,2'-pyrano[3,2-b]pyridin]-4'-one hydrochloride (80 mg, 0.35 mmol, Intermediate 18) and 92 µL DIPEA (0.53 mmol) in 2 mL CH₂Cl₂ was added. After addition, the mixture was allowed to warm to room temperature, and stirred at this temperature for 24 hours. After the completion of the reaction (monitored by TLC), the mixture was washed with water (2x10 mL), then with brine. The organic layer was dried over anhydrous Na₂SO₄, filtered and concentrated *in vacuo,* to give 60 mg of the crude compound. The residue was chromatographed on silica gel, eluting with a mixture of CH₂Cl₂ and MeOH (99:1) to yield 7 mg (5 %) of the title compound. LC-MS (ESI) m/z [M+H] ⁺ = 377.2

### Preparation of pharmaceutical compositions

The following formulation examples illustrate representative pharmaceutical compositions of this invention. The present invention, however, is not limited to the following pharmaceutical compositions.

### A) Solid oral dosage forms

### I., Tablets

| | |
|---|---|
| Active ingredient(s) | 0.01 - 90 % |
| Filler | 1 - 99.9% |
| Binder | 0 - 20 % |
| Disintegrant | 0 - 20 % |
| Lubricant | 0 - 10 % |
| Other specific excipient(s) | 0 - 50 % |

### II., Orodispersible films

| | |
|---|---|
| Active ingredient(s) | 0.01 - 90 % |
| Film forming agent | 1 - 99.9 % |
| Plasticizer | 0 - 40 % |
| Other specific excipient(s) | 0 - 50 % |

### B) Liquid oral dosage forms

### III., Oral suspensions

| | |
|---|---|
| Active ingredient(s) | 0.01 - 50 % |
| Liquid vehicle | 10 - 99.9 % |
| Wetting agent | 0 - 50 % |
| Thickener | 0 - 50 % |
| Buffering agent | quantum satis |
| Osmotic agent | 0 - 50 % |
| Preservatives | quantum satis |

### IV., Syrups

| | |
|---|---|
| Active ingredient(s) | 0.01 - 50 % |
| Solvent | 10 - 99.9 % |
| Sugar component | 1 - 20 % |
| Flavouring agents | 0 - 10 % |

### C) Parenteral dosage forms

### V., Intravenous injections

| | |
|---|---|
| Active ingredient(s) | 0.01 - 50 % |
| Solvent | 10 - 99.9 % |
| Co-solvent | 0 - 99.9 % |
| Osmotic agent | 0 - 50 % |
| Buffering agent | quantum satis |

### D) Other dosage forms

### VI., Suppositories

| | |
|---|---|
| Active ingredient(s) | 0.01 - 50 % |
| Suppository base | 1 - 99.9 % |
| Surface-active agents | 0 - 20 % |
| Lubricants | 0 - 20 % |
| Preservatives | quantum satis |

### VII., Nasal drops or nasal sprays

| | |
|---|---|
| Active ingredient(s) | 0.01 - 50 % |
| Water | 0 - 99.9 % |
| Solvent | 0 - 99.9 % |
| Co-solvent | 0 - 99.9 % |
| Osmotic agent | 0 - 20 % |
| Viscosity enhancer | 0 - 20 % |
| Buffering agent | quantum satis |
| Preservatives | quantum satis |

### BIOLOGICAL ACTIVITY

### Human α7 nicotinic acetylcholine receptor [Ca2+]i assay

Cells: Flp-In 293 cells stably expressing human α7 nAchR and human RIC-3 (α7 cells, generated in house.)

Materials: 96-well plates coated with PDL (Falcon), culture medium, assay buffer, DMSO, FLIPR Calcium 5 kit (Molecular Devices), probenecid, agonist and PAM test compounds.

Culture medium: Assay buffer:

| | |
|---|---|
| - DMEM (Dulbecco's Modified Eagle Medium, Gibco) | - 140 mM NaCl |
| - 10 % FBS (Fetal Bovine Serum, Gibco) | - 5 mM KCl |
| - 1 % glutamine (Sigma G) | - 10 mM HEPES |
| - 50 µg/ml Hygromycin B | - 2 mM MgCl₂ |
| - 800 µg/ml G418 | - 2 mM CaCl₂ |
| - 1 % penicillin-streptomycin-antimycotic sol. | - 10 mM glucose |
| (PSA, Sigma) | - 2 mM probenecid, |
| | pH=7.4 |

### Brief description of the method (Ca²⁺ fluorometry)

α7 cells cells stably expressing human α7 nAchR were cultured in the medium detailed above, and were split twice a week. For the fluorometric measurements of cytosolic Ca²⁺ ion concentration ([Ca²⁺]ᵢ) cells were seeded in 96-well microplates at a density of 60000 cells/well and maintained overnight in a tissue culture incubator at 37 °C under an atmosphere of 95 % air/5 % CO₂. The plating medium was identical with the culture medium. 50 µl of the growth medium was aspirated with a cell washer (BioTek Elx405UCVWS). Then 50 µl/well Calcium 5 kit diluted 2-fold in assay buffer was added manually using an 8-channel pipette. After an incubation period (20 minutes, 37 °C) 50 µl/well assay buffer containing vehicle (DMSO, 4 % added) or reference α7 PAMs (4× of the final concentration) were added manually and the cells were incubated for an additional 10 minutes at 37 °C. Baseline and agonist-evoked [Ca²⁺]ᵢ-changes were monitored with FlexStation II (Molecular Devices, Sunnyvale, CA), a plate reader fluorometer with integrated 8-channel fluid addition capability. Fluorescence measurements were carried out at 37 °C. The dye was excited at 485 nm, emission was sampled at 525 nm at 1.4-s intervals. Baseline was recorded for 20 seconds followed by agonist stimulation. 50 µl 4× concentrated agonist solution was added to the cells using the pipettor of FlexStation II and fluorescence was monitored for an additional 40 seconds. Final DMSO concentration was 1 % for all treatments. To achieve this, a series of DMSO stock solutions were prepared from all test compounds. These stocks were stored under 0 °C and were further diluted in assay buffer to obtain the desired final concentration immediately before the measurement. Agonist and PAM concentration-response studies were conducted in the presence of saturating concentrations of PAMs (mostly PNU-120596, 5 µM) and agonists (mostly PNU-282987, 1 µM), respectively. Results were expressed as ΔF/F values using SoftMax Pro software (Molecular Devices), where F was the resting fluorescence preceding agonist application and ΔF was the increase in fluorescence at a given time (ΔF = maximum fluorescence intensity values after stimulation minus average fluorescence intensity values before stimulation). In all experiments, all treatments were measured in multiple wells in parallel, and the mean ΔF/F values were used for analysis. Table 4 shows the PAM EC₅₀ values in the [Ca²⁺]ᵢ assay:

**Table 4**

| **Example** | **EC₅₀ (nM)** |
|---|---|
| **1** | 120 |
| **3** | 120 |
| **5** | 210 |
| **5a** | 430 |
| **5b** | 400 |
| **6** | 190 |
| **7** | 230 |
| **10** | 100 |
| **21** | 280 |
| **25** | 230 |
| **26** | 390 |
| **37** | 130 |
| **38** | 220 |
| **39** | 730 |
| **41** | 60 |
| **43** | 360 |
| **50** | 150 |
| **52** | 130 |
| **53** | 850 |
| **58** | 470 |
| **59** | 500 |
| **65** | 750 |
| **69** | 120 |
| **71** | 2300 |

### In vivo pharmacology (place recognition test)

### Animals: Male NMRI mice (Toxicoop, Hungary)

Substances: Scopolamine was dissolved in saline and administered at 1 mg/kg dose i.p. Test compounds were administered 30 minutes before the acquisition trial (T1) and scopolamine after the acquisition trial at a volume of 0.1 ml/10 g.

Procedure: The task was carried out in a transparent plexiglass Y-maze (each arm has a length of 40 cm, an inner width of 11 cm and a height of 30 cm). Numerous visual cues were placed around the arms and were kept constant during the experiment. The test consisted of two trials (T1 and T2) separated by an intertrial interval of 30 minutes. Mice were placed in the starting arm of the maze at the beginning of each trial. In T1, one of the symmetric arms of the maze was closed (it will be novel in T2) and the animals were allowed to explore the maze for 5 minutes (acquisition phase). In T2, mice had free access to all three arms for 2 minutes (retrieval phase). The time spent with exploration in the novel and familiar arms during T2 was measured. Differences between the exploration times spent in the familiar vs. novel arms of the maze for each group were evaluated by MANOVA, followed by Duncan post hoc test.

Table 5 shows the reversal of the scopolamine-induced amnesia in the place recognition assay in mice:

**Table 5**

| | **Dose (i.p.)** | | |
|---|---|---|---|
| | **1 mg/kg** | **3 mg/kg** | **10 mg/kg** |
| **Example 1** | ++ | - | - |
| **Example 6** | + | ++ | +++ |
| **Example 7** | +++ | +++ | +++ |
| **Example 26** | - | + | - |
| **Example 41** | + | + | - |
| **Example 43** | + | +++ | +++ |
| **Example 52** | - | - | + |
| **Example 61** | - | ++ | +++ |

| | | | |
|---|---|---|---|
| ⁺p<0.05; ⁺⁺p<0.01; ⁺⁺⁺p<0.001 | | | |

Significant differences (⁺p<0.05; ⁺⁺p<0.01; ⁺⁺⁺p<0.001) were observed between the exploration times spent in the novel vs. familiar arms of the maze.

## Claims

1. A compound of formula (I) for use in the treatment or prevention of a disease associated with α7 nicotinic acetylcholine receptor activity, 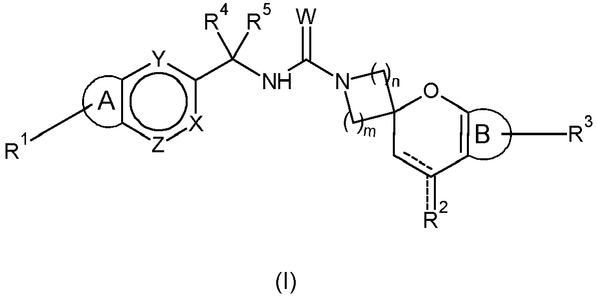 wherein:
**A** is a five or six membered heterocycle;
**B** is a six membered carbocycle or heterocycle;
**X** is C or N;
**Y** is CorN;
**Z** is CorN;
**W** is O or S;
**R¹** is H, C₁₋₆alkyl, halogen or haloC₁₋₆alkyl;
**R²** is H or O;
**R³** is H, C₁₋₆alkyl, halogen, haloC₁₋₆alkyl or C₁₋₆alkoxy;
**R⁴** is H or C₁₋₆alkyl;
**R⁵** is H or C₁₋₆alkyl;
**n and m** are independently 1 or 2;
- - - is a single - or double-bond;
or pharmaceutically acceptable salts, racemates, enantiomers, diastereomers, solvates and hydrates thereof.

2. A compound for use according to claim 1, wherein
**A** is five membered heterocycle, wherein the members of the ring are selected from the group consisting of carbon, nitrogen, oxygen, and sulphur;
**B** is a six membered carbocycle or a heterocycle, wherein the members of the ring are selected from the group consisting of carbon, nitrogen, oxygen, and sulphur; **X** is C;
**Y** is C;
**Z** is C or N;
**W** is O or S;
**R¹** is H, C₁₋₆alkyl, halogen or halo C₁₋₆alkyl;
**R²** is H or O;
**R³** is H, C₁₋₆alkyl, halogen, haloC₁₋₆alkyl or C₁₋₆alkoxy;
**R⁴** is H;
**R⁵** is H;
**n and m** are independently 1 or 2;
- - - is a single - or double-bond.

3. A compound of formula (II), 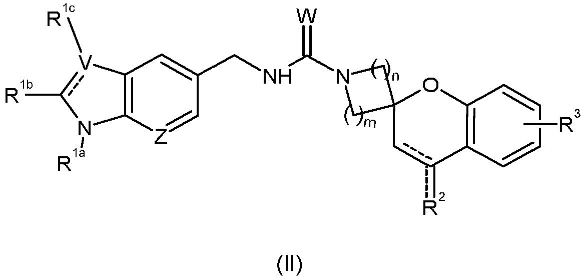 wherein
**V** is C or S;
**Z** is C or N;
**W** is O or S;
**R^{1a}** is H, C₁₋₆alkyl, or haloC₁₋₆alkyl;
**R^{1b}** is H, C₁₋₆alkyl, halogen, or haloC₁₋₆alkyl;
**R^{1c}** is H, C₁₋₆alkyl, halogen, or haloC₁₋₆alkyl when V is carbon; or R^{1c} is absent when V is sulphur;
**R²** is H or O;
**R³** is H, C₁₋₆alkyl, halogen, haloC₁₋₆alkyl, or C₁₋₆alkoxy;
**n and m** are independently 1 or 2;
- - - is a single - or double-bond;
or pharmaceutically acceptable salts, racemates, enantiomers, diastereomers, solvates and hydrates thereof.

4. A compound according to claim 3, wherein
**V** is C;
**Z** is C or N;
**W** is O;
**R^{1a}** is H, C₁₋₆alkyl, or haloC₁₋₆alkyl;
**R^{1b}** is H, C₁₋₆alkyl, halogen, or haloC₁₋₆alkyl;
**R^{1c}** is H, C₁₋₆alkyl, halogen, or haloC₁₋₆alkyl;
**R²** is H or O;
**R³** is H, C₁₋₆alkyl, halogen, or haloC₁₋₆alkyl;
**n and m** are independently 1 or 2;
- - - is a single - or double-bond.

5. A compound as defined in any one of claims 1 or 4, selected from the group of:
6'-Fluoro-N-[(2-methyl-1H-indol-5-yl)methyl]-4'-oxo-3',4'-dihydrospiro[azetidine-3,2'-[1]benzopyran]-1-carboxamide;
6-chloro-N-[(1-methyl-1H-indol-5-yl)methyl]-4-oxo-3,4-dihydrospiro[1-benzopyran-2,4'-piperidine]-1'-carboxamide;
6'-chloro-N-[(1-methyl-1H-indol-5-yl)methyl]-4'-oxo-3',4'-dihydrospiro[azetidine-3,2'-[1]benzopyran]-1-carboxamide;
7'-fluoro-N-[(1-methyl-1H-indol-5-yl)methyl]-4'-oxo-3',4'-dihydrospiro[azetidine-3,2'-[1]benzopyran]-1-carboxamide;
N-[(1-methyl-1H-indol-5-yl)methyl]-4'-oxo-3',4'-dihydrospiro[azetidine-3,2'-[1]benzopyran]-1-carboxamide;
6-chloro-N-[(1-methyl-1H-indol-5-yl)methyl]-3,4-dihydrospiro[1]-benzopyran-2,4'-piperidine]-1'-carboxamide;
N-[(3-chloro-1-methyl-1H-indol-5-yl)methyl]-6'-fluoro-4'-oxo-3',4'-dihydrospiro[azetidine-3,2'-[1]benzopyran]-1-carboxamide;
6'-chloro-N-[(1H-indol-5-yl)methyl]-4'-oxo-3',4'-dihydrospiro[azetidine-3,2'-[1]benzopyran]-1-carboxamide;
6'-fluoro-N-[(1-methyl-1H-indol-5-yl)methyl]-3',4'-dihydrospiro[azetidine-3,2'-[1]benzopyran]-1-carboxamide;
6'-fluoro-N-[(1-methyl-1H-indol-5-yl)methyl] spiro[azetidine-3,2'-chromene]-1-carboxamide;
N-[(1-methyl-1H-indol-5-yl)methyl]-3',4'-dihydrospiro[azetidine-3,2'-[1]benzopyran]-1-carboxamide;
6'-chloro-N-[(2-methyl-1H-indol-5-yl)methyl]-4'-oxo-3',4'-dihydrospiro[azetidine-3,2'-[1]benzopyran]-1-carboxamide;
6',8'-difluoro-N-[(2-methyl-1H-indol-5-yl)methyl]-4'-oxo-3',4'-dihydrospiro[azetidine-3,2'-[1]benzopyran]-1-carboxamide;
7'-chloro-N-[(2-chloro-1H-indol-5-yl)methyl]-4'-oxo-3',4'-dihydrospiro[azetidine-3,2'-[1]benzopyran]-1-carboxamide;
7'-chloro-N-[(2-methyl-1H-indol-5-yl)methyl]-4'-oxo-3',4'-dihydrospiro[azetidine-3,2'-[1]benzopyran]-1-carboxamide;
7'-fluoro-N-[(2-methyl-1H-indol-5-yl)methyl]-4'-oxo-3',4'-dihydrospiro[azetidine-3,2'-[1]benzopyran]-1-carboxamide;
N-[(2-methyl-1H-indol-5-yl)methyl]-4'-oxo-6'-(trifluoromethyl)-3',4'-dihydrospiro[azetidine-3,2'-[1]benzopyran]-1-carboxamide;
N-[(1-methyl-1H-indol-5-yl)methyl]-4'-oxo-7'-(trifluoromethyl)-3',4'-dihydrospiro[azetidine-3,2'-[1]benzopyran]-1-carboxamide;
N-[(2-methyl-1H-indol-5-yl)methyl]-4'-oxo-7'-(trifluoromethyl)-3',4'-dihydrospiro[azetidine-3,2'-[1]benzopyran]-1-carboxamide;
6', 8'-dichloro-N-[(2-methyl-1H-indol-5-yl)methyl]-4'-oxo-3',4'-dihydrospiro[azetidine-3,2'-[1]benzopyran]-1-carboxamide;
7'-fluoro-4'-oxo-N-{[2-(trifluoromethyl)-1H-indol-5-yl]methyl}-3',4'-dihydrospiro[azetidine-3,2'-[1]benzopyran]-1-carboxamide;
N-{[2-(difluoromethyl)-1H-indol-5-yl]methyl}-7'-fluoro-4'-oxo-3',4'-dihydrospiro[azetidine-3,2'-[1]benzopyran]-1-carboxamide;
or pharmaceutically acceptable salts, racemates, enantiomers, diastereomers,
solvates and hydrates thereof.

6. A compound according to any one of claims 3 to 5, for use in the treatment or prevention of a disease associated with α7 nicotinic acetylcholine receptor activity.

7. A compound for use according to any one of claims 1, 2 and 6, wherein the disease is selected from the group of psychotic disorders, including, but not limited to, schizophrenia, schizophreniform disorder, schizoaffective disorder, delusional disorder, brief psychotic disorder, psychotic disorder due to a general medical condition, substance-induced psychotic disorder or psychotic disorder not otherwise specified, cognitive impairment, including, but not limited to, cognitive impairment as a result of stroke, Alzheimer's disease, Huntington's disease, Pick disease, HIV associated dementia, frontotemporal dementia, Lewy body dementia, vascular dementia, cerebrovascular disease or other dementia states and dementia associated to other degenerative disorders, including, but not limited to, amyotrophic lateral sclerosis, other acute or sub-acute conditions that may cause cognitive decline, including, but not limited to, delirium, traumatic brain injury, senile dementia, mild cognitive impairment, Down's syndrome, depression and cognitive deficit related to other diseases, and dyskinetic disorders including, but not limited to, Parkinson's disease, neuroleptic-induced parkinsonism, or tardive dyskinesias, depression and mood disorders, including, but not limited to, depressive disorders and episodes, bipolar disorders, cyclothymic disorder, and bipolar disorder not otherwise specified, other mood disorders, substance-induced mood disorder and mood disorder not otherwise specified, anxiety disorders, panic disorder and panic attacks, obsessive compulsive disorder, posttraumatic stress disorder, acute stress disorder, generalized anxiety disorder, anxiety disorder due to a general medical condition, substance-induced anxiety disorder, phobias, and anxiety disorder not otherwise specified, substance related disorders, including, but not limited to, substance use or substance-induced disorders, including, but not limited to, alcohol-, nicotine-, amphetamine-, phencyclidine-, opioid-, cannabis-, cocaine-, caffeine-, hallucinogen-, inhalant-, sedative-, hypnotic-, anxiolytic-, polysubstance- or other substance-related disorders, sleep disorders, including, but not limited to, narcolepsy, dyssomnias, primary hypersomnia, breathing-related sleep disorders, circadian rhythm sleep disorder and dyssomnia not otherwise specified, parasomnias, sleep terror disorder, sleepwalking disorder and parasomnia not otherwise specified, sleep disorders related to another mental disorder, sleep disorder due to a general medical condition and substance-induced sleep disorder, metabolic and eating disorders, including, but not limited to, anorexia nervosa, bulimia nervosa, obesity, compulsive eating disorder, binge eating disorder and eating disorder not otherwise specified, diabetes mellitus, ulcerative colitis, Crohn's disease, irritable bowel syndrome, autism spectrum disorders, including, but not limited to, autistic disorder, Asperger's disorder, Rett's disorder, childhood disintegrative disorder and pervasive developmental disorder not otherwise specified, attention deficit hyperactivity disorder, disruptive behaviour disorders, oppositional defiant disorder and disruptive behaviour disorder not otherwise specified, and tic disorders, including, but not limited to, Tourette's disorder, personality disorders, sexual dysfunctions such as sexual desire disorders, sexual arousal disorders, orgasmic disorders, sexual pain disorder, sexual dysfunction not otherwise specified, paraphilias, gender identity disorders, infertility, premenstrual syndrome and sexual disorders not otherwise specified, disorders of the respiratory system like cough, asthma, chronic obstructive pulmonary disease, lung inflammation, disorders of the cardiovascular system such as cardiac failure, heart arrhythmia, hypertension, inflammation, inflammatory and neuropathic pain, rheumatoid arthritis, osteoarthritis, allergy, sarcoidosis, psoriasis, ataxia, dystonia, systemic lupus erythematosus, mania, restless legs syndrome, progressive supranuclear palsy, epilepsy, myoclonus, migraine, amnesia, chronic fatigue syndrome, cataplexy, brain ischemia, multiple sclerosis, encephalomyelitis, jetlag, cerebral amyloid angiopathy, and sepsis.

8. A compound for use according to claim 7, wherein the disease is selected from the group of cognitive impairment, schizophrenia, and autism.

9. A pharmaceutical composition comprising as active ingredient a compound as defined in any one of claims 1 to 5 and at least one pharmaceutically acceptable excipient.

10. A pharmaceutical composition according to claim 9, wherein the composition further comprises at least one other active ingredient.

11. A pharmaceutical composition according to claim 10, wherein the other active ingredient(s) are selected from the group of acetylcholinesterase inhibitors, NMDA receptor antagonists, beta- secretase inhibitors, antipsychotics, GABA_{A} receptor alpha5 subunit NAMs or PAMs, histamine H₃ receptor antagonists, 5-HT₆ receptor antagonists, M1 or M4 mAChR agonists or PAMs, mGluR2 antagonists or NAMs or PAMs, and levodopa.

12. Combination of a compound as defined in any one of claims 1 to 5 and at least one other active ingredient for use in the treatment or prevention of a disease associated with α7 nicotinic acetylcholine receptor activity.

13. Combination for use according to claim 12, wherein the other active ingredient(s) are selected from the group of acetylcholinesterase inhibitors, NMDA receptor antagonists, beta- secretase inhibitors, antipsychotics, GABA_{A} receptor alpha5 subunit NAMs or PAMs, histamine H₃ receptor antagonists, 5-HT₆ receptor antagonists, M1 or M4 mAChR agonists or PAMs, mGluR2 antagonists or NAMs or PAMs, and levodopa.

14. Process for the manufacture of compounds of formula (II) according to claim 3, **characterized by** reacting a compound of formula (III) 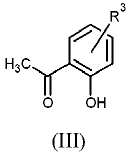
- wherein the meaning of R³ is described above for compound of formula (II) - with a compound of formula (IV) 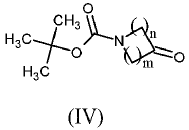
- wherein the meaning of n and m is described above for compound of formula (II) - either in two separated steps via formula (VII) 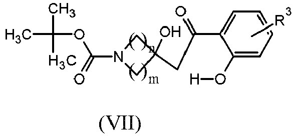
- wherein the meaning of n and m is described above for compound of formula (II),
or directly in one step to provide a compound of formula (V) 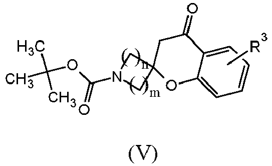
- wherein the meaning of R³, n and m is as described above for formula (II) - which is then reacted with
a.) hydrogen chloride to provide formula (VI) 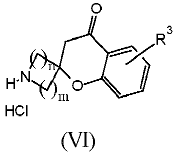
- wherein the meaning of R³, n and m is as described above for formula (II),
b.) complex hydrides to provide formula (VIII) 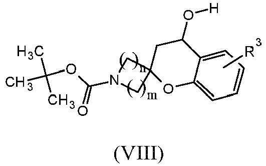
- wherein the meaning of R³, n and m is as described above for formula (II) - then compound of formula (VIII) is reduced with triethylphosphine to provide formula (IX) 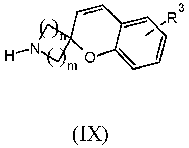
- wherein the meaning of R³, n and m is as described above for formula (II) - then the obtained derivative of formula (IX) or formula (VI) -is reacted with formula (X) 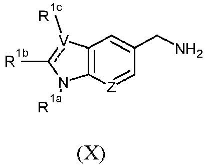
- wherein the meaning of R^{1a}, R^{1b}, R^{1c},V, and Z is as described above for formula (II) - providing formula (II) 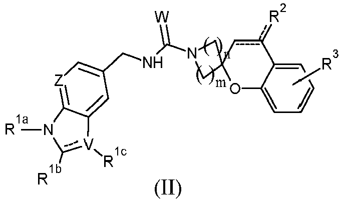
- wherein the meaning of R^{1a}, R^{1b}, R^{1c}, R², R³,V, Z, n, m, and W are as described above for formula (II).

15. A compound of {1,2-dimethyl-1H-pyrrolo[2,3-b]pyridin-5-yl}methanamine.

16. A compound of {3-Bromo-1-methyl-1H-pyrrolo[2,3-b]pyridin-5-yl}methanamine.

17. A compound of 3',4'-Dihydrospiro[azetidine-3,2'-pyrano[3,2-b]pyridin]-4'-one hydrochloride.

## Patentansprüche

1. Verbindung der Formel (I) zur Verwendung bei der Behandlung oder Vorbeugung einer Krankheit, die mit α7-Nicotin-Acetylcholin-Rezeptor-Aktivität assoziiert ist, 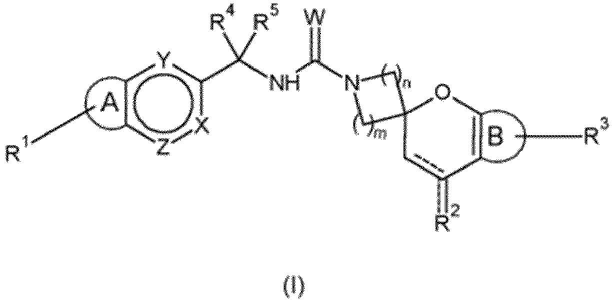 worin:
**A** ein fünf- oder sechsgliedriger Heterocyclus ist;
**B** ein sechsgliedriger Carbocyclus oder Heterocyclus ist;
**X** C oder N ist;
**Y** C oder N ist;
**Z** C oder N ist;
**W** 0 oder S ist;
**R¹** H, C₁₋₆-Alkyl, Halogen oder Halogen-C₁₋₆-Alkyl ist;
**R²** H oder 0 ist;
**R³** H, C₁₋₆-Alkyl, Halogen, Halogen-C₁₋₆-Alkyl oder C₁₋₆-Alkoxy ist;
**R⁴** H oder C₁₋₆-Alkyl ist;
**R⁵** H oder C₁₋₆-Alkyl ist;
**n und m** sind unabhängig voneinander 1 oder 2;
- - - eine Einzel- oder Doppelbindung ist;
oder pharmazeutisch akzeptable Salze, Racemate, Enantiomere, Diastereomere, Solvate und Hydrate davon.

2. Verbindung zur Verwendung gemäß Anspruch 1, worin
**A** ein fünfgliedriger Heterocyclus ist, worin die Glieder des Rings ausgewählt sind, aus der Gruppe bestehend aus Kohlenstoff, Stickstoff, Sauerstoff und Schwefel;
**B** ein sechsgliedriger Carbocyclus oder ein Heterocyclus ist, worin die Glieder des Rings ausgewählt sind, aus der Gruppe bestehend aus Kohlenstoff, Stickstoff, Sauerstoff und Schwefel;
**X** C ist;
**Y** C ist;
**Z** C oder N ist;
**W** 0 oder S ist;
**R¹** H, C₁₋₆-Alkyl, Halogen oder Halogen-C₁₋₆-Alkyl ist;
**R²** H oder 0 ist;
**R³** H, C₁₋₆-Alkyl, Halogen, Halogen-C₁₋₆-Alkyl oder C₁₋₆-Alkoxy ist;
**R⁴** H ist;
**R⁵** H ist;
**n und m** sind unabhängig voneinander 1 oder 2;
- - - eine Einzel- oder Doppelbindung ist.

3. Verbindung der Formel (II), 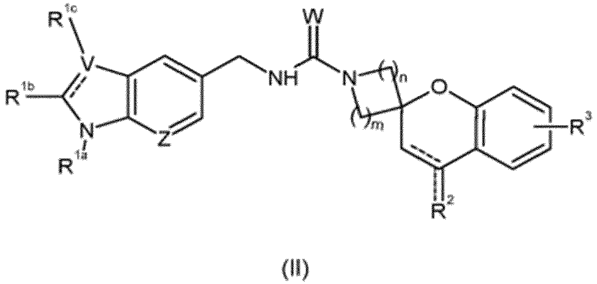 worin
**V** C oder S ist;
**Z** C oder N ist;
**W** 0 oder S ist;
**R^{1a}** H, C₁₋₆-Alkyl oder Halogen-C₁₋₆-Alkyl ist;
**R^{1b}** H, C₁₋₆-Alkyl, Halogen oder Halogen-C₁₋₆-Alkyl ist;
**R^{1c}** H, C₁₋₆-Alkyl, Halogen oder Halogen-C₁₋₆-Alkyl ist, wenn V Kohlenstoff ist; oder R^{1c} ist nicht vorhanden, wenn V Schwefel ist;
**R²** H oder 0 ist;
**R³** H, C₁₋₆-Alkyl, Halogen, Halogen-C₁₋₆-Alkyl oder C₁₋₆-Alkoxy ist;
**n und m** sind unabhängig voneinander 1 oder 2;
- - - eine Einzel- oder Doppelbindung ist;
oder pharmazeutisch akzeptable Salze, Racemate, Enantiomere, Diastereomere, Solvate und Hydrate davon.

4. Verbindung gemäß Anspruch 3, worin
**V** C ist;
**Z** C oder N ist;
**W** O ist;
**R^{1a}** H, C₁₋₆-Alkyl oder Halogen-C₁₋₆-Alkyl ist;
**R^{1b}** H, C₁₋₆-Alkyl, Halogen oder Halogen-C₁₋₆-Alkyl ist;
**R^{1c}** H, C₁₋₆-Alkyl, Halogen oder Halogen-C₁₋₆-Alkyl ist;
**R²** H oder 0 ist;
**R³** H, C₁₋₆-Alkyl, Halogen oder Halogen-C₁₋₆-Alkyl ist;
**n und m** sind unabhängig voneinander 1 oder 2;
- - - eine Einzel- oder Doppelbindung ist.

5. Verbindung gemäß mindestens einem der Ansprüche 1 oder 4, ausgewählt aus der folgenden Gruppe:
6'-Fluor-N-[(2-methyl-1H-indol-5-yl)methyl]-4'-oxo-3',4'-dihydrospiro[azetidin-3,2'-[1]benzopyran]-1-carboxamid;
6-Chlor-N-[(1-methyl-1H-indol-5-yl)methyl]-4-oxo-3,4-dihydrospiro[1-benzopyran-2,4'-piperidin]-1'-carboxamid;
6'-Chlor-N-[(1-methyl-1H-indol-5-yl)methyl]-4'-oxo-3',4'-dihydrospiro[azetidin-3,2'-[1]benzopyran]-1-carboxamid;
7'-Fluor-N-[(1-Methyl-1H-indol-5-yl)methyl]-4'-oxo-3',4'-dihydrospiro[azetidin-3,2'-[1]benzopyran]-1-carboxamid;
N-[(1-Methyl-1H-indol-5-yl)methyl]-4'-oxo-3',4'-dihydrospiro[azetidin-3,2'-[1]benzopyran]-1-carboxamid;
6-Chlor-N-[(1-methyl-1H-indol-5-yl)methyl]-3,4-dihydrospiro[1]-benzopyran-2,4'-piperidin]-1'-carboxamid;
N-[(3-Chlor-1-methyl-1H-indol-5-yl)methyl]-6'-fluor-4'-oxo-3',4'-dihydrospiro[azetidin-3,2'-[1]benzopyran]-1-carboxamid;
6'-Chlor-N-[(1H-indol-5-yl)methyl]-4'-oxo-3',4'-dihydrospiro[azetidin-3,2'-[1]benzopyran]-1-carboxamid;
6'-Fluor-N-[(1-methyl-1H-indol-5-yl)methyl]-3',4'-dihydrospiro[azetidin-3,2'-[1]benzopyran]-1-carboxamid;
6'-Fluor-N-[(1-methyl-1H-indol-5-yl)methyl]spiro[azetidin-3,2'-chromen]-1-carboxamid;
N-[(1-Methyl-1H-indol-5-yl)methyl]-3',4'-dihydrospiro[azetidin-3,2'-[1]benzopyran]-1-carboxamid;
6'-Chlor-N-[(2-methyl-1H-indol-5-yl)methyl]-4'-oxo-3',4'-dihydrospiro[azetidin-3,2'-[1]benzopyran]-1-carboxamid;
6',8'-Difluor-N-[(2-methyl-1H-indol-5-yl)methyl]-4'-oxo-3',4'-dihydrospiro[azetidin-3,2'-[1]benzopyran]-1-carboxamid;
7'-Chlor-N-[(2-chlor-1H-indol-5-yl)methyl]-4'-oxo-3',4'-dihydrospiro[azetidin-3,2'-[1]benzopyran]-1-carboxamid;
7'-Chlor-N-[(2-methyl-1H-indol-5-yl)methyl]-4'-oxo-3',4'-dihydrospiro[azetidin-3,2'-[1]benzopyran]-1-carboxamid;
7'-Fluor-N-[(2-methyl-1H-indol-5-yl)methyl]-4'-oxo-3',4'-dihydrospiro[azetidin-3,2'-[1]benzopyran]-1-carboxamid;
N-[(2-Methyl-1H-indol-5-yl)methyl]-4'-oxo-6'-(trifluormethyl)-3',4'-dihydrospiro[azetidin-3,2'-[1]benzopyran]-1-carboxamid;
N-[(1-Methyl-1H-indol-5-yl)methyl]-4'-oxo-7'-(trifluormethyl)-3',4'-dihydrospiro[azetidin-3,2'-[1]benzopyran]-1-carboxamid;
N-[(2-Methyl-1H-indol-5-yl)methyl]-4'-oxo-7'-(trifluormethyl)-3',4'-dihydrospiro[azetidin-3,2'-[1]benzopyran]-1-carboxamid;
6',8'-Dichlor-N-[(2-methyl-1H-indol-5-yl)methyl]-4'-oxo-3',4'-dihydrospiro[azetidin-3,2'-[1]benzopyran]-1-carboxamid;
7'-Fluor-4'-oxo-N-{[2-(trifluormethyl)-1H-indol-5-yl]methyl}-3',4'-dihydrospiro[azetidin-3,2'-[1]benzopyran]-1-carboxamid;
N-{[2-(Difluormethyl)-1H-indol-5-yl]methyl}-7'-fluor-4'-oxo-3',4'-dihydrospiro[azetidin-3,2'-[1]benzopyran]-1-carboxamid;
oder pharmazeutisch akzeptable Salze, Racemate, Enantiomere, Diastereomere, Solvate und Hydrate davon.

6. Verbindung gemäß mindestens einem der Ansprüche 3 bis 5, zur Verwendung bei der Behandlung oder Vorbeugung einer Krankheit, die mit α7-Nicotin-Acetylcholin-Rezeptor-Aktivität assoziiert ist.

7. Verbindung zur Verwendung gemäß mindestens einem der Ansprüche 1, 2 und 6, wobei die Krankheit ausgewählt ist, aus der Gruppe von psychotischen Störungen, einschließlich, aber nicht beschränkt auf, Schizophrenie, schizophreniforme Störung, schizoaffektive Störung, wahnhafte Störung, kurze psychotische Störung, psychotische Störung aufgrund eines allgemeinen medizinischen Zustands, substanzinduzierte psychotische Störung oder nicht anderweitig spezifizierte psychotische Störung, kognitive Beeinträchtigung, einschließlich, aber nicht beschränkt auf kognitive Beeinträchtigung infolge eines Schlaganfalls, der Alzheimer-Krankheit, der Huntington-Krankheit, der Pick-Krankheit, der HIV-assoziierten Demenz, der frontotemporalen Demenz, der Lewy-Körper-Demenz, der vaskulären Demenz, der zerebrovaskulären Krankheit oder anderer Demenzzustände und der Demenz assoziiert mit anderen degenerativen Erkrankungen, einschließlich, aber nicht beschränkt auf amyotrophe Lateralsklerose, andere akute oder subakute Zustände, die einen kognitiven Verfall verursachen können, einschließlich, aber nicht beschränkt auf Delirium, traumatische Hirnverletzungen, senile Demenz, leichte kognitive Beeinträchtigung, Down-Syndrom, Depression und kognitive Defizite im Zusammenhang mit anderen Krankheiten, und dyskinetische Störungen, einschließlich, aber nicht beschränkt auf, Parkinson-Krankheit, Neuroleptika-induzierter Parkinsonismus oder tardive Dyskinesien, Depressionen und Stimmungsstörungen, einschließlich, aber nicht beschränkt auf depressive Störungen und Episoden, bipolare Störungen, zyklothymische Störungen und nicht anderweitig spezifizierte bipolare Störungen, andere Stimmungsstörungen, substanzinduzierte Stimmungsstörungen und nicht anderweitig spezifizierte Stimmungsstörungen, Angststörungen, Panikstörungen und Panikattacken, Zwangsstörungen, posttraumatische Belastungsstörungen, akute Belastungsstörungen, generalisierte Angststörungen, Angststörungen aufgrund eines allgemeinen medizinischen Zustands, substanzinduzierte Angststörungen, Phobien und nicht anderweitig spezifizierte Angststörungen, substanzbezogene Störungen, einschließlich, aber nicht beschränkt auf, Substanzgebrauch oder substanzinduzierte Störungen, einschließlich, aber nicht beschränkt auf Alkohol-, Nikotin-, Amphetamin-, Phencyclidin-, Opioid-, Cannabis-, Kokain-, Koffein-, Halluzinogen-, Inhalations-, Sedativa-, Hypnotika-, Anxiolytika-, Polysubstanz- oder andere substanzbezogene Störungen, Schlafstörungen, einschließlich, aber nicht beschränkt auf Narkolepsie, Dyssomnien, primäre Hypersomnie, Schlafstörungen im Zusammenhang mit der Atmung, Störungen des zirkadianen Schlafrhythmus und nicht anderweitig spezifizierte Dyssomnie, Parasomnien, Schlafterror, Schlafwandeln und nicht anderweitig spezifizierte Parasomnie, Schlafstörungen im Zusammenhang mit einer anderen psychischen Störung, Schlafstörungen aufgrund eines allgemeinen medizinischen Zustands und substanzinduzierte Schlafstörungen, Stoffwechsel- und Essstörungen, einschließlich, aber nicht beschränkt auf Anorexia nervosa, Bulimia nervosa, Adipositas, zwanghafte Essstörungen, Binge-Eating-Störungen und nicht anderweitig spezifizierte Essstörungen, Diabetes mellitus, Colitis ulcerosa, Morbus Crohn, Reizdarmsyndrom, Autismus-Spektrum-Störungen, einschließlich, aber nicht beschränkt auf autistische Störungen, Asperger-Syndrom, Rett-Syndrom, Desintegrative Störungen im Kindesalter und tiefgreifende Entwicklungsstörungen, die nicht anderweitig spezifiziert sind, Aufmerksamkeitsdefizit-Hyperaktivitätsstörungen, Störungen des störenden Verhaltens, oppositionelle Verhaltensstörungen und Störungen des störenden Verhaltens, die nicht anderweitig spezifiziert sind, und Tic-Störungen, einschließlich, aber nicht beschränkt auf das Tourette-Syndrom, Persönlichkeitsstörungen, sexuelle Funktionsstörungen wie Störungen des sexuellen Verlangens, Störungen der sexuellen Erregung, Orgasmusstörungen, Störungen des sexuellen Schmerzes, nicht anderweitig spezifizierte sexuelle Funktionsstörungen, Paraphilien, Störungen der Geschlechtsidentität, Unfruchtbarkeit, prämenstruelles Syndrom und nicht anderweitig spezifizierte sexuelle Störungen, Störungen des Atmungssystems wie Husten, Asthma, chronisch obstruktive Lungenerkrankung, Lungenentzündung, Störungen des Herz-Kreislauf-Systems wie Herzinsuffizienz, Herzrhythmusstörungen, Bluthochdruck, Entzündungen, entzündliche und neuropathische Schmerzen, rheumatoide Arthritis, Osteoarthritis, Allergie, Sarkoidose, Psoriasis, Ataxie, Dystonie, systemischer Lupus erythematodes, Manie, Restless-Legs-Syndrom, progressive supranukleäre Lähmung, Epilepsie, Myoklonus, Migräne, Amnesie, chronisches Müdigkeitssyndrom, Kataplexie, Hirnischämie, Multiple Sklerose, Enzephalomyelitis, Jetlag, zerebrale Amyloid-Angiopathie und Sepsis.

8. Verbindung zur Verwendung gemäß Anspruch 7, wobei die Krankheit ausgewählt ist aus der Gruppe von kognitiver Beeinträchtigung, Schizophrenie und Autismus.

9. Pharmazeutische Zusammensetzung umfassend als Wirkstoff eine Verbindung gemäß mindestens einem der Ansprüche 1 bis 5 und zumindest einen pharmazeutisch akzeptablen Hilfsstoff.

10. Pharmazeutische Zusammensetzung gemäß Anspruch 9, wobei die Zusammensetzung ferner zumindest einen weiteren Wirkstoff umfasst.

11. Pharmazeutische Zusammensetzung gemäß Anspruch 10, wobei der/die andere(n) Wirkstoff(e) ausgewählt sind aus der Gruppe von Acetylcholinesterase-Inhibitoren, NMDA-Rezeptor-Antagonisten, Beta-Sekretase-Inhibitoren, Antipsychotika, GABA_{A}-Rezeptor-alpha5-Untereinheit NAMs oder PAMs, Histamin-H₃-Rezeptor-Antagonisten, 5-HT₆-Rezeptor Antagonisten, M1- oder M4-mAChR-Agonisten oder PAMs, mGluR2-Antagonisten oder NAMs oder PAMs, und Levodopa.

12. Kombination einer Verbindung gemäß mindestens einem der Ansprüche 1 bis 5 und zumindest einem weiteren Wirkstoff zur Verwendung bei der Behandlung oder Vorbeugung einer Krankheit, die mit α7-Nicotin-Acetylcholin-Rezeptor-Aktivität assoziiert ist.

13. Kombination zur Verwendung gemäß Anspruch 12, wobei der/die andere(n) Wirkstoff(e) ausgewählt sind aus der Gruppe von Acetylcholinesterase-Inhibitoren, NMDA-Rezeptor-Antagonisten, Beta-Sekretase-Inhibitoren, Antipsychotika, GABA_{A}-Rezeptor-alpha5-Untereinheit NAMs oder PAMs, Histamin-H₃-Rezeptor-Antagonisten, 5-HT₆-Rezeptor Antagonisten, M1- oder M4-mAChR-Agonisten oder PAMs, mGluR2-Antagonisten oder NAMs oder PAMs, und Levodopa.

14. Verfahren zur Herstellung einer Verbindung der Formel (II) gemäß Anspruch 3, **gekennzeichnet durch** Umsetzen einer Verbindung der Formel (III) 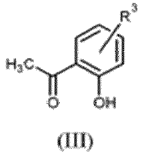
- wobei die Bedeutung von R³ wie oben für die Verbindung der Formel (II) beschrieben ist - mit einer Verbindung der Formel (IV) 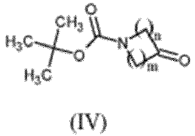
- wobei die Bedeutung von n und m wie oben für die Verbindung der Formel (II) beschrieben ist - entweder in zwei getrennten Schritten über die Formel (VII) 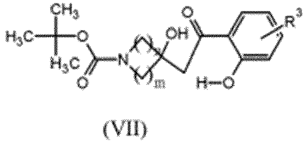
- wobei die Bedeutung von n und m wie oben für die Verbindung der Formel (II) beschrieben ist, oder direkt in einem Schritt um eine Verbindung der Formel (V) zu erhalten 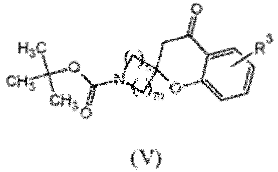
- wobei die Bedeutung von R³, n und m wie oben für die Verbindung der Formel (II) beschrieben ist - die dann umgesetzt wird mit
a.) Chlorwasserstoff um die Formel (VI) zu erhalten 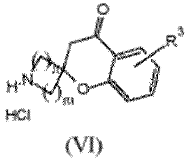
- wobei die Bedeutung von R³, n und m wie oben für die Verbindung der Formel (II) beschrieben ist,
b.) komplexen Hydriden um die Formel (VIII) zu erhalten 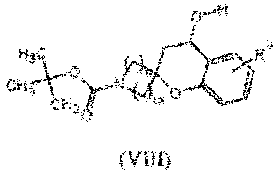
- wobei die Bedeutung von R³, n und m wie oben für die Verbindung der Formel (II) beschrieben ist - dann wird die Verbindung der Formel (VIII) mit Triethylphospin reduziert, um die Formel (IX) zu erhalten 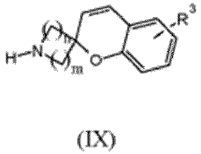
- wobei die Bedeutung von R³, n und m wie oben für die Verbindung der Formel (II) beschrieben ist - dann wird das erhaltene Derivat der Formel (IX) oder Formel (VI) mit der Formel (X) umgesetzt 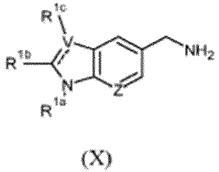
- wobei die Bedeutung von R^{1a}, R^{1b}, R^{1c}, V und Z wie oben für die Verbindung der Formel (II) beschrieben ist - um die Formel (II) zu erhalten 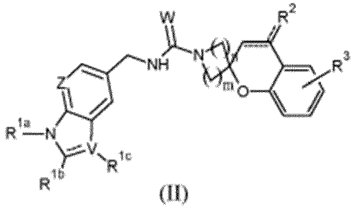
- wobei die Bedeutung von R^{1a}, R^{1b}, R^{1c}, R², R³, V, Z, n, m und W wie oben für die Verbindung der Formel (II) beschrieben ist.

15. Verbindung von {1,2-Dimethyl-1H-pyrrolo[2,3-b]pyridin-5-yl}methanamin.

16. Verbindung von {3-Brom-1-methyl-1H-pyrrolo[2,3-b]pyridin-5-yl}methanamin.

17. Verbindung von 3',4'-Dihydrospiro[azetidin-3,2'-pyrano[3,2-b]pyridin]-4'-on Hydrochlorid.

## Revendications

1. Composé de formule (I) pour une utilisation dans le traitement ou la prévention d'une maladie associée à l'activité du récepteur nicotinique α7 de l'acétylcholine, 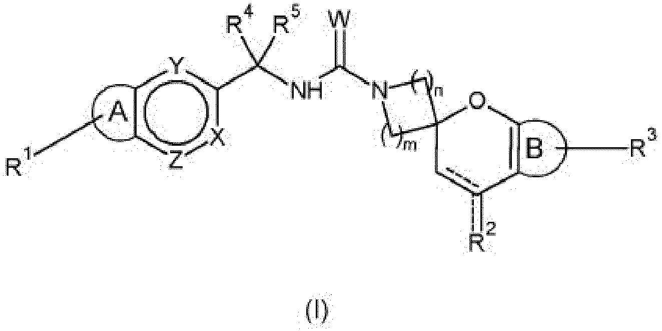 dans lequel :
A est un hétérocycle à cinq ou six chaînons ;
B est un carbocycle ou hétérocycle à six chaînons ;
X est C ou N ;
Y est C ou N ;
Z est C ou N ;
W est O ou S ;
R¹ est H, un alkyle en C₁₋₆, un halogène ou un haloalkyle en C₁₋₆,
R² est H ou O ;
R³ est H, un alkyle en C₁₋₆, un halogène, un haloalkyle en C₁₋₆ ou un alkyle en C₁₋₆ ;
R⁴ est H ou un alkyle en C₁₋₆ ;
R⁵ est H ou un alkyle en C₁₋₆ ;
n et m sont indépendamment 1 ou 2 ;
- - - est une liaison simple ou double ;
ou les sels pharmaceutiquement acceptables, les racémates, les énantiomères, les diastéréoisomères, les solvates et les hydrates de celui-ci.

2. Composé pour une utilisation selon la revendication 1, dans lequel
A est un hétérocycle à cinq chaînons, dans lequel les éléments du cycle sont sélectionnés dans le groupe consistant en le carbone, l'azote, l'oxygène et le soufre ;
B est un carbocycle à six chaînons ou un hétérocycle, dans lequel les éléments du cycle sont sélectionnés dans le groupe consistant en le carbone, l'azote, l'oxygène et le soufre ;
X est C ;
Y est C ;
Z est C ou N ;
W est O ou S ;
R¹ est H, un alkyle en C₁₋₆, un halogène ou un haloalkyle en C₁₋₆,
R² est H ou O ;
R³ est H, un alkyle en C₁₋₆, un halogène, un haloalkyle en C₁₋₆ ou un alkyle en C₁₋₆ ;
R⁴ est H ;
R⁵ est H ;
n et m sont indépendamment 1 ou 2 ;
--- est une liaison simple ou double.

3. Composé de formule (II), 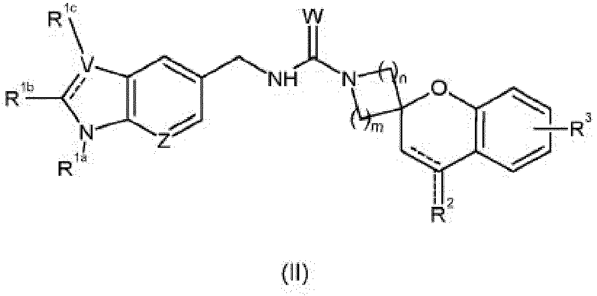 dans lequel
V est C ou S ;
Z est C ou N ;
W est O ou S ;
R^{1a} est H, un alkyle en C₁₋₆, ou un haloalkyle en C₁₋₆ ;
R^{2b} est H, un alkyle en C₁₋₆, un halogène, ou un haloalkyle en C₁₋₆ ;
R^{3c} est H, un alkyle en C₁₋₆, un halogène, ou un haloalkyle en C₁₋₆ quand V est un carbone ; ou R^{1c} est absent quand V est un soufre ;
R² est H ou O ;
R³ est H, un alkyle en C₁₋₆, un halogène, un haloalkyle en C₁₋₆, ou un alkyle en C₁₋₆ ;
n et m sont indépendamment 1 ou 2 ;
--- est une liaison simple ou double ;
ou les sels pharmaceutiquement acceptables, les racémates, les énantiomères, les diastéréoisomères, les solvates et les hydrates de celui-ci.

4. Composé selon la revendication 3, dans lequel
V est C ;
Z est C ou N ;
W est O ;
R^{1a} est H, un alkyle en C₁₋₆, ou un haloalkyle en C₁₋₆ ;
R^{1b} est H, un alkyle en C₁₋₆, un halogène, ou un haloalkyle en C₁₋₆ ;
R^{1c} est H, un alkyle en C₁₋₆, un halogène, ou un haloalkyle en C₁₋₆ ;
R² est H ou O ;
R³ est H, un alkyle en C₁₋₆, un halogène, ou un haloalkyle en C₁₋₆ ;
n et m sont indépendamment 1 ou 2 ;
--- est une liaison simple ou double.

5. Composé tel que défini dans l'une quelconque des revendications 1 ou 4, sélectionné dans le groupe suivant :
le 6'-fluoro-N-[(2-méthyl-1H-indol-5-yl)méthyl]-4'-oxo-3',4'-dihydrospiro[azétidine-3,2'-[1]benzopyrane]-1-carboxamide ;
le 6-chloro-N-[(1-méthyl-1H-indol-5-yl)méthyl]-4-oxo-3,4-dihydrospiro[1-benzopyrane-2,4'-pipéridine]-1'-carboxamide ;
le 6'-chloro-N-[(1-méthyl-1H-indol-5-yl)méthyl]-4'-oxo-3',4'-dihydrospiro[azétidine-3,2'-[1]benzopyrane]-1-carboxamide ;
le 7'-fluoro-N-[(1-méthyl-1H-indol-5-yl)méthyl]-4'-oxo-3',4'-dihydrospiro[azétidine-3,2'-[1]benzopyrane]-1-carboxamide ;
le N-[(1-méthyl-1H-indol-5-yl)méthyl]-4'-oxo-3',4'-dihydrospiro[azétidine-3,2'-[1]benzopyrane]-1-carboxamide ;
le 6-chloro-N-[(1-méthyl-1H-indol-5-yl)méthyl]-3,4-dihydrospiro[1]-benzopyrane-2,4'-pipéridine]-1'-carboxamide ;
le N-[(3-chloro-1-méthyl-1H-indol-5-yl)méthyl]-6'-fluoro-4'-oxo-3',4'-dihydrospiro[azétidine-3,2'-[1]benzopyrane]-1-carboxamide ;
le 6'-chloro-N-[(1H-indol-5-yl)méthyl]-4'-oxo-3',4'-dihydrospiro[azétidine-3,2'-[1]benzopyrane]-1-carboxamide ;
le 6'-fluoro-N-[(1-méthyl-1H-indol-5-yl)méthyl]-3',4'-dihydrospiro[azétidine-3,2'-[1]benzopyrane]-1-carboxamide ;
le 6'-fluoro-N-[(1-méthyl-1H-indol-5-yl)méthyl]spiro[azétidine-3,2'-chromène]-1-carboxamide,
le N-[(1-méthyl-1H-indol-5-yl)méthyl]-3',4'-dihydrospiro[azétidine-3,2'-[1]benzopyrane]-1-carboxamide ;
le 6'-chloro-N-[(2-méthyl-1H-indol-5-yl)méthyl]-4'-oxo-3',4'-dihydrospiro[azétidine-3,2'-[1]benzopyrane]-1-carboxamide ;
le 6',8'-difluoro-N-[(2-méthyl-1H-indol-5-yl)méthyl]-4'-oxo-3',4'-dihydrospiro[azétidine-3,2'-[1]benzopyrane]-1-carboxamide ;
le 7'-chloro-N-[(2-chloro-1H-indol-5-yl)méthyl]-4'-oxo-3',4'-dihydrospiro[azétidine-3,2'-[1]benzopyrane]-1-carboxamide ;
le 7'-chloro-N-[(2-méthyl-1H-indol-5-yl)méthyl]-4'-oxo-3',4'-dihydrospiro[azétidine-3,2'-[1]benzopyrane]-1-carboxamide ;
le 7'-fluoro-N-[(2-méthyl-1H-indol-5-yl)méthyl]-4'-oxo-3',4'-dihydrospiro[azétidine-3,2'-[1]benzopyrane]-1-carboxamide ;
le N-[(2-méthyl-1H-indol-5-yl)méthyl]-4'-oxo-6'-(trifluorométhyl)-3',4'-dihydrospiro[azétidine-3,2'-[1]benzopyrane]-1-carboxamide ;
le N-[(1-méthyl-1H-indol-5-yl)méthyl]-4'-oxo-7'-(trifluorométhyl)-3',4'-dihydrospiro[azétidine-3,2'-[1]benzopyrane]-1-carboxamide ;
le N-[(2-méthyl-1H-indol-5-yl)méthyl]-4'-oxo-7'-(trifluorométhyl)-3',4'-dihydrospiro[azétidine-3,2'-[1]benzopyrane]-1-carboxamide ;
le 6',8'-dichloro-N-[(2-méthyl-1H-indol-5-yl)méthyl]-4'-oxo-3',4'-dihydrospiroazétidine-3,2'-[1]benzopyrane]-1-carboxamide ;
le 7'-fluoro-4'-oxo-N-{[2-(trifluorométhyl)-1H-indol-5-yl]méthyl}-3',4'-dihydrospiro[azétidine-3,2'-[1]benzopyrane]-1-carboxamide ;
le N-{[2-(difluorométhyl)-1H-indol-5-yl]méthyl}-7'-fluoro-4'-oxo-3',4'-dihydrospiro[azétidine-3,2'-[1]benzopyrane]-1-carboxamide ;
ou les sels pharmaceutiquement acceptables, les racémates, les énantiomères, les diastéréoisomères, les solvates et les hydrates de ceux-ci.

6. Composé selon l'une quelconque des revendications 3 à 5, pour une utilisation dans le traitement ou la prévention d'une maladie associée à l'activité du récepteur nicotinique α7 de l'acétylcholine.

7. Composé pour une utilisation selon l'une quelconque des revendications 1, 2 et 6, dans lequel la maladie est sélectionnée dans le groupe comprenant les troubles psychotiques, incluant, mais sans que cela soit limitatif, la schizophrénie, le trouble schizophréniforme, le trouble schizo-affectif, le trouble délirant, le trouble psychotique bref, le trouble psychotique dû à une affection médicale générale, le trouble psychotique induit par une substance ou un trouble psychotique non spécifié autrement, les troubles cognitifs, incluant, mais sans que cela soit limitatif, les troubles cognitifs résultant d'un accident vasculaire cérébral, la maladie d'Alzheimer, la maladie de Huntington, la maladie de Pick, la démence associée au VIH, la démence frontotemporale, la démence à corps de Lewy, la démence vasculaire, une maladie cérébrovasculaire ou d'autres états de démence et les démences associées à d'autres troubles dégénératifs, incluant, mais sans que cela soit limitatif, la sclérose latérale amyotrophique, d'autres affections aiguës ou subaiguës pouvant entraîner un déclin cognitif, incluant, mais sans que cela soit limitatif, le délire, une lésion cérébrale traumatique, la démence sénile, la déficience cognitive légère, le syndrome de Down, la dépression et un déficit cognitif associé à d'autres maladies, et les troubles dyskinétiques, incluant, mais sans que cela soit limitatif, la maladie de Parkinson, le parkinsonisme induit par les neuroleptiques, ou les dyskinésies tardives, la dépression et les troubles de l'humeur, incluant, mais sans que cela soit limitatif, les troubles et épisodes dépressifs, les troubles bipolaires, les troubles cyclothymiques, et un trouble bipolaire non spécifié autrement, les autres troubles de l'humeur, le trouble de l'humeur induit par une substance et un trouble de l'humeur non spécifié autrement, les troubles anxieux, les troubles paniques et les attaques de panique, le trouble obsessionnel-compulsif, le trouble de stress post-traumatique, le trouble de stress aigu, le trouble anxieux généralisé, le trouble anxieux dû à une affection médicale générale, le trouble anxieux induit par une substance, les phobies, et un trouble anxieux non spécifié autrement, les troubles associés à une substance, incluant, mais sans que cela soit limitatif, la consommation de substances ou les troubles induits par une substance, incluant, mais sans que cela soit limitatif, les troubles associés à l'alcool, à la nicotine, aux amphétamines, à la phéncyclidine, aux opioïdes, au cannabis, à la cocaïne, à la caféine, aux hallucinogènes, aux inhalants, aux sédatifs, aux hypnotiques, aux anxiolytiques, à une polytoxicomanie ou à d'autres substances, les troubles du sommeil, incluant, mais sans que cela soit limitatif, la narcolepsie, les dyssomnies, l'hypersomnie primaire, les troubles du sommeil associés à la respiration, un trouble du rythme circadien du sommeil et une dyssomnie non spécifiée autrement, les parasomnies, le trouble de terreur du sommeil, le trouble de somnambulisme et une parasomnie non spécifiée autrement, les troubles du sommeil associés à un autre trouble mental, le trouble du sommeil dû à une affection médicale générale et le trouble du sommeil induit par une substance, les troubles métaboliques et de l'alimentation, incluant, mais sans que cela soit limitatif, l'anorexie mentale, la boulimie, l'obésité, le trouble de l'alimentation compulsive, l'hyperphagie boulimique et un trouble de l'alimentation non spécifié autrement, le diabète sucré, la colite ulcéreuse, la maladie de Crohn, le syndrome du côlon irritable, les troubles du spectre autistique, incluant, mais sans que cela soit limitatif, le trouble autistique, le syndrome d'Asperger, le syndrome de Rett, le trouble désintégratif de l'enfance et un trouble envahissant du développement non spécifié autrement, le trouble déficitaire de l'attention avec hyperactivité, les troubles de comportement perturbateur, le trouble oppositionnel avec provocation et un trouble de comportement perturbateur non spécifié autrement, et les tics, incluant, mais sans que cela soit limitatif, le syndrome de la Tourette, les troubles de la personnalité, les dysfonctionnements sexuels tels que les troubles du désir sexuel, les troubles de l'éveil du désir sexuel, les troubles orgasmiques, les troubles de douleur sexuelle, les dysfonctionnements sexuels non spécifiés autrement, les paraphilies, les troubles d'identité de genre, l'infertilité, le syndrome prémenstruel et les troubles sexuels non spécifiés autrement, les troubles du système respiratoire comme la toux, l'asthme, la bronchopneumopathie chronique obstructive, l'inflammation pulmonaire, les troubles du système cardiovasculaire tels que l'insuffisance cardiaque, l'arythmie cardiaque, l'hypertension, l'inflammation, la douleur inflammatoire et neuropathique, la polyarthrite rhumatoïde, l'arthrose, l'allergie, la sarcoïdose, le psoriasis, l'ataxie, la dystonie, le lupus érythémateux disséminé, la manie, le syndrome des jambes sans repos, la paralysie supranucléaire progressive, l'épilepsie, la myoclonie, la migraine, l'amnésie, le syndrome de fatigue chronique, la cataplexie, l'ischémie cérébrale, la sclérose en plaques, l'encéphalomyélite, les troubles du décalage horaire, l'angiopathie amyloïde cérébrale, et la septicémie.

8. Composé pour une utilisation selon la revendication 7, dans lequel la maladie est sélectionnée dans le groupe comprenant les troubles cognitifs, la schizophrénie et l'autisme.

9. Composition pharmaceutique comprenant, en tant que principe actif, un composé tel que défini dans l'une quelconque des revendications 1 à 5 et au moins un excipient pharmaceutiquement acceptable.

10. Composition pharmaceutique selon la revendication 9, dans laquelle la composition comprend en outre au moins un autres principe actif.

11. Composition pharmaceutique selon la revendication 10, dans laquelle le ou les autres principes actifs sont sélectionnés dans le groupe comprenant les inhibiteurs d'acétylcholinestérase, les antagonistes des récepteurs NMDA, les inhibiteurs de bêta-sécrétase, les antipsychotiques, les NAM ou PAM de la sous-unité alpha5 du récepteur GABA_{A}, les antagonistes du récepteur histaminique H₃, les antagonistes du récepteur 5-HT₆, les agonistes ou PAM de mAChR M1 ou M4, les antagonistes ou NAM ou PAM de mGluR2, et la lévodopa.

12. Combinaison d'un composé tel que défini dans l'une quelconque des revendications 1 à 5 et d'au moins un autres principe actif pour une utilisation dans le traitement ou la prévention d'une maladie associée à l'activité du récepteur nicotinique α7 de l'acétylcholine.

13. Combinaison pour une utilisation selon la revendication 12, dans laquelle le ou les autres principes actifs sont sélectionnés dans le groupe comprenant les inhibiteurs d'acétylcholinestérase, les antagonistes des récepteurs NMDA, les inhibiteurs de bêta-sécrétase, les antipsychotiques, les NAM ou PAM de la sous-unité alpha5 du récepteur GABA_{A}, les antagonistes du récepteur histaminique H₃, les antagonistes du récepteur 5-HT₆, les agonistes ou PAM de mAChR M1 ou M4, les antagonistes ou NAM ou PAM de mGluR2, et la lévodopa.

14. Procédé de préparation de composés de formule (II) selon la revendication 3, **caractérisé par** la réaction d'un composé de formule (III) 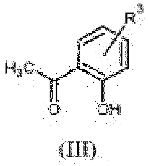
- dans lequel la signification de R³ est décrite ci-dessus pour le composé de formule (II) - avec un composé de formule (IV) 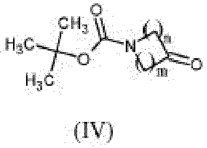
- dans lequel la signification de n et m est décrite ci-dessus pour le composé de formule (II) - soit en deux étapes séparées via la formule (VII) 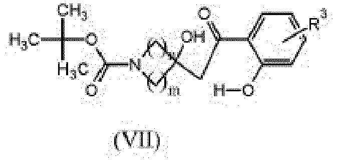
- dans lequel la signification de n et m est décrite ci-dessus pour le composé de formule (II), soit directement en une étape pour fournir un composé de formule (V) 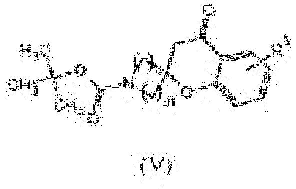
- dans lequel la signification de R³, n et m est telle que décrite ci-dessus pour la formule (II) - qui est ensuite mis à réagir avec
a.) du chlorure d'hydrogène pour fournir la formule (VI) 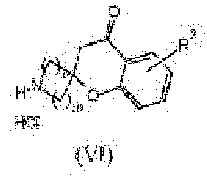
- dans lequel la signification de R³, n et m est telle que décrite ci-dessus pour la formule (II),
b.) des hydrures complexes pour fournir la formule (VIII) 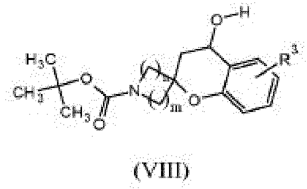
- dans lequel la signification de R³, n et m est telle que décrite ci-dessus pour la formule (II) - ensuite le composé de formule (VIII) est réduit avec de la triéthylphosphine pour fournir la formule (IX) 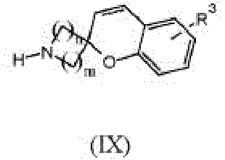
- dans lequel la signification de R³, n et m est telle que décrite ci-dessus pour la formule (II) - ensuite le dérivé obtenu de formule (IX) ou de formule (VI) - est mis à réagir avec la formule (X) 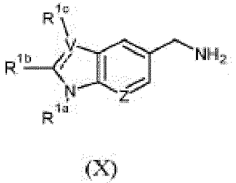
- dans lequel la signification de R^{1a}, R^{1b}, R^{1c}, V, et Z est telle que décrite ci-dessus pour la formule (II) - pour fournir la formule (II) 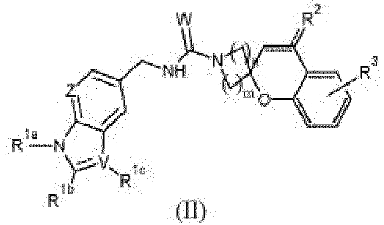
- dans lequel la signification de R^{1a}, R^{1b}, R^{1c}, R², R³, V, Z, n, m, et W est telle que décrite ci-dessus pour la formule (II).

15. Composé de la {1,2-diméthyl-1H-pyrrolo[2,3-b]pyridin-5-yl}méthanamine.

16. Composé de la {3-bromo-1-méthyl-1H-pyrrolo[2,3-b]pyridin-5-yl}méthanamine.

17. Composé du chlorhydrate de la 3',4'-dihydrospiro[azétidine-3,2'-pyrano[3,2-b]pyridin]-4'-one.
